# EUROPEAN PATENT APPLICATION

(11) **EP 2 871 237 A1**
(43) Date of publication of application: **13.05.2015**
(21) Application number: 13813919.1
(22) Date of filing: 08.07.2013
(51) Int. Cl.: C12N 15/09, A01H 1/00, A01H 5/00, A01H 5/10, C12N 15/113

(54) **WHEAT WITH INCREASED GRAIN-BEARING NUMBER AND METHOD FOR PRODUCING SAME, AND CHEMICAL FOR INCREASING GRAIN-BEARING NUMBER OF WHEAT**

(30) Priority: 06.07.2012 JP 2012152765
(71) Applicant: National Institute of Agrobiological Sciences, Tsukuba-shi, Ibaraki 305-8602 (JP)
(72) Inventor: KOMATSUDA, Takao, Tsukuba-shi Ibaraki 305-8602 (JP); MATSUMOTO, Takashi, Tsukuba-shi Ibaraki 305-8602 (JP); SAKUMA, Shun, Tsukuba-shi Ibaraki 305-8602 (JP); OGAWA, Taiichi, Tsukuba-shi Ibaraki 305-8602 (JP)
(74) Representative: Campbell, Patrick John Henry
(86) International application number: PCT/JP2013/068675
(87) International publication number: WO 2014/007396

(57) **Abstract**

The Vrs1 gene was found to be highly expressed in the pistils of sterile lateral spikelets of two-row barley, showing that the VRS1 protein suppresses the fertility of florets in lateral spikelets. In addition, it was also found that the introduction of siRNA specific to the Vrs1 gene into two-row barley successfully restored the fertility of lateral spikelets and thus can increase the number of grains. The vrs1 gene derived from wheat was isolated for the first time, and the expression site of the gene was found to be specific to upper florets to be sterile in spikelets. Furthermore, it was confirmed that the introduction of siRNA specific to the wheat Vrs1 gene into wheat successfully increased the number of florets and the number of grains per spikelet, enabling provision of a method and an agent for increasing the number of grains per spikelet of wheat.

SEQUENCE LISTING
<110>  NATIONAL INSTITUTE OF AGROBIOLOGICAL SCIENCES
<120>
\222\205\227±\220\224\202ª\221\235\211Á\202µ\202½\203R\203\200\203M\213y\202Ñ
\202»\202Ì\220¶\216Y\225û\226@\201A\225À\202Ñ\202É\203R\203\200\203M\202Ì\222
\205\227±\220\224\202ð\221\235\211Á\202³\202¹\202é\202½\202ß\202Ì\226ò\215Ü
<130>  IBPF13-529WO
<150>  JP2012/152765
<151>  2012-07-06
<160>  77
<170>  PatentIn version 3.1

<210>  1
<211>  1472
<212>  DNA
<213>  Triticum monococcum
<400>  1
cgcactttcc cctccttcaa ctagcgcttt gcggccgttg gtcctcccct cgatccagtt      60
cctcacctca ccacaccaag agcgcaaacc caccgtgtct ccccttcctc taatcttcca     120
attccaacca tcccttctct cccctcctca gctccgaaca acaggcatgg acaaccagca     180
gctctttggt tcctcctacg tggacacgcc tttcttcgcg gccaatggta cgtacgacgc     240
cgcccgcccc cggtgtttgc gcatgcgatg atgcagctgc agtagcttca ttttcaccgg     300
ccaggacacg catgtgatgc ccttttttcc atgtcgttgt tgtgtgtgtg caggcacggc     360
gcagcgggag agccagccga aggctcggcg caggcggcgg agggccgcga gatgcggcgg     420
aggggatggt aacggctggg agatggacgg aggaggggac cccaagaagc ggcggctcac     480
cgatgagcag gcggagatgc tggagctgag cttccgggag gaacgcaagc tggagaccgg     540
ccggaaggtg tatctggccg ccgagctcgg gctcgacccc aagcaagtcg ccgtgtggtt     600
ccagaaccgc cgggcgcgcc acaagagcaa gacgctcgag caggagttcg ccaggctcaa     660
gcacgcccac gacgcagcca tcctccacaa atgccacctc gagaacgagg taagcttgct     720
catacacact cacactggcg tacatacaaa ttcctcttcg ttcttgcaca agctgactgc     780
cagccaggtg ctgaggctga aggagaagct gagagcgact gcggaggagg tgcagcgcct     840
caggtcggca gctgggatcc acggggcatc cgccgatggc ggagactccg ctggcgccgt     900
tagcgtgtgc ggcgggagcc cgggctcatc cttctcgacg ggaacctgcc agcagcatcc     960
gggtttcagc ggggcagacg tgctggggcg ggacgatgac ctgatgatgt gcgtccccga    1020
gtggtttttc gcatgaatta gctagagttt atggtggcta cgccgatagc agcgtgttcg    1080
agtgtttttt aattagcatg aaataaaatc tcccacaaaa taggcgctat agctgctcgg    1140
aagcctcacg gctacgctat ggctgccgcc gccaaatcct ccacaaatcc ctccaaaggg    1200
cttcagaatc agcaaaaacc ttcagaaatc atctaccccg ccggaaaagt tgttgctatt    1260
tgccgcgatt gcctgctatg cggccgctat agctactgag agtggccgcc cctaaatcct    1320
ttctctggcg attttaatct ttggttttta gcctgtatat atggggtgat ttgaaattga    1380
cacgagctgg acatcaacta catgctgatc gattactatt ctagttagcc atagttttaa    1440
ttattaatta agttgggacg atctctatgc gt                                  1472

<210>  2
<211>  1268
<212>  DNA
<213>  Triticum monococcum
<220>
<221>  CDS
<222>  (167)..(832)
<223>
<400>  2
cgcactttcc cctccttcaa ctagcgcttt gcggccgttg gtcctcccct cgatccagtt      60
cctcacctca ccacaccaag agcgcaaacc caccgtgtct ccccttcctc taatcttcca     120
attccaacca tcccttctct cccctcctca gctccgaaca acaggc atg gac aac       175
                                                   Met Asp Asn
                                                   1
cag cag ctc ttt ggt tcc tcc tac gtg gac acg cct ttc ttc gcg gcc      223
Gln Gln Leu Phe Gly Ser Ser Tyr Val Asp Thr Pro Phe Phe Ala Ala
    5                   10                  15
aat ggc acg gcg cag cgg gag agc cag ccg aag gct cgg cgc agg cgg      271
Asn Gly Thr Ala Gln Arg Glu Ser Gln Pro Lys Ala Arg Arg Arg Arg
20                  25                  30                  35

cgg agg gcc gcg aga tgc ggc gga ggg gat ggt aac ggc tgg gag atg 319
Arg Arg Ala Ala Arg Cys Gly Gly Gly Asp Gly Asn Gly Trp Glu Met
                40                  45                  50
gac gga gga ggg gac ccc aag aag cgg cgg ctc acc gat gag cag gcg 367
Asp Gly Gly Gly Asp Pro Lys Lys Arg Arg Leu Thr Asp Glu Gln Ala
            55                  60                  65
gag atg ctg gag ctg agc ttc cgg gag gaa cgc aag ctg gag acc ggc 415
Glu Met Leu Glu Leu Ser Phe Arg Glu Glu Arg Lys Leu Glu Thr Gly
        70                  75                  80
cgg aag gtg tat ctg gcc gcc gag ctc ggg ctc gac ccc aag caa gtc 463
Arg Lys Val Tyr Leu Ala Ala Glu Leu Gly Leu Asp Pro Lys Gln Val
    85                  90                  95
gcc gtg tgg ttc cag aac cgc cgg gcg cgc cac aag agc aag acg ctc 511
Ala Val Trp Phe Gln Asn Arg Arg Ala Arg His Lys Ser Lys Thr Leu
100                 105                 110                 115
gag cag gag ttc gcc agg ctc aag cac gcc cac gac gca gcc atc ctc 559
Glu Gln Glu Phe Ala Arg Leu Lys His Ala His Asp Ala Ala Ile Leu
                120                 125                 130
cac aaa tgc cac ctc gag aac gag gtg ctg agg ctg aag gag aag ctg 607
His Lys Cys His Leu Glu Asn Glu Val Leu Arg Leu Lys Glu Lys Leu
            135                 140                 145
aga gcg act gcg gag gag gtg cag cgc ctc agg tcg gca gct ggg atc 655
Arg Ala Thr Ala Glu Glu Val Gln Arg Leu Arg Ser Ala Ala Gly Ile
        150                 155                 160
cac ggg gca tcc gcc gat ggc gga gac tcc gct ggc gcc gtt agc gtg 703
His Gly Ala Ser Ala Asp Gly Gly Asp Ser Ala Gly Ala Val Ser Val
    165                 170                 175
tgc ggc ggg agc ccg ggc tca tcc ttc tcg acg gga acc tgc cag cag 751
Cys Gly Gly Ser Pro Gly Ser Ser Phe Ser Thr Gly Thr Cys Gln Gln
180                 185                 190                 195
cat ccg ggt ttc agc ggg gca gac gtg ctg ggg cgg gac gat gac ctg 799
His Pro Gly Phe Ser Gly Ala Asp Val Leu Gly Arg Asp Asp Asp Leu
                200                 205                 210
atg atg tgc gtc ccc gag tgg ttt ttc gca tga attagctaga gtttatggtg 852
Met Met Cys Val Pro Glu Trp Phe Phe Ala
            215                 220
gctacgccga tagcagcgtg ttcgagtgtt ttttaattag catgaaataa aatctcccac 912
aaaataggcg ctatagctgc tcggaagcct cacggctacg ctatggctgc cgccgccaaa 972
tcctccacaa atccctccaa agggcttcag aatcagcaaa aaccttcaga aatcatctac 1032
cccgccggaa aagttgttgc tatttgccgc gattgcctgc tatgcggccg ctatagctac 1092
tgagagtggc cgcccctaaa tcctttctct ggcgatttta atctttggtt tttagcctgt 1152
atatatgggg tgatttgaaa ttgacacgag ctggacatca actacatgct gatcgattac 1212
tattctagtt agccatagtt ttaattatta attaagttgg gacgatctct atgcgt 1268

<210> 3
<211> 221
<212> PRT
<213> Triticum monococcum
<400> 3
Met Asp Asn Gln Gln Leu Phe Gly Ser Ser Tyr Val Asp Thr Pro Phe
1               5                   10                  15
Phe Ala Ala Asn Gly Thr Ala Gln Arg Glu Ser Gln Pro Lys Ala Arg
            20                  25                  30
Arg Arg Arg Arg Arg Ala Ala Arg Cys Gly Gly Gly Asp Gly Asn Gly
        35                  40                  45
Trp Glu Met Asp Gly Gly Gly Asp Pro Lys Lys Arg Arg Leu Thr Asp
    50                  55                  60
Glu Gln Ala Glu Met Leu Glu Leu Ser Phe Arg Glu Glu Arg Lys Leu
65                  70                  75                  80
Glu Thr Gly Arg Lys Val Tyr Leu Ala Ala Glu Leu Gly Leu Asp Pro
                85                  90                  95
Lys Gln Val Ala Val Trp Phe Gln Asn Arg Arg Ala Arg His Lys Ser
            100                 105                 110

Lys Thr Leu Glu Gln Glu Phe Ala Arg Leu Lys His Ala His Asp Ala
        115                 120                 125
Ala Ile Leu His Lys Cys His Leu Glu Asn Glu Val Leu Arg Leu Lys
    130                 135                 140
Glu Lys Leu Arg Ala Thr Ala Glu Glu Val Gln Arg Leu Arg Ser Ala
145                 150                 155                 160
Ala Gly Ile His Gly Ala Ser Ala Asp Gly Gly Asp Ser Ala Gly Ala
                165                 170                 175
Val Ser Val Cys Gly Gly Ser Pro Gly Ser Ser Phe Ser Thr Gly Thr
            180                 185                 190
Cys Gln Gln His Pro Gly Phe Ser Gly Ala Asp Val Leu Gly Arg Asp
        195                 200                 205
Asp Asp Leu Met Met Cys Val Pro Glu Trp Phe Phe Ala
    210                 215                 220

<210>  4
<211>  1263
<212>  DNA
<213>  Triticum monococcum
<400>  4
tcaaatagtg ccttgcggtg gtcctcctct cgatccagtt cctcaccaca ccaacaaaac      60
acaacccacc gggcctcccc tctaacctcc ccaccaccac cccttctctc ccctcctcag     120
ctcccaacag caagcatgga caagcagcag gtctttggtt cctcctacgt ggacatgcct     180
ttcttcgcgg ccaatggtac gtacgacgcc gcgcgcgccc gctgtttctt tgcgcatgcg     240
atgatgcggc tgctgtagct tcagtttcac cggccaggag cccaggacac gcatatgatg     300
cccttttttc ggttccgtgt gtgtaggtgc agccatggca cagggggaga gccggccgag     360
ggcgcggcgc aggaggcgga gggcggcgag gtgcggcgga ggggacggtg acggcgggga     420
catggacgga ggaggggatc ccaagaagcg gcggctgacc gacgaccagg tgcacggtct     480
ggagctgagc ttccgggagg agcgcaagct ggagaccagc cgaaaggttc atctcgctgc     540
cgagctcggg ctcgatccca agcaagtcgc ggtgtggttc cagaaccgac gggcgcgaca     600
caagagcaag cttatggagg aggagttcac caagctcaag caggcccacg acgcggccat     660
cctccacaaa tgccatctcg agaacgaggt atgcttgctc gcacacaccc acactggcat     720
acatatggcg ccgtacatca taaattcctc tccgttcttg cacaagctga ctgacgcaca     780
tatggccagt tgctgaggct gaaggagagg ctgggagcga ccgaggagga ggtgcggcgc     840
ctcaggtcgg cagctgggag ccacggagca tccggcaacg gcggagacgc cgctgacgcc     900
gttggcgcgt gcggcgggag cccgagctcg tccttctcga cgggaacctg ccagcagcat     960
ccgggtttca gcggcgcagg ccagctgggg ccggacgatg agctgatgat gtgcgtcccc    1020
gagtatggtg gctacgccga tagcagcgtg gtcgagtggt ttagcctgta tgggctaatt    1080
tgaaattgag acgagctggg tatcaactgg atgcagatcg attactattc tagttagcca    1140
tagcgttaat taagctgggg tgatctctag ggcgtgcctg tttaattatg tagatagtgt    1200
tgattaatgg caaagcttgc aagctagcaa gtgtagtact actggtagct ctcttgaagt    1260
tca                                                                  1263

<210>  5
<211>  1032
<212>  DNA
<213>  Triticum monococcum
<220>
<221>  CDS
<222>  (136)..(852)
<223>
<400>  5
tcaaatagtg ccttgcggtg gtcctcctct cgatccagtt cctcaccaca ccaacaaaac      60
acaacccacc gggcctcccc tctaacctcc ccaccaccac cccttctctc ccctcctcag     120
ctcccaacag caagc atg gac aag cag cag gtc ttt ggt tcc tcc tac gtg     171
                 Met Asp Lys Gln Gln Val Phe Gly Ser Ser Tyr Val
                 1               5                   10
gac atg cct ttc ttc gcg gcc aat ggt gca gcc atg gca cag ggg gag     219
Asp Met Pro Phe Phe Ala Ala Asn Gly Ala Ala Met Ala Gln Gly Glu
        15                  20                  25
agc cgg ccg agg gcg cgg cgc agg agg cgg agg gcg gcg agg tgc ggc     267
Ser Arg Pro Arg Ala Arg Arg Arg Arg Arg Arg Ala Ala Arg Cys Gly
    30                  35                  40

gga ggg gac ggt gac ggc ggg gac atg gac gga gga ggg gat ccc aag     315
Gly Gly Asp Gly Asp Gly Gly Asp Met Asp Gly Gly Gly Asp Pro Lys
45                  50                  55                  60
aag cgg cgg ctg acc gac gac cag gtg cac ggt ctg gag ctg agc ttc     363
Lys Arg Arg Leu Thr Asp Asp Gln Val His Gly Leu Glu Leu Ser Phe
                65                  70                  75
cgg gag gag cgc aag ctg gag acc agc cga aag gtt cat ctc gct gcc     411
Arg Glu Glu Arg Lys Leu Glu Thr Ser Arg Lys Val His Leu Ala Ala
            80                  85                  90
gag ctc ggg ctc gat ccc aag caa gtc gcg gtg tgg ttc cag aac cga     459
Glu Leu Gly Leu Asp Pro Lys Gln Val Ala Val Trp Phe Gln Asn Arg
        95                  100                 105
cgg gcg cga cac aag agc aag ctt atg gag gag gag ttc acc aag ctc     507
Arg Ala Arg His Lys Ser Lys Leu Met Glu Glu Glu Phe Thr Lys Leu
    110                 115                 120
aag cag gcc cac gac gcg gcc atc ctc cac aaa tgc cat ctc gag aac     555
Lys Gln Ala His Asp Ala Ala Ile Leu His Lys Cys His Leu Glu Asn
125                 130                 135                 140
gag ttg ctg agg ctg aag gag agg ctg gga gcg acc gag gag gag gtg     603
Glu Leu Leu Arg Leu Lys Glu Arg Leu Gly Ala Thr Glu Glu Glu Val
                145                 150                 155
cgg cgc ctc agg tcg gca gct ggg agc cac gga gca tcc ggc aac ggc     651
Arg Arg Leu Arg Ser Ala Ala Gly Ser His Gly Ala Ser Gly Asn Gly
            160                 165                 170
gga gac gcc gct gac gcc gtt ggc gcg tgc ggc ggg agc ccg agc tcg     699
Gly Asp Ala Ala Asp Ala Val Gly Ala Cys Gly Gly Ser Pro Ser Ser
        175                 180                 185
tcc ttc tcg acg gga acc tgc cag cag cat ccg ggt ttc agc ggc gca     747
Ser Phe Ser Thr Gly Thr Cys Gln Gln His Pro Gly Phe Ser Gly Ala
    190                 195                 200
ggc cag ctg ggg ccg gac gat gag ctg atg atg tgc gtc ccc gag tat     795
Gly Gln Leu Gly Pro Asp Asp Glu Leu Met Met Cys Val Pro Glu Tyr
205                 210                 215                 220
ggt ggc tac gcc gat agc agc gtg gtc gag tgg ttt agc ctg tat ggg     843
Gly Gly Tyr Ala Asp Ser Ser Val Val Glu Trp Phe Ser Leu Tyr Gly
                225                 230                 235
cta att tga aattgagacg agctgggtat caactggatg cagatcgatt             892
Leu Ile

actattctag ttagccatag cgttaattaa gctggggtga tctctagggc gtgcctgttt   952
aattatgtag atagtgttga ttaatggcaa agcttgcaag ctagcaagtg tagtactact   1012
ggtagctctc ttgaagttca                                               1032

<210>  6
<211>  238
<212>  PRT
<213>  Triticum monococcum
<400>  6
Met Asp Lys Gln Gln Val Phe Gly Ser Ser Tyr Val Asp Met Pro Phe
1               5                   10                  15
Phe Ala Ala Asn Gly Ala Ala Met Ala Gln Gly Glu Ser Arg Pro Arg
            20                  25                  30
Ala Arg Arg Arg Arg Arg Arg Ala Ala Arg Cys Gly Gly Gly Asp Gly
        35                  40                  45
Asp Gly Gly Asp Met Asp Gly Gly Gly Asp Pro Lys Lys Arg Arg Leu
    50                  55                  60
Thr Asp Asp Gln Val His Gly Leu Glu Leu Ser Phe Arg Glu Glu Arg
65                  70                  75                  80
Lys Leu Glu Thr Ser Arg Lys Val His Leu Ala Ala Glu Leu Gly Leu
                85                  90                  95
Asp Pro Lys Gln Val Ala Val Trp Phe Gln Asn Arg Arg Ala Arg His
            100                 105                 110
Lys Ser Lys Leu Met Glu Glu Glu Phe Thr Lys Leu Lys Gln Ala His

115                 120                 125
Asp Ala Ala Ile Leu His Lys Cys His Leu Glu Asn Glu Leu Leu Arg
    130                 135                 140
Leu Lys Glu Arg Leu Gly Ala Thr Glu Glu Glu Val Arg Arg Leu Arg
145                 150                 155                 160
Ser Ala Ala Gly Ser His Gly Ala Ser Gly Asn Gly Gly Asp Ala Ala
                165                 170                 175
Asp Ala Val Gly Ala Cys Gly Gly Ser Pro Ser Ser Ser Phe Ser Thr
            180                 185                 190
Gly Thr Cys Gln Gln His Pro Gly Phe Ser Gly Ala Gly Gln Leu Gly
        195                 200                 205
Pro Asp Asp Glu Leu Met Met Cys Val Pro Glu Tyr Gly Gly Tyr Ala
    210                 215                 220
Asp Ser Ser Val Val Glu Trp Phe Ser Leu Tyr Gly Leu Ile
225                 230                 235

<210>  7
<211>  19
<212>  DNA
<213>  Artificial
<220>
<223>  Artificially synthesized primer sequence
<400>  7
atgtggatat caggaagga                                                19

<210>  8
<211>  22
<212>  DNA
<213>  Artificial
<220>
<223>  Artificially synthesized primer sequence
<400>  8
tcgcaactta gaagcacttc cg                                            22

<210>  9
<211>  25
<212>  DNA
<213>  Artificial
<220>
<223>  Artificially synthesized primer sequence
<400>  9
catacttaac gcacgcctag agatc                                         25

<210>  10
<211>  21
<212>  DNA
<213>  Artificial
<220>
<223>  Artificially synthesized primer sequence
<400>  10
tcacgatccc ttctttccct c                                             21

<210>  11
<211>  24
<212>  DNA
<213>  Artificial
<220>
<223>  Artificially synthesized primer sequence
<400>  11
gacgcactcc cctccttcaa ctag                                          24

<210>  12
<211>  24

<212>  DNA
<213>  Artificial
<220>
<223>  Artificially synthesized primer sequence
<400>  12
aacacaacac atcgtctctg aact                                              24

<210>  13
<211>  24
<212>  DNA
<213>  Artificial
<220>
<223>  Artificially synthesized primer sequence
<400>  13
aagtacagtg tctggattgg aggg                                              24

<210>  14
<211>  20
<212>  DNA
<213>  Artificial
<220>
<223>  Artificially synthesized primer sequence
<400>  14
ccgagatagc tgctgccgcc                                                   20

<210>  15
<211>  20
<212>  DNA
<213>  Artificial
<220>
<223>  Artificially synthesized primer sequence
<400>  15
tgcatcgcgg gcaatggaga                                                   20

<210>  16
<211>  24
<212>  DNA
<213>  Artificial
<220>
<223>  Artificially synthesized primer sequence
<400>  16
gcgtggtcga gtggtttagc ctgt                                              24

<210>  17
<211>  22
<212>  DNA
<213>  Artificial
<220>
<223>  Artificially synthesized primer sequence
<400>  17
cgcctacaga tcatcccagc tt                                                22

<210>  18
<211>  25
<212>  DNA
<213>  Artificial
<220>
<223>  Artificially synthesized primer sequence
<400>  18
ggtttttagc atgaattaga gttta                                             25

<210>  19
<211>  25

<212>  DNA
<213>  Artificial
<220>
<223>  Artificially synthesized primer sequence
<400>  19
tatacaggct aaaaaccaaa gatta                                          25

<210>  20
<211>  21
<212>  DNA
<213>  Artificial
<220>
<223>  Artificially synthesized primer sequence
<400>  20
gacgcactcc cctccttcaa c                                              21

<210>  21
<211>  21
<212>  DNA
<213>  Artificial
<220>
<223>  Artificially synthesized primer sequence
<400>  21
gctgaagtaa aacatatact a                                              21

<210>  22
<211>  20
<212>  DNA
<213>  Artificial
<220>
<223>  Artificially synthesized primer sequence
<400>  22
gatcggacga ttgcgtcgca                                                20

<210>  23
<211>  20
<212>  DNA
<213>  Artificial
<220>
<223>  Artificially synthesized primer sequence
<400>  23
tatcggcact ttgcatcggc                                                20

<210>  24
<211>  40
<212>  DNA
<213>  Artificial
<220>
<223>  Artificially synthesized primer sequence
<400>  24
gatctttctt tcttcttttt gtgggtagaa tttgaatccc                          40

<210>  25
<211>  40
<212>  DNA
<213>  Artificial
<220>
<223>  Artificially synthesized primer sequence
<400>  25
cgggactcta atcataaaaa cccatctcat aaataacgtc                          40

<210>  26
<211>  40

<212>  DNA
<213>  Artificial
<220>
<223>  Artificially synthesized primer sequence
<400>  26
agctatatgt ggattttttt agccctgcct tcatacgcta                              40

<210>  27
<211>  9
<212>  PRT
<213>  Artificial
<220>
<223>  Artificially synthesized polypeptide antigen
<220>
<221>  MISC_FEATURE
<222>  (2)..(2)
<223>  X represents aminohexanoic acid
<400>  27
Cys Xaa Val Pro Glu Trp Phe Leu Ala
1               5

<210>  28
<211>  9
<212>  PRT
<213>  Hordeum vulgare
<400>  28
Arg Arg Arg Arg Arg Arg Ser Ala Arg
1               5

<210>  29
<211>  13
<212>  PRT
<213>  Hordeum vulgare
<400>  29
Arg Pro Arg Ala Arg Arg Arg Arg Arg Arg Ala Ala Arg
1               5                   10

<210>  30
<211>  21
<212>  DNA
<213>  Artificial
<220>
<223>  Artificially synthesized primer sequence
<400>  30
caaagaatcc agagtctcga a                                                  21

<210>  31
<211>  21
<212>  DNA
<213>  Artificial
<220>
<223>  Artificially synthesized primer sequence
<400>  31
cgtgtcaatt tcaaatcacc c                                                  21

<210>  32
<211>  21
<212>  DNA
<213>  Artificial
<220>
<223>  Artificially synthesized primer sequence
<400>  32
cgcactttcc cctccttcaa c                                                  21

<210>  33
<211>  21
<212>  DNA
<213>  Artificial
<220>
<223>  Artificially synthesized primer sequence
<400>  33
tccacgtagg aggaaccaaa g                                                21

<210>  34
<211>  21
<212>  DNA
<213>  Artificial
<220>
<223>  Artificially synthesized primer sequence
<400>  34
atgatgcagc tgcagtagct t                                                21

<210>  35
<211>  21
<212>  DNA
<213>  Artificial
<220>
<223>  Artificially synthesized primer sequence
<400>  35
agtgtgtatg agcaagctta c                                                21

<210>  36
<211>  21
<212>  DNA
<213>  Artificial
<220>
<223>  Artificially synthesized primer sequence
<400>  36
tggtttttcg catgaattag c                                                21

<210>  37
<211>  21
<212>  DNA
<213>  Artificial
<220>
<223>  Artificially synthesized primer sequence
<400>  37
ctttggaggg atttgtggag g                                                21

<210>  38
<211>  21
<212>  DNA
<213>  Artificial
<220>
<223>  Artificially synthesized primer sequence
<400>  38
ccaaagatta aaatcgccag a                                                21

<210>  39
<211>  16
<212>  DNA
<213>  Artificial
<220>
<223>  Artificially synthesized primer sequence
<400>  39
cagagggccg atcttt                                                      16

<210>  40
<211>  21
<212>  DNA
<213>  Artificial
<220>
<223>  Artificially synthesized primer sequence
<400>  40
tgcaagcttt gccattaatc a                                                21

<210>  41
<211>  20
<212>  DNA
<213>  Artificial
<220>
<223>  Artificially synthesized primer sequence
<400>  41
acttggctgc accatacgtt                                                  20

<210>  42
<211>  21
<212>  DNA
<213>  Artificial
<220>
<223>  Artificially synthesized primer sequence
<400>  42
aaacgtatgg tgcagccaag t                                                21

<210>  43
<211>  21
<212>  DNA
<213>  Artificial
<220>
<223>  Artificially synthesized primer sequence
<400>  43
atcaaatagt gccttgcggt g                                                21

<210>  44
<211>  21
<212>  DNA
<213>  Artificial
<220>
<223>  Artificially synthesized primer sequence
<400>  44
ctttggttcc tcctacgtgg a                                                21

<210>  45
<211>  22
<212>  DNA
<213>  Artificial
<220>
<223>  Artificially synthesized primer sequence
<400>  45
agagcaagct tatggaggag ga                                               22

<210>  46
<211>  22
<212>  DNA
<213>  Artificial
<220>
<223>  Artificially synthesized primer sequence
<400>  46
tggccatatg tgcgtcagtc ag                                               22

<210>  47
<211>  24
<212>  DNA
<213>  Artificial
<220>
<223>  Artificially synthesized primer sequence
<400>  47
gcgtggtcga gtggtttagc ctgt                                              24

<210>  48
<211>  21
<212>  DNA
<213>  Artificial
<220>
<223>  Artificially synthesized primer sequence
<400>  48
tcaccccagc ttaattaacg c                                                 21

<210>  49
<211>  21
<212>  DNA
<213>  Artificial
<220>
<223>  Artificially synthesized primer sequence
<400>  49
tgtccagctc gtgtcaattt c                                                 21

<210>  50
<211>  21
<212>  DNA
<213>  Artificial
<220>
<223>  Artificially synthesized primer sequence
<400>  50
cactcccctt ccttacttaa c                                                 21

<210>  51
<211>  21
<212>  DNA
<213>  Artificial
<220>
<223>  Artificially synthesized primer sequence
<400>  51
ataggcgcta tagctgctcg g                                                 21

<210>  52
<211>  24
<212>  DNA
<213>  Artificial
<220>
<223>  Artificially synthesized primer sequence
<400>  52
gcgtggtcga gtggtttagc ctgt                                              24

<210>  53
<211>  24
<212>  DNA
<213>  Artificial
<220>
<223>  Artificially synthesized primer sequence
<400>  53
gccctagaga tcaccccagc ttaa                                              24

<210>  54
<211>  25
<212>  DNA
<213>  Artificial
<220>
<223>  Artificially synthesized primer sequence
<400>  54
cacctggttt ttcgcatgaa ttagc                                              25

<210>  55
<211>  21
<212>  DNA
<213>  Artificial
<220>
<223>  Artificially synthesized primer sequence
<400>  55
ccaaagatta aaatcgccgg a                                                  21

<210>  56
<211>  1355
<212>  DNA
<213>  Triticum aestivum
<400>  56
cttcaactag cgctttgcgg ccgttggtcc tcccctcgat ccagttcctc acctcaccac      60
accaagagcg caacccaccg cgtctcccct tcctctaatc ttccaattcc aaccatccct     120
tctcttccct cctgagctcc gaacagcagg catggacaac cagcagctct ttggttcctc     180
ctacgtggac acgcctttct tcgcggccaa tggtacgtac gacgccgcgc gcccccggtg     240
tttgcgcatg cgatgatgca gctgcagtag cttcattttc accggccagg acacgcatgt     300
gatgcccttt tttccatgtc gttgttgtgt gtgtgcaggc acggcgcagc gggagagcca     360
gccgaaggct cggcgcaggc ggcggagggc cgcgagatgc ggcggagggg atggtaacgg     420
ctgggagatg gacggaggag gggaccccaa gaagcggcgg ctcaccgatg agcaggcgga     480
gatgctggag ctgagcttcc gggaggaacg caagctggag accggccgga aggtgtatct     540
ggccgccgag ctcgggctcg accccaagca ggtcgccgtg tggttccagt accgccgggc     600
gcgccacaag agcaagacgc tcgagcagga gttcgccagg ctcaagcacg cccacgacgc     660
agccatcctc cacaaatgcc acctcgagaa cgaggtaagc ttgctcatac acactcacac     720
tggcgtacat acaaattcct cttcgttctt gcacaagctg actgccagcc aggtgctgag     780
gctgaaggag aagctgagag cgactgcgga ggaggtgcag cgcctcaggt cggcagctgg     840
gatccacgcc gatggcggag actccgctgg cgccgttagc gtgtgcggcg ggagcccgcg     900
ctcatccttc tcgacgggaa cctgccagca gcatccgggt ttcagcgggg cagacgtgct     960
ggggcgggac gatgacctga tgatgtgcgt ccccgagtgg tttttcgcat gaattagcta    1020
gagtttatgg tggctatgcc gatagcagcg tgttcgagtg ttttttaatt agcatgaaat    1080
aaaatctccc acaaaatagg cgctatagct gctcgggagc ctcgccgcta cgctatggct    1140
gccgccgcca aatcctccac aaatccctcc aaagggcttc agaatcagca aaaacctcta    1200
gaaatcatct gtcccgccgg aaaagttgtt gctatttgcc gcgattgtct gctatgcggc    1260
cgctatagct gctgagagtg gccgccctta aatcctttct ccggcgattt taatctttgg    1320
tttttagcct gtatatatgg ggtgatttga aattg                               1355

<210>  57
<211>  657
<212>  DNA
<213>  Triticum aestivum
<220>
<221>  CDS
<222>  (1)..(657)
<223>
<400>  57
atg gac aac cag cag ctc ttt ggt tcc tcc tac gtg gac acg cct ttc      48
Met Asp Asn Gln Gln Leu Phe Gly Ser Ser Tyr Val Asp Thr Pro Phe
1               5                   10                  15
ttc gcg gcc aat ggc acg gcg cag cgg gag agc cag ccg aag gct cgg      96
Phe Ala Ala Asn Gly Thr Ala Gln Arg Glu Ser Gln Pro Lys Ala Arg
            20                  25                  30

cgc agg cgg cgg agg gcc gcg aga tgc ggc gga ggg gat ggt aac ggc 144
Arg Arg Arg Arg Arg Ala Ala Arg Cys Gly Gly Gly Asp Gly Asn Gly
        35                  40                  45
tgg gag atg gac gga gga ggg gac ccc aag aag cgg cgg ctc acc gat 192
Trp Glu Met Asp Gly Gly Gly Asp Pro Lys Lys Arg Arg Leu Thr Asp
    50                  55                  60
gag cag gcg gag atg ctg gag ctg agc ttc cgg gag gaa cgc aag ctg 240
Glu Gln Ala Glu Met Leu Glu Leu Ser Phe Arg Glu Glu Arg Lys Leu
65                  70                  75                  80
gag acc ggc cgg aag gtg tat ctg gcc gcc gag ctc ggg ctc gac ccc 288
Glu Thr Gly Arg Lys Val Tyr Leu Ala Ala Glu Leu Gly Leu Asp Pro
                85                  90                  95
aag cag gtc gcc gtg tgg ttc cag tac cgc cgg gcg cgc cac aag agc 336
Lys Gln Val Ala Val Trp Phe Gln Tyr Arg Arg Ala Arg His Lys Ser
            100                 105                 110
aag acg ctc gag cag gag ttc gcc agg ctc aag cac gcc cac gac gca 384
Lys Thr Leu Glu Gln Glu Phe Ala Arg Leu Lys His Ala His Asp Ala
        115                 120                 125
gcc atc ctc cac aaa tgc cac ctc gag aac gag gtg ctg agg ctg aag 432
Ala Ile Leu His Lys Cys His Leu Glu Asn Glu Val Leu Arg Leu Lys
    130                 135                 140
gag aag ctg aga gcg act gcg gag gag gtg cag cgc ctc agg tcg gca 480
Glu Lys Leu Arg Ala Thr Ala Glu Glu Val Gln Arg Leu Arg Ser Ala
145                 150                 155                 160
gct ggg atc cac gcc gat ggc gga gac tcc gct ggc gcc gtt agc gtg 528
Ala Gly Ile His Ala Asp Gly Gly Asp Ser Ala Gly Ala Val Ser Val
                165                 170                 175
tgc ggc ggg agc ccg cgc tca tcc ttc tcg acg gga acc tgc cag cag 576
Cys Gly Gly Ser Pro Arg Ser Ser Phe Ser Thr Gly Thr Cys Gln Gln
            180                 185                 190
cat ccg ggt ttc agc ggg gca gac gtg ctg ggg cgg gac gat gac ctg 624
His Pro Gly Phe Ser Gly Ala Asp Val Leu Gly Arg Asp Asp Asp Leu
        195                 200                 205
atg atg tgc gtc ccc gag tgg ttt ttc gca tga 657
Met Met Cys Val Pro Glu Trp Phe Phe Ala
    210                 215

<210> 58
<211> 218
<212> PRT
<213> Triticum aestivum
<400> 58
Met Asp Asn Gln Gln Leu Phe Gly Ser Ser Tyr Val Asp Thr Pro Phe
1               5                   10                  15
Phe Ala Ala Asn Gly Thr Ala Gln Arg Glu Ser Gln Pro Lys Ala Arg
            20                  25                  30
Arg Arg Arg Arg Arg Ala Ala Arg Cys Gly Gly Gly Asp Gly Asn Gly
        35                  40                  45
Trp Glu Met Asp Gly Gly Gly Asp Pro Lys Lys Arg Arg Leu Thr Asp
    50                  55                  60
Glu Gln Ala Glu Met Leu Glu Leu Ser Phe Arg Glu Glu Arg Lys Leu
65                  70                  75                  80
Glu Thr Gly Arg Lys Val Tyr Leu Ala Ala Glu Leu Gly Leu Asp Pro
                85                  90                  95
Lys Gln Val Ala Val Trp Phe Gln Tyr Arg Arg Ala Arg His Lys Ser
            100                 105                 110
Lys Thr Leu Glu Gln Glu Phe Ala Arg Leu Lys His Ala His Asp Ala
        115                 120                 125
Ala Ile Leu His Lys Cys His Leu Glu Asn Glu Val Leu Arg Leu Lys
    130                 135                 140
Glu Lys Leu Arg Ala Thr Ala Glu Glu Val Gln Arg Leu Arg Ser Ala
145                 150                 155                 160
Ala Gly Ile His Ala Asp Gly Gly Asp Ser Ala Gly Ala Val Ser Val

                165                 170                 175
Cys Gly Gly Ser Pro Arg Ser Ser Phe Ser Thr Gly Thr Cys Gln Gln
            180                 185                 190
His Pro Gly Phe Ser Gly Ala Asp Val Leu Gly Arg Asp Asp Asp Leu
        195                 200                 205
Met Met Cys Val Pro Glu Trp Phe Phe Ala
    210                 215

<210>  59
<211>  1406
<212>  DNA
<213>  Triticum aestivum
<400>  59
   cgcactttcc cctccttcaa ctagcgcttt gcggccggtg gtcctcccct cctcgatcca      60
   gttcctcacc acaccaacag cgcaacccac cgtgcctccc cttcctctaa tcttccaatt     120
   ccaaccatcg ttttctcccc tcctcagctc cgaacagccg gcatggacaa ccagcagctc     180
   tttggttcct cctacgtgga cgcgcctttc ttcgcggcca atggtacgtc cgacgccgcg     240
   cgcccccggt gtttgcgcat gcgatggtgc agctgcagca gcttcagttt caccggccag     300
   gacacgcatg tgatgccctt ttttccatgt cgtggttgta tgtgtgcagg cacggcgcag     360
   cgggagagcc agccgaaggc tcggcgcagg cggcggaagg ccgcgagatg cggcggaggg     420
   gatggtaacg gcggggagat ggacggagga ggggacccca agaagcggcg gctcaccgat     480
   gagcaggcag agatgctgga gctgagcttc cgggaggaac gcaagctgga gactggccgg     540
   aaggtgtatc tggccgccga gctcgggctc gaccccaagc aggtcgccgt gtggttccag     600
   aaccgccggg cgcgccacaa gagcaagacg ctcgagcagg agttcaccag gctcaagcac     660
   gctcacgacg cagacatcct ccacaaatgc cacctccaga acgaggtaag cttactcgca     720
   cacactcaca ctggcataca tatggcgccg tgcatatcag ttcctcttcg ttcttgcaca     780
   agagctgact gacacacata tggccaggtg ctgaggctga aggagaggct gagagcgact     840
   gaggaggagg tgcagcgcct caggtcggca gctgggagcc acggggcatc cggcgatggc     900
   ggagacgccg ctggcgccgt tagcgtgtgc ggcgggagcc caggctcgtc cttctcgacg     960
   ggaacctggc agcagcatct gggtttcagc ggggcaaacg agctggggac ggacgatgac    1020
   ctgatgatgt gcgtacccga ctgttttttc gcatgaatta gctagagttt atggtggcta    1080
   cgccgatagc agcgtgttcg agtgtttttt agcatgaaat aaaatctccc acaaaatagg    1140
   tgcgttaatt gcgctatagc tgctcgggag cctcgccgct atgctatggc tgccgccgcc    1200
   aaatcctcca caaatacctc caaagggctg cagaatcagg aaatacctct agaaatcatc    1260
   tgtcccgccg gataagttgt tgctatttgc tgtgattgcc tgctttgcgg ccggtatagc    1320
   tgctgagaga ggctgccact aaatcctttc tccggcgatt ttaatctttg gtttttagcc    1380
   tgtatatatg gggtgatttg aaattg                                          1406

<210>  60
<211>  666
<212>  DNA
<213>  Triticum aestivum
<220>
<221>  CDS
<222>  (1)..(666)
<223>
<400>  60
atg gac aac cag cag ctc ttt ggt tcc tcc tac gtg gac gcg cct ttc       48
Met Asp Asn Gln Gln Leu Phe Gly Ser Ser Tyr Val Asp Ala Pro Phe
1               5                   10                  15
ttc gcg gcc aat ggc acg gcg cag cgg gag agc cag ccg aag gct cgg       96
Phe Ala Ala Asn Gly Thr Ala Gln Arg Glu Ser Gln Pro Lys Ala Arg
            20                  25                  30
cgc agg cgg cgg aag gcc gcg aga tgc ggc gga ggg gat ggt aac ggc      144
Arg Arg Arg Arg Lys Ala Ala Arg Cys Gly Gly Gly Asp Gly Asn Gly
        35                  40                  45
ggg gag atg gac gga gga ggg gac ccc aag aag cgg cgg ctc acc gat      192
Gly Glu Met Asp Gly Gly Gly Asp Pro Lys Lys Arg Arg Leu Thr Asp
    50                  55                  60
gag cag gca gag atg ctg gag ctg agc ttc cgg gag gaa cgc aag ctg      240
Glu Gln Ala Glu Met Leu Glu Leu Ser Phe Arg Glu Glu Arg Lys Leu
65                  70                  75                  80
gag act ggc cgg aag gtg tat ctg gcc gcc gag ctc ggg ctc gac ccc      288

Glu Thr Gly Arg Lys Val Tyr Leu Ala Ala Glu Leu Gly Leu Asp Pro
                85                  90                  95
aag cag gtc gcc gtg tgg ttc cag aac cgc cgg gcg cgc cac aag agc       336
Lys Gln Val Ala Val Trp Phe Gln Asn Arg Arg Ala Arg His Lys Ser
            100                 105                 110
aag acg ctc gag cag gag ttc acc agg ctc aag cac gct cac gac gca       384
Lys Thr Leu Glu Gln Glu Phe Thr Arg Leu Lys His Ala His Asp Ala
        115                 120                 125
gac atc ctc cac aaa tgc cac ctc cag aac gag gtg ctg agg ctg aag       432
Asp Ile Leu His Lys Cys His Leu Gln Asn Glu Val Leu Arg Leu Lys
    130                 135                 140
gag agg ctg aga gcg act gag gag gag gtg cag cgc ctc agg tcg gca       480
Glu Arg Leu Arg Ala Thr Glu Glu Glu Val Gln Arg Leu Arg Ser Ala
145                 150                 155                 160
gct ggg agc cac ggg gca tcc ggc gat ggc gga gac gcc gct ggc gcc       528
Ala Gly Ser His Gly Ala Ser Gly Asp Gly Gly Asp Ala Ala Gly Ala
                165                 170                 175
gtt agc gtg tgc ggc ggg agc cca ggc tcg tcc ttc tcg acg gga acc       576
Val Ser Val Cys Gly Gly Ser Pro Gly Ser Ser Phe Ser Thr Gly Thr
            180                 185                 190
tgg cag cag cat ctg ggt ttc agc ggg gca aac gag ctg ggg acg gac       624
Trp Gln Gln His Leu Gly Phe Ser Gly Ala Asn Glu Leu Gly Thr Asp
        195                 200                 205
gat gac ctg atg atg tgc gta ccc gac tgt ttt ttc gca tga               666
Asp Asp Leu Met Met Cys Val Pro Asp Cys Phe Phe Ala
    210                 215                 220

<210>  61
<211>  221
<212>  PRT
<213>  Triticum aestivum
<400>  61
Met Asp Asn Gln Gln Leu Phe Gly Ser Ser Tyr Val Asp Ala Pro Phe
1               5                   10                  15
Phe Ala Ala Asn Gly Thr Ala Gln Arg Glu Ser Gln Pro Lys Ala Arg
            20                  25                  30
Arg Arg Arg Arg Lys Ala Ala Arg Cys Gly Gly Gly Asp Gly Asn Gly
        35                  40                  45
Gly Glu Met Asp Gly Gly Gly Asp Pro Lys Lys Arg Arg Leu Thr Asp
    50                  55                  60
Glu Gln Ala Glu Met Leu Glu Leu Ser Phe Arg Glu Glu Arg Lys Leu
65                  70                  75                  80
Glu Thr Gly Arg Lys Val Tyr Leu Ala Ala Glu Leu Gly Leu Asp Pro
                85                  90                  95
Lys Gln Val Ala Val Trp Phe Gln Asn Arg Arg Ala Arg His Lys Ser
            100                 105                 110
Lys Thr Leu Glu Gln Glu Phe Thr Arg Leu Lys His Ala His Asp Ala
        115                 120                 125
Asp Ile Leu His Lys Cys His Leu Gln Asn Glu Val Leu Arg Leu Lys
    130                 135                 140
Glu Arg Leu Arg Ala Thr Glu Glu Glu Val Gln Arg Leu Arg Ser Ala
145                 150                 155                 160
Ala Gly Ser His Gly Ala Ser Gly Asp Gly Gly Asp Ala Ala Gly Ala
                165                 170                 175
Val Ser Val Cys Gly Gly Ser Pro Gly Ser Ser Phe Ser Thr Gly Thr
            180                 185                 190
Trp Gln Gln His Leu Gly Phe Ser Gly Ala Asn Glu Leu Gly Thr Asp
        195                 200                 205
Asp Asp Leu Met Met Cys Val Pro Asp Cys Phe Phe Ala
    210                 215                 220

<210>  62
<211>  1533

<212>  DNA
<213>  Triticum aestivum
<400>  62
tcaactagcg ctttgcggcc ggtggtcctc ccctcgatcc agttcctcac cacaccaaga      60
gcgcaaccca ccgtgtctcc ccttcctcta atcttccaat tccaaccatc ccttctctcc     120
cctcctcagc tccgaacgcc aggcatggac aaccagcagc tctttggttc ctcctacgtg     180
gacacgtctt tcttcgcggc caatggtacg tacgacgccg cccttcaact agcgctttgc     240
ggccggtggt cctcccctcg atccagttcc tcaccacacc aagagcgcaa cccaccgtgt     300
ctccccttcc tctaatcttc caattccaac catcccttct ctcccctcct cagctccgaa     360
cgccaggcat ggacaaccag cagctctttg gttcctccta cgtggacacg tctttcttcg     420
cggccaatgg tacgtacgac gccgcccccc cccccccccg gtgtttgcgc atgcgatgat     480
gcagctgcag tagcttcatt ttcaccggcc aggacacgca tgtgatgccc tttttttttc     540
catgtcgtgg ttgtatgtgt gcaggcacgg cgcagcggga gagtcagccg aaggctcggc     600
gcaggcggcg gagggccgcg agatgcggcg gaggggatgg taacggcggg gagatggacg     660
aaggagggga ccccaagaag cggcggctca ccgatgagca ggcggagatg ctggagctga     720
gcttccggga ggagcgcaag ctggagactg gccggaaggt gtatctggcc gccgagctcg     780
ggctcgaccc caagcaggtc gccgtgtggt tccagaaccg ccgggcgcgc cacaagagca     840
agacgctcga gcaggagttc gccaggctca agcacgccca cgacgcagac atcctccaca     900
aatgccacct cgagaacgag gtaagcttgc tcgcacacac acacacgggc gtacatatca     960
attcctcttc gttcttgcac aagctgactg acacccagct gctgaggctg aaggagaagc    1020
tgagagcgac tgaggaggag gtgcagcgcc tcaggtcggc agctgggtgc cacggggcat    1080
ccgccgatgg cggagactca gctggcgccg ttagcgtgtg cggcgggagc ccgggctcgt    1140
tcttctcgac gggaacctgc cagcagcatc cgggtttcag cggggcagac gtgctgaggc    1200
cggacgatga cctgatgatg tgcgtccccg agtggttttt cgcatgaatt acctagagtt    1260
tatggtggct acgccgatag cagcgtgttc gagtgttttt tatcatgaaa taaaatctcc    1320
cacaaaatag gcgctataac tgctcggaag cctcgccgct atgctatggc tgcatgccgc    1380
cgccaaatcc tccacaaatc ccttcaaagg cctgctatgc ggtcgctata gctgctggaa    1440
aaggccgccg ctaaatcgtt tctcccacga ttttaatctt tggtttttag cctgtatata    1500
tggggtgatt tgaaattgac acgagctgga caa                                 1533

<210>  63
<211>  666
<212>  DNA
<213>  Triticum aestivum
<220>
<221>  CDS
<222>  (1)..(666)
<223>
<400>  63
atg gac aac cag cag ctc ttt ggt tcc tcc tac gtg gac acg tct ttc      48
Met Asp Asn Gln Gln Leu Phe Gly Ser Ser Tyr Val Asp Thr Ser Phe
1               5                   10                  15
ttc gcg gcc aat ggc acg gcg cag cgg gag agt cag ccg aag gct cgg      96
Phe Ala Ala Asn Gly Thr Ala Gln Arg Glu Ser Gln Pro Lys Ala Arg
            20                  25                  30
cgc agg cgg cgg agg gcc gcg aga tgc ggc gga ggg gat ggt aac ggc     144
Arg Arg Arg Arg Arg Ala Ala Arg Cys Gly Gly Gly Asp Gly Asn Gly
        35                  40                  45
ggg gag atg gac gaa gga ggg gac ccc aag aag cgg cgg ctc acc gat     192
Gly Glu Met Asp Glu Gly Gly Asp Pro Lys Lys Arg Arg Leu Thr Asp
    50                  55                  60
gag cag gcg gag atg ctg gag ctg agc ttc cgg gag gag cgc aag ctg     240
Glu Gln Ala Glu Met Leu Glu Leu Ser Phe Arg Glu Glu Arg Lys Leu
65                  70                  75                  80
gag act ggc cgg aag gtg tat ctg gcc gcc gag ctc ggg ctc gac ccc     288
Glu Thr Gly Arg Lys Val Tyr Leu Ala Ala Glu Leu Gly Leu Asp Pro
                85                  90                  95
aag cag gtc gcc gtg tgg ttc cag aac cgc cgg gcg cgc cac aag agc     336
Lys Gln Val Ala Val Trp Phe Gln Asn Arg Arg Ala Arg His Lys Ser
            100                 105                 110
aag acg ctc gag cag gag ttc gcc agg ctc aag cac gcc cac gac gca     384
Lys Thr Leu Glu Gln Glu Phe Ala Arg Leu Lys His Ala His Asp Ala
        115                 120                 125

gac atc ctc cac aaa tgc cac ctc gag aac gag ctg ctg agg ctg aag     432
Asp Ile Leu His Lys Cys His Leu Glu Asn Glu Leu Leu Arg Leu Lys
    130                 135                 140
gag aag ctg aga gcg act gag gag gag gtg cag cgc ctc agg tcg gca     480
Glu Lys Leu Arg Ala Thr Glu Glu Glu Val Gln Arg Leu Arg Ser Ala
145                 150                 155                 160
gct ggg tgc cac ggg gca tcc gcc gat ggc gga gac tca gct ggc gcc     528
Ala Gly Cys His Gly Ala Ser Ala Asp Gly Gly Asp Ser Ala Gly Ala
                165                 170                 175
gtt agc gtg tgc ggc ggg agc ccg ggc tcg ttc ttc tcg acg gga acc     576
Val Ser Val Cys Gly Gly Ser Pro Gly Ser Phe Phe Ser Thr Gly Thr
            180                 185                 190
tgc cag cag cat ccg ggt ttc agc ggg gca gac gtg ctg agg ccg gac     624
Cys Gln Gln His Pro Gly Phe Ser Gly Ala Asp Val Leu Arg Pro Asp
        195                 200                 205
gat gac ctg atg atg tgc gtc ccc gag tgg ttt ttc gca tga             666
Asp Asp Leu Met Met Cys Val Pro Glu Trp Phe Phe Ala
    210                 215                 220

<210>  64
<211>  221
<212>  PRT
<213>  Triticum aestivum
<400>  64
Met Asp Asn Gln Gln Leu Phe Gly Ser Ser Tyr Val Asp Thr Ser Phe
1               5                   10                  15
Phe Ala Ala Asn Gly Thr Ala Gln Arg Glu Ser Gln Pro Lys Ala Arg
            20                  25                  30
Arg Arg Arg Arg Arg Ala Ala Arg Cys Gly Gly Gly Asp Gly Asn Gly
        35                  40                  45
Gly Glu Met Asp Glu Gly Gly Asp Pro Lys Lys Arg Arg Leu Thr Asp
    50                  55                  60
Glu Gln Ala Glu Met Leu Glu Leu Ser Phe Arg Glu Glu Arg Lys Leu
65                  70                  75                  80
Glu Thr Gly Arg Lys Val Tyr Leu Ala Ala Glu Leu Gly Leu Asp Pro
                85                  90                  95
Lys Gln Val Ala Val Trp Phe Gln Asn Arg Arg Ala Arg His Lys Ser
            100                 105                 110
Lys Thr Leu Glu Gln Glu Phe Ala Arg Leu Lys His Ala His Asp Ala
        115                 120                 125
Asp Ile Leu His Lys Cys His Leu Glu Asn Glu Leu Leu Arg Leu Lys
    130                 135                 140
Glu Lys Leu Arg Ala Thr Glu Glu Glu Val Gln Arg Leu Arg Ser Ala
145                 150                 155                 160
Ala Gly Cys His Gly Ala Ser Ala Asp Gly Gly Asp Ser Ala Gly Ala
                165                 170                 175
Val Ser Val Cys Gly Gly Ser Pro Gly Ser Phe Phe Ser Thr Gly Thr
            180                 185                 190
Cys Gln Gln His Pro Gly Phe Ser Gly Ala Asp Val Leu Arg Pro Asp
        195                 200                 205
Asp Asp Leu Met Met Cys Val Pro Glu Trp Phe Phe Ala
    210                 215                 220

<210>  65
<211>  21
<212>  DNA
<213>  Artificial
<220>
<223>  Artificially synthesized primer sequence
<400>  65
tgtgcgtccc cgagtggttt t                                              21

<210>  66

<211>  24
<212>  DNA
<213>  Artificial
<220>
<223>  Artificially synthesized primer sequence
<400>  66
acaaaatagg cgctatagct gctc                                              24

<210>  67
<211>  24
<212>  DNA
<213>  Artificial
<220>
<223>  Artificially synthesized primer sequence
<400>  67
cgggacagat gatttctaga ggtt                                              24

<210>  68
<211>  21
<212>  DNA
<213>  Artificial
<220>
<223>  Artificially synthesized primer sequence
<400>  68
acaaaatagg tgcgttaatt g                                                 21

<210>  69
<211>  21
<212>  DNA
<213>  Artificial
<220>
<223>  Artificially synthesized primer sequence
<400>  69
ggtatttcct gattctgcag c                                                 21

<210>  70
<211>  20
<212>  DNA
<213>  Artificial
<220>
<223>  Artificially synthesized primer sequence
<400>  70
gctatgctat ggctgcatgc                                                   20

<210>  71
<211>  20
<212>  DNA
<213>  Artificial
<220>
<223>  Artificially synthesized primer sequence
<400>  71
gatttagcgg cggccttttc                                                   20

<210>  72
<211>  21
<212>  DNA
<213>  Artificial
<220>
<223>  Artificially synthesized primer sequence
<400>  72
cgactgggca gatgaacatg g                                                 21

<210>  73

<211>  21
<212>  DNA
<213>  Artificial
<220>
<223>  Artificially synthesized primer sequence
<400>  73
tttccccgtt gactgcctct t                                                21

<210>  74
<211>  21
<212>  DNA
<213>  Artificial
<220>
<223>  Artificially synthesized primer sequence
<400>  74
gccgacgcga agcgggtaga t                                                21

<210>  75
<211>  21
<212>  DNA
<213>  Artificial
<220>
<223>  Artificially synthesized primer sequence
<400>  75
gcgcgttggc ggtaacaaga a                                                21

<210>  76
<211>  19
<212>  DNA
<213>  Artificial
<220>
<223>  Artificially synthesized primer sequence
<400>  76
gtctgcacca tcgtcaacc                                                   19

<210>  77
<211>  20
<212>  DNA
<213>  Artificial
<220>
<223>  Artificially synthesized primer sequence
<400>  77
gaagtccagc tgccagaaac                                                  20

## Description

### [Technical Field]

The present invention relates to a wheat with an increased number of grains and a propagation material of the wheat. The present invention also relates to a processed product made from the seeds of the wheat with an increased number of grains. The present invention further relates to a method for producing the wheat with an increased number of grains. The present invention also relates to a progeny and clone of the wheat produced by the method. The present invention further relates to an agent for increasing the number of wheat grains.

### [Background Art]

In 1960s, when the global food crisis was concerned, high-yielding wheat and the like resistant to heavy fertilization in short stems were grown and the rapid spread of such varieties avoided the global food crisis. It is so-called "Green Revolution." However, the use of huge amounts of chemical fertilizers resulted in soil deterioration, and furthermore the use of agrochemicals to control the increase of weeds due to heavy fertilization polluted soil and water. Therefore, further yield increase from heavy fertilization cannot be expected, thereby causing problems of limitation of yields and the like.

While the world population continues to increase explosively, the rapid decrease of arable land occurs because of environmental pollution, global warming, and desertification, so that the chronic food shortage still continues mainly in developing countries. In particular, wheat is the most demanded grain in the world because it is widely used not only for bread, Japanese noodle udon, Chinese noodle, pasta, cereal, and confectionery, but also for materials of alcohols such as beer, as well as livestock feed and the like. In China and India, which are countries with the first and second largest population, dietary habits are changing with rapid economic growth to cause global outpacing of demand over production. This results in a tendency to decrease the year-end inventory of wheat and thus a tendency to increase the international market price. Therefore, there is urgent need for development of a novel method for increasing the yield of grains and the like independently of high-yielding wheat and the like resistant to heavy fertilization in short stems.

An important factor to determine the grain yield per plant at the harvest stage is the structure of inflorescence. In particular, flowers (florets) of grasses, such as wheat (Triticum monococcum, Triticum aestivum), barley (Hordeum vulgare), rice (Oryza sativa), and maize (Zea mays), emerge in differentiated short shoots, called spikelets. Accordingly, the number of fertile spikelets per inflorescence or the number of fertile florets in the spikelets closely relates to the grain yield.

Regarding such grasses, identification of genes associated with the grain yield has been intensively studied. For rice, genes responsible for some major quantitative trait locus (QTLs) are cloned, such as Grain number 1a (Gn1a) encoding cytokinin oxidase, and WEALTHY FARMERS PANICLE 1 (WFP1) encoding SQUAMOSA PROMOTER BINDING PROTEIN-LIKE 14 (SPL14). For wheat, however, genes associated with major QTLs or the grain yield per inflorescence have not been cloned yet, except for Vrs1 derived from barley (six-rowed spike 1) and intermedium spike-c (int-c) (PTL 1 and NPLs 1 to 3).

Barley is a plant classified into Hordeum. The inflorescence of barley is called spike, including three spikelets (one central spikelet, two lateral spikelets) per node. However, the plants belonging to Triticum bear a plurality of florets per spikelet, whereas all the 31 varieties belonging to Hordeum have the unique characteristic of bearing only one floret per spikelet.

Furthermore, barley is roughly classified into two-rowed and six-rowed based on the shape of the spike. In two-rowed spikes (two-row spikes), only central spikelets are fertile to develop grains. In six-rowed spikes (six-row spikes), on the other hand, three spikelets are fertile to develop grains. Six-rowed barley accordingly bears a tree-times larger amount of grains than two-rowed barley.

The row of barley is controlled by the Vrs1 gene present in the long arm of chromosome 2H. The wild-type Vrs1 gene encodes a homeodomain leucine zipper (HD-Zip) I transcription factor, and the expression of the Vrs1 gene in floret primordia of lateral spikelets controls the development of lateral spikelets to cause two- rowed spikes of barley or so, as found by the present inventors (PTL 1 and NPLs 1 and 3).

In barley, however, the molecular function or expression pattern of the VRS1 protein has not been well understood yet, and whether loss of the function of the Vrs1 gene in two-row spikes recovers the fertility of lateral spikelets was unknown. Although barley and wheat are plants of the same Poaceae family as described above, they belong to different genera. The spikelets of barley and wheat have very different shapes and, of course, wheat has no six-rowed or two-rowed spikelets and has no lateral spikelets either. Accordingly, the presence of DNA and the like capable of giving the fertility to sterile florets has not been revealed in wheat. Therefore, a method for increasing the yield of wheat, particularly a method for increasing the number of grains by increasing the number of fertile florets per spikelet of wheat independently of heavy fertilization has not been developed yet.

### [Citation List]

### [Patent Literature]

[PTL 1] Japanese Unexamined Patent Application Publication No. 2007-159479

### [Non Patent Literature]

[NTL 1] Komatsuda, T. et al., Proc Natl Acad Sci USA, 2007, volume 104, 1424-1429 pages
[NPL 2] Ramsay, L. et al., Nat Genet, 2011, volume 43, 169-172 pages
[NPL 3] Gottwald, S. et al., BMC Research Notes, 2009, volume 2, 258-272 pages

### [Summary of Invention]

### [Technical Problem]

The present invention has been made in consideration of the above-described problems in the conventional techniques. An object of the present invention is to provide a method, an agent, and the like which can increase the number of fertile florets per spikelet of wheat and thus can increase the number of grains.

### [Solution to Problem]

The present inventors detected the expression of the Vrs1 gene and the VRS1 protein in the floret primordium organs of barley which were more developed than organs around at the glume primordium stage at which the expression of the barley Vrs1 gene was observed before (PTL 1 and NPL 1). As a result, it was found that the Vrs1 gene and the VRS1 protein were highly expressed in the florets, particularly pistils, in lateral spikelets at the white anther stage. It was also found that, in two-rowed barley, the suppression of the expression of the wild-type Vrs1 gene by RNAi recovered the fertility of sterile lateral spikelets in two-row spikes to increase the number of grains.

However, described above is the function of the Vrs1 gene found in barley. Barley and wheat are plants of the same Poaceae family, but they belong to different genera. Unlike plants belonging to Triticum, such as wheat, barley has the unique characteristic of bearing only one floret per spikelet. In contrast, wheat and the like bear a plurality of florets per spikelet.

Therefore, barley and wheat are both Poaceae plants, but are very different in shape of spikelets. Of course, wheat has no six-rowed or two-rowed spikelets, and has no lateral spikelets either. Accordingly, the relation between the sterility of florets and the Vrs1 gene was not considered at all in wheat (Sakuma, S, et al. , Plant Cell Physiol, 2011, volume 52, 738-749 pages).

The present inventors isolated wheat Vrs1 genes (TmVrs1 gene, TaVrs1-A gene, TaVrs1-B gene, and TaVrs1-D gene). As a result, the amino acid sequences of the proteins encoded by the wheat Vrs1 genes had a high homology to the amino acid sequence of the barley VRS1, and the homeodomain (HD) motif and leucine zipper (LZ) motif of the barley VRS1 were well conserved in the wheat VRS1. Accordingly, wheat VRS1 was found to have similar biological functions to the barley VRS1.

The expression of the genes at the floret primordium stage of wheat was analyzed by the RNA in situ hybridization method. As a result, it was found that the genes were specifically expressed in upper florets that are usually expected to degenerate and become sterile, in other words, the Vrs1 genes are specifically expressed in pistils of upper florets in wheat, thereby inhibiting development of upper florets to make upper florets sterile. Therefore, the suppression of the function of the wheat Vrs1 genes can cause grain bearing in upper florets whose development is originally expected be inhibited. The above results lead to completion of the present invention including a method for increasing the number of wheat grains by artificially suppressing the function of the endogenous wheat Vrs1 genes. The present inventors have further confirmed that the number of florets and the number of grains per spikelet of wheat can be increased by suppressing the function of the wheat Vrs1 genes with RNAi.

Specifically, the present invention provides the followings.
<1> A wheat with an increased number of grains as compared with a control wheat, wherein the function of an endogenous wheat Vrs1 gene is artificially suppressed.
<2> The wheat according to <1>, wherein the function of the endogenous wheat Vrs1 gene is artificially suppressed by a double-stranded RNA method, an antisense method, or a ribozyme method.
<3> The wheat according to <1>, wherein the function of the endogenous wheat Vrs1 gene is artificially suppressed by introducing one or more mutations into the gene.
<4> A propagation material of the wheat according to any one of <1> to <3>.
<5> A processed product made from seeds of the wheat according to any one of <1> to <3>.
<6> A method for producing a wheat with an increased number of grains as compared with a control wheat, the method comprising a step of artificially suppressing the function of the endogenous wheat Vrs1 gene.
<7> A progeny wheat or cloned wheat of the wheat produced by the method according to <6>, wherein the function of the endogenous wheat Vrs1 gene is artificially suppressed to increase the number of grains as compared with a control wheat.
<8> A agent for increasing the number of wheat grains, the agent comprising as an active ingredient at least one DNA selected from the group consisting of (a) to (c) below or a vector having the inserted DNA:
   (a) a DNA encoding a double-stranded RNA complementary to the transcript of the wheat Vrs1 gene;
   (b) a DNA encoding an antisense RNA complementary to the transcript of the wheat Vrs1 gene;
   (c) a DNA encoding a RNA having a ribozyme activity to specifically cleave the transcript of the wheat Vrs1 gene.

### [Advantageous Effects of Invention]

The present invention can increase the number of fertile florets per spikelet of wheat and thus can increase the number of grains and the yield.

### [Brief Description of Drawings]

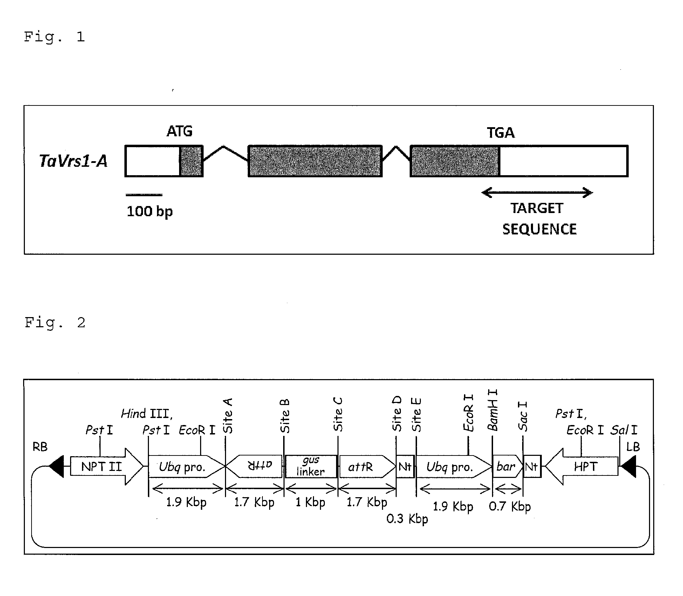

[Fig. 1] Fig. 1 is a schematic diagram illustrating the structure of the wheat Vrs1 gene (genomic DNA of TaVrs1-A gene). In the figure, "ATG" and "TGA" represent the positions of the start codon and the stop codon, respectively. The "target sequence" represents the position of the target sequence of a siRNA suitable for suppressing the expression of the TaVrs1-A gene, TaVrs1-B gene, and TaVrs1-D.
[Fig. 2] Fig. 2 is a schematic diagram illustrating the structure of a vector (pANDA-β vector) suitable for expressing the double-stranded RNA complementary to the transcript of the wheat Vrs1 gene.

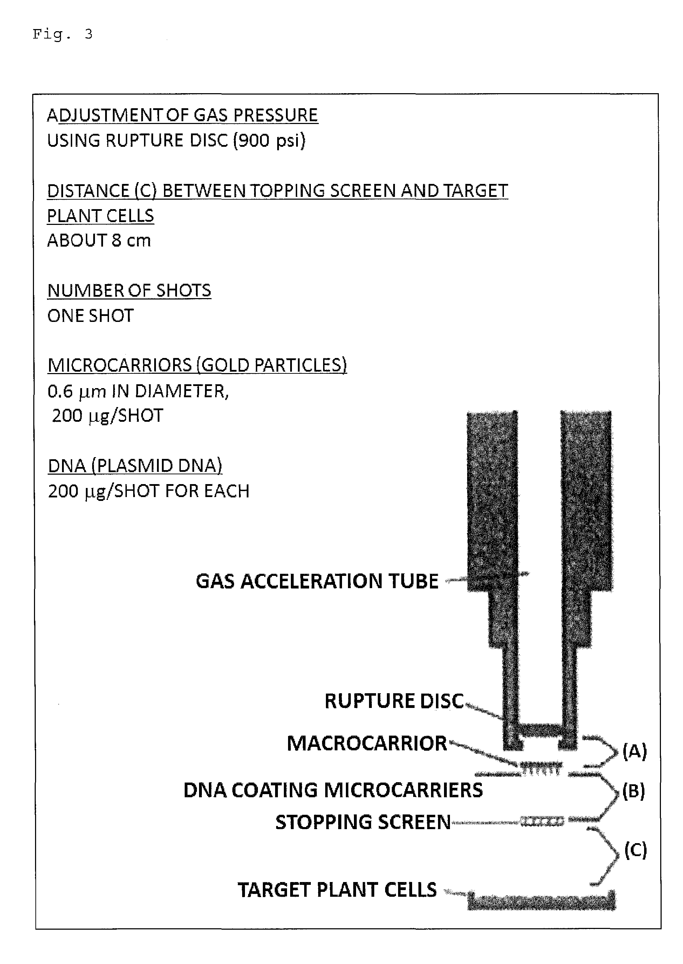

[Fig. 3] Fig. 3 is a figure describing the conditions of the particle bombardment method suitable for introducing into wheat cells (target plant cells) the vector having the inserted DNA encoding the double-stranded RNA complementary to the transcript of the wheat Vrs1 gene.

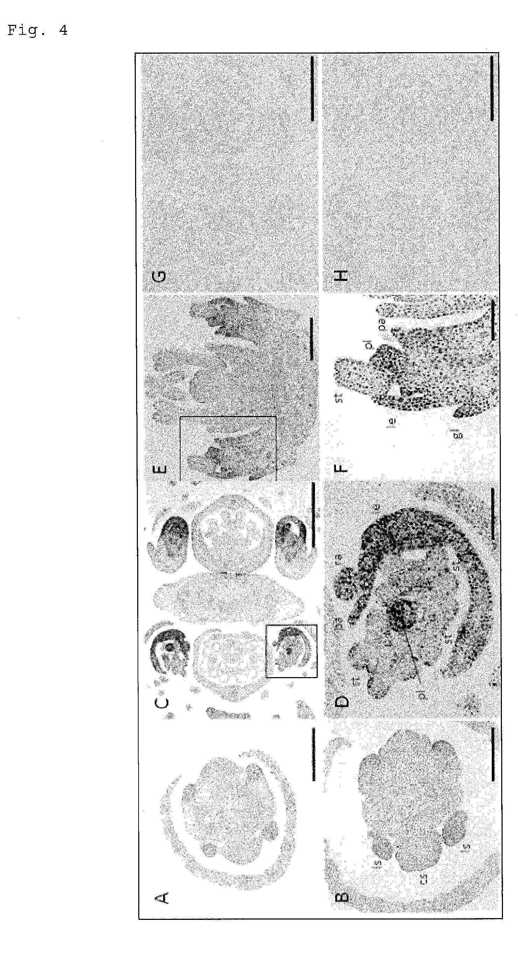

[Fig. 4] Fig. 4 is micrographs illustrating the results of the detected localization of the barley Vrs1 mRNA in barley immature spikes by RNA in situ hybridization. A illustrates the result of the observed transverse section of two-row spike barley Bonus cv. at the triple-mound stage. B illustrates the result of the observed transverse section at the glume primordium stage. C illustrates the result of the observed transverse section at the white anther stage. D illustrates the result of the observed lateral spikelet part enclosed in the rectangle in C at high magnification. E illustrates the result of the observed longitudinal section at the white anther stage. F illustrates the result of the observed lateral spikelet part enclosed in the rectangle in E at high magnification. G illustrates the result of the observed transverse section at the white anther stage after the hybridization with a sense RNA (negative control). H illustrates the result of the observed six-row spike mutant completely deficient in the Vrs1 gene at the white anther stage. In the figure, "cs" indicates a central spikelet, "ls" a lateral spikelet, "le" a lemma, "pa" an palea, "st" a stamen, "pi" a pistil, and "ra" a rachis, respectively. The scale bars indicate 200 µm in A, B, and E, 500 µm in C, G and H, and 100 µm in D and F. Barley spikes mature through the developmental stages of triple-mound stage, glume primordium stage, lemma primordium stage, stamen primordium stage, awn primordium stage, white anther stage, green anther stage, yellow anther stage, and flag-leaf stage.

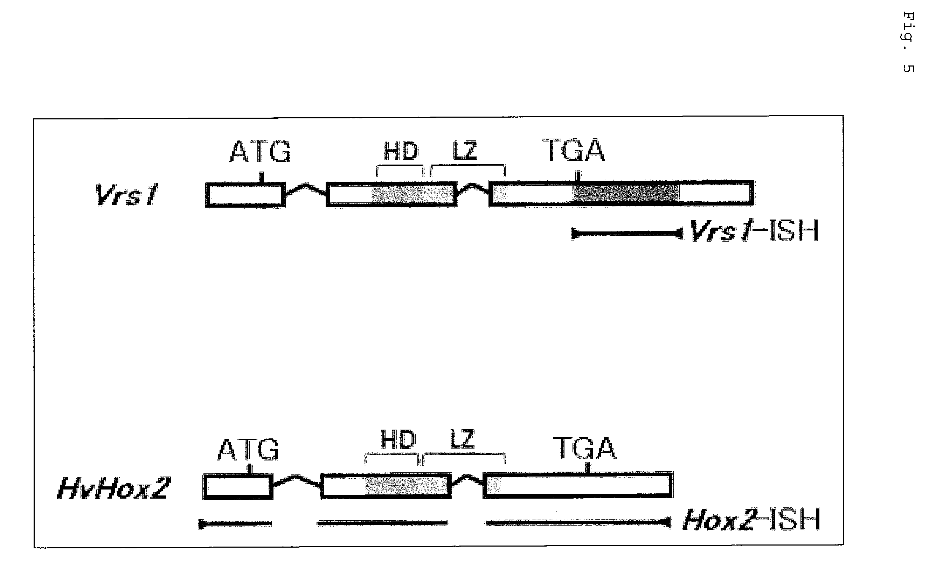

[Fig. 5] Fig. 5 is a schematic diagram illustrating the structures of genomic DNAs of the barley Vrs1 gene and the HvHox2 gene. In the figure, "ATG" and "TGA" represent the positions of the start codon and the stop codon, respectively. "ISH" represents the position of the probe used for detecting each gene by RNA in situ hybridization. In particular, "Vrs1-ISH" is a sequence specific to the Vrs1 gene. "HD" and "LZ" represent a homeodomain motif and a leucine zipper motif, respectively. The start end and the stop end represent the transcription start point and the polyadenylation site in each gene, respectively.

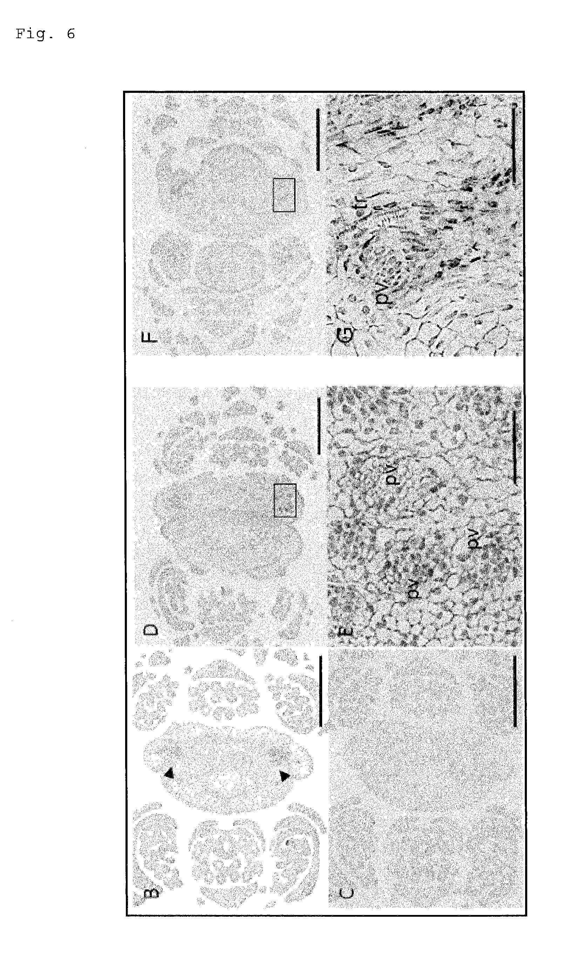

[Fig. 6] Fig. 6 is micrographs illustrating the results of the detected localization of HvHox2 mRNA in barley immature spikes of a hex-v.3 mutant (Vrs1 gene knockout mutant) by RNA in situ hybridization. B, D, and F illustrate the results of the observed immature spikes at the white anther stage after the hybridization with an antisense probe. E and G illustrate the results of the observed regions enclosed in the rectangle in D and F at high magnification, respectively. C illustrates the result of the observed immature spike at the white anther stage after the hybridization with a sense probe in the immature spike. The triangles in B indicate the signal of HvHox2 mRNA. "pv" represents provascular tissue cells and "tr" represents tracheids. The scale bars indicate 500 µm in B, C, D, and F, and indicate 50 µm in E and G.

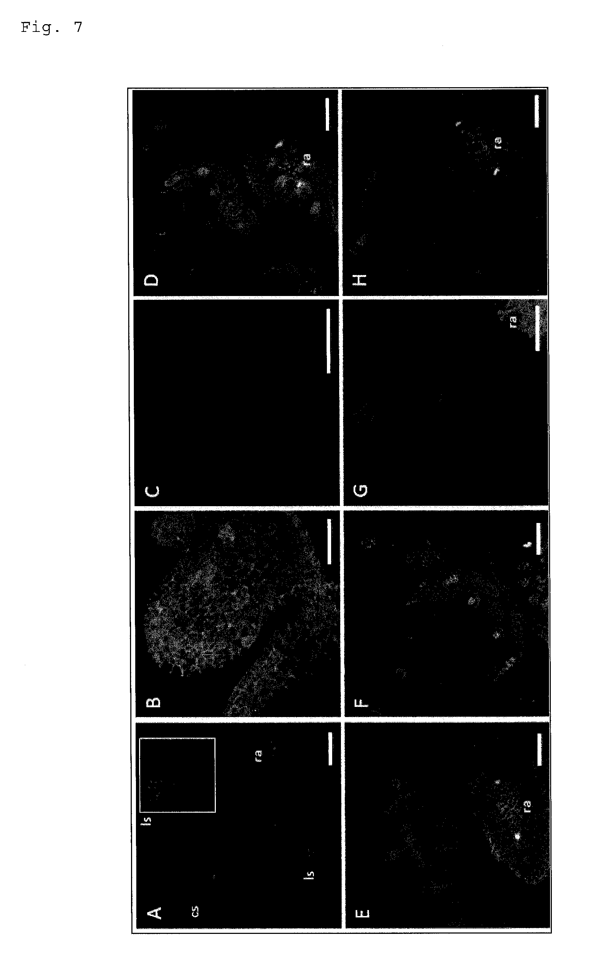

[Fig. 7] Fig. 7 is fluorescence microscope photographs illustrating the results of immunostaining of barley immature spikes using an anti-barley VRS1 antibody. A to D illustrate the results of the observed immature spikes of two-rowed barley cv. Bonus at the awn primordium stage. B illustrates the result of the observed lateral spikelet part enclosed in the rectangle in A at high magnification. C illustrates the result of costaining with the anti-VRS1 antibody and DAPI. D illustrates the result of no staining with the antibody or DAPI (negative control). E illustrates the result of the observed immature spike of a hex-v.3 six-row spike mutant (Vrs1 gene knockout) at the awn primordium stage. F illustrates the result of the observed hex-v.3 six-row spike mutant at the white anther stage. G illustrates the result of the observed hex-v.3 six-row spike mutant at the awn primordium stage, where the mutant was labeled with the anti-VRS1 antibody and further stained with DAPI. H illustrates the result of no staining with the antibody or DAPI (negative control). "cs" represents a central spikelet, "ls" represents a lateral spikelet, and "ra" represents a rachis. The scale bars indicate 100 µm in A and D to H, and indicates 50 µm in B and C.

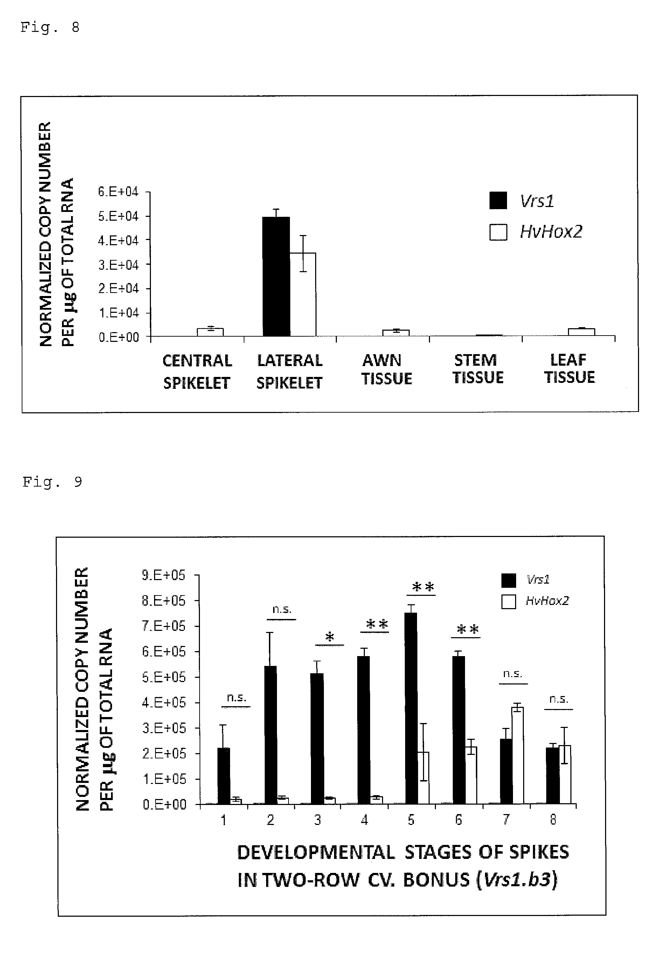

[Fig. 8] Fig. 8 is a graph illustrating the results of quantitative RT-PCR analysis on the organ-specific expression of the barley Vrs1 gene and HvHox2 gene. Each organ was isolated from a plant of Golden Promise at the flag-leaf stage or at the green anther stage of the spike. The values indicated in the figure are the means ± standard errors of the values obtained from biological triplicates (using a bulk of 50 mg F.W. (weight of biological tissues) per replication). In the quantitative RT-PCR, constitutively-expressed HvActin gene was used as a control gene. As a result of the analysis by the quantitative RT-PCR, there was no significant difference at the 5% probability level between the mean of the expression of the Vrs1 gene and the mean of the expression of the HvHox2 gene in lateral spikelets.
[Fig. 9] Fig. 9 is a graph illustrating the results of quantitative RT-PCR analysis on the transcription level of the barley Vrs1 gene and the HvHox2 gene in spikes (two-rowed barley cv. Bonus (Vrs1.b3 allele)) during the developmental stages. In the quantitative RT-PCR analysis, the RNA level was normalized using constitutively-expressed HvActin gene. The numbers along the abscissa of the graph indicates the following developmental stages targeted for the analysis, respectively. 1: triple-mound stage, 2: glume primordium stage, 3: lemma primordium stage, 4: stamen primordium stage, 5: awn primordium stage, 6: white anther stage, 7: green anther stage, and 8: yellow anther stage. The values indicated in the figure are the means ± standard errors of the values obtained from biological triplicates (using a bulk of 50 mg F.W. (weight of biological tissues) per replication). "**" and "*" indicate a significant difference at the 1% probability level and a significant difference at the 5% probability level, respectively, and "n.s." indicates no significant difference.

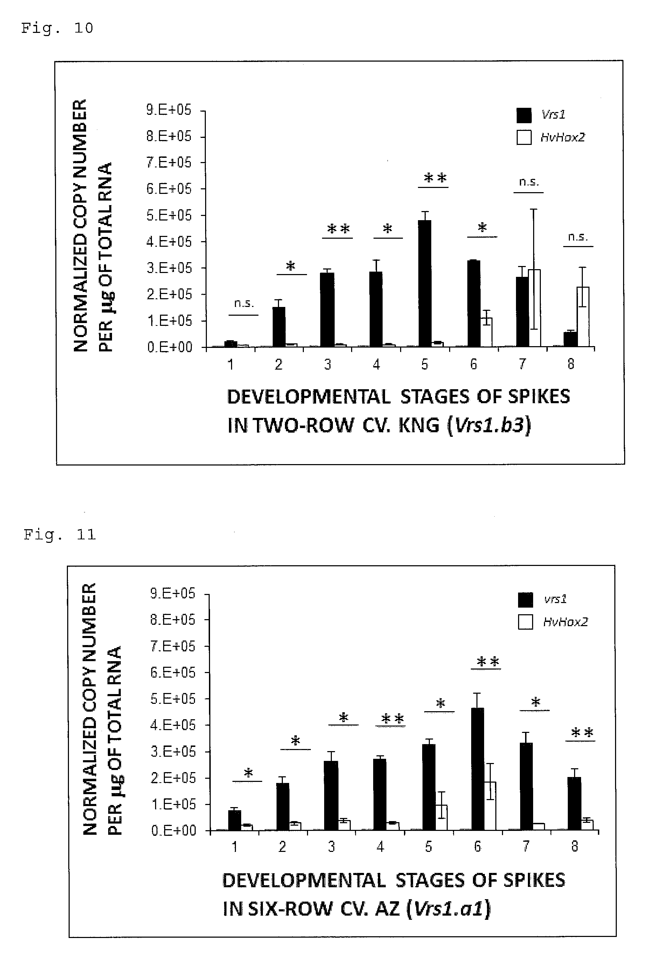

[Fig. 10] Fig. 10 is a graph illustrating the results of quantitative RT-PCR analysis on the transcription level of the barley Vrs1 gene and the HvHox2 gene in spikes (two-rowed barley cv. KNG (Vrs1.b3 allele)) during the developmental stages. The symbols and the like in the figure are the same as those in Fig. 9.
[Fig. 11] Fig. 11 is a graph illustrating the results of quantitative RT-PCR analysis on the transcription level of the barley Vrs1 gene and the HvHox2 gene in spikes (six-rowed barley cv. AZ (Vrs1.al allele)) during the developmental stages. The symbols and the like in the figure are the same as those in Fig. 9.

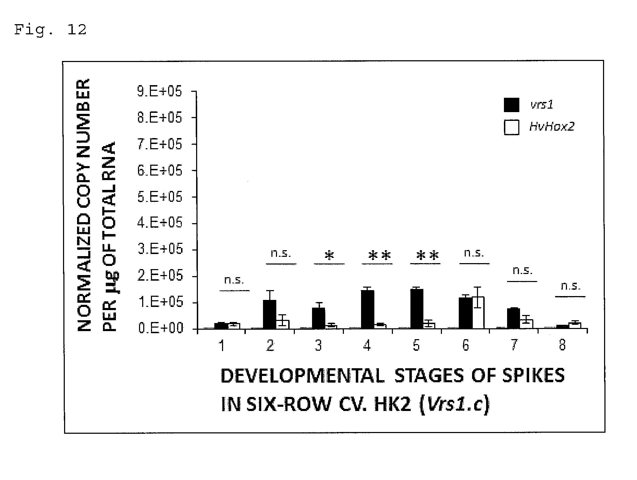

[Fig. 12] Fig. 12 is a graph illustrating the results of quantitative RT-PCR analysis on the transcription level of the barley Vrs1 gene and the HvHox2 gene in spikes (six-rowed barley cv. HK2 (Vrs1.cl allele)) during the developmental stages. The symbols and the like in the figure are the same as those in Fig. 9.

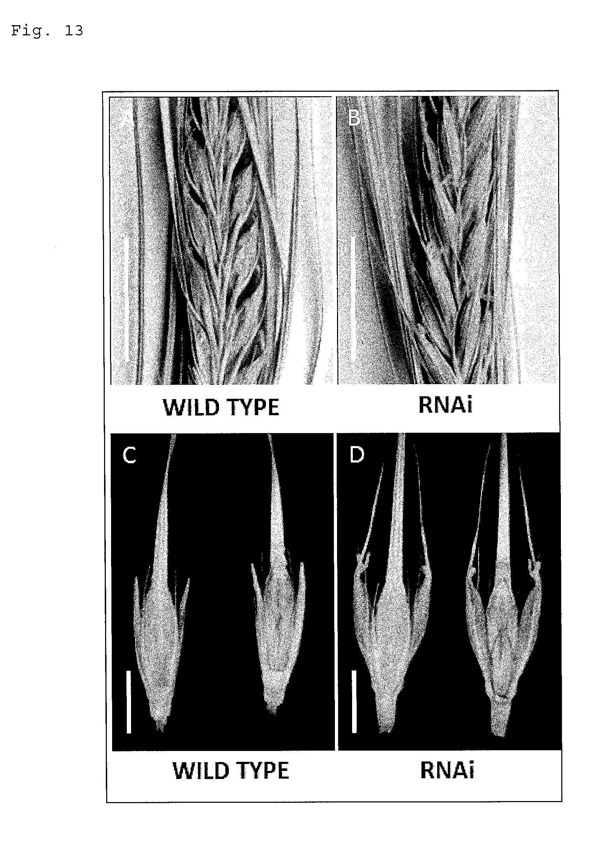

[Fig. 13] Fig. 13 is photographs illustrating the results of the analysis on a transgenic barley in which the barley Vrs1 gene is knocked down by RNAi. A and C indicate that development of lateral spikelets is suppressed in wild-type cv. Golden Promise. In contrast, B and D indicate that lateral spikelets develop to bear grains in an RNAi transgenic plant against the Vrs1 gene (BG87/1E18, T1 generation). In A and B, the scale bars indicate 10 mm. In C and D, the scale bars indicate 5 mm.

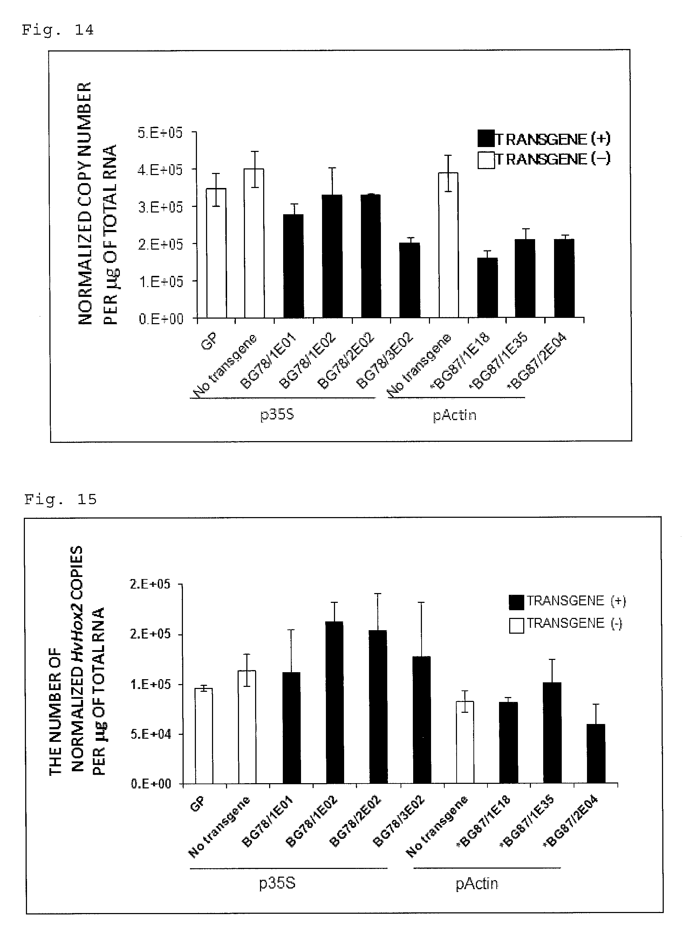

[Fig. 14] Fig. 14 is a graph illustrating the results of the analysis on the expression level of the barley Vrs1 gene by quantitative RT-PCR. Specifically, the graph illustrates the results of the analysis on immature spikes at the white anther stage of transgenic plants and non-transgenic plants (T1 generation). The values indicated in the figure are the means ± standard errors of the values obtained from biological triplicates (using a bulk of 50 mg F.W. (weight of biological tissues) per replication). The wild-type plant ("GP" in the figure) and the non-transgenic plants ("No transgene" in the figure) separated from T1 generation were used as control plants. In the figure, the white bars indicate the results of plant individuals to which no RNAi is introduced, and the black bars indicate the results of plant individuals to which RNAi is introduced.
[Fig. 15] Fig. 15 is a graph illustrating the results of the quantitative RT-PCR analysis on the expression level of the HvHox 2 gene in transgenic lines in which the expression of the barley Vrs1 gene is suppressed. Specifically, it is a graph illustrating the results of the analysis on immature spikes at the white anther stage of non-transgenic plants (T1 generation) and transgenic plants having RNAi specific to the Vrs1 gene whose expression is under the control of a CaMV 35S (p35S) promoter or rice actin 1 (pActin) promoter. The values indicated in the figure are the means ± standard errors of the values obtained from biological triplicates (using a bulk of 50 mg F.W. (weight of biological tissues) per replication).

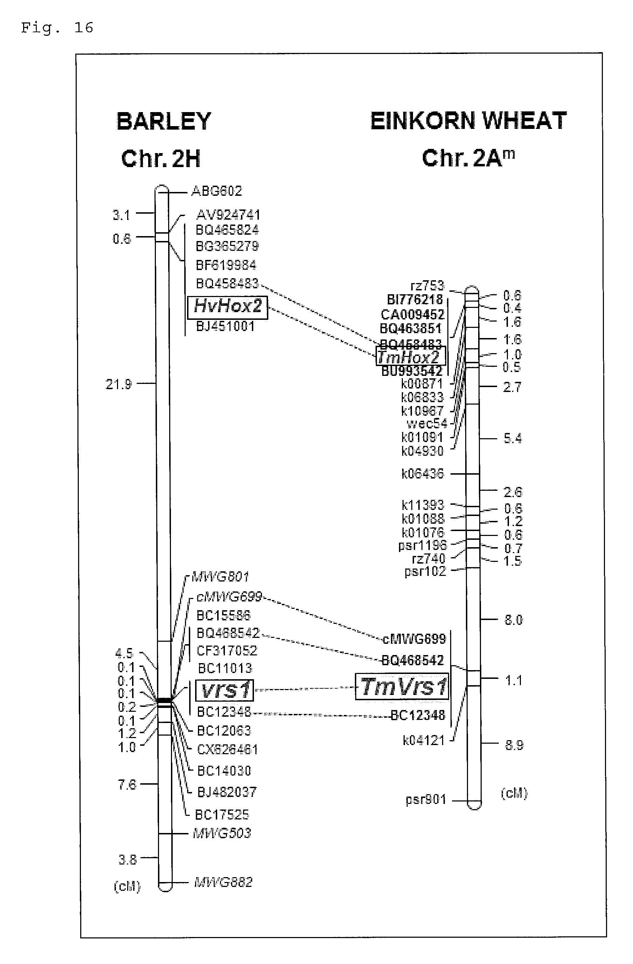

[Fig. 16] Fig. 16 is a schematic diagram illustrating the micro-collinearity between barley and Einkorn wheat in the Vrs1 and HvHox2 gene regions.

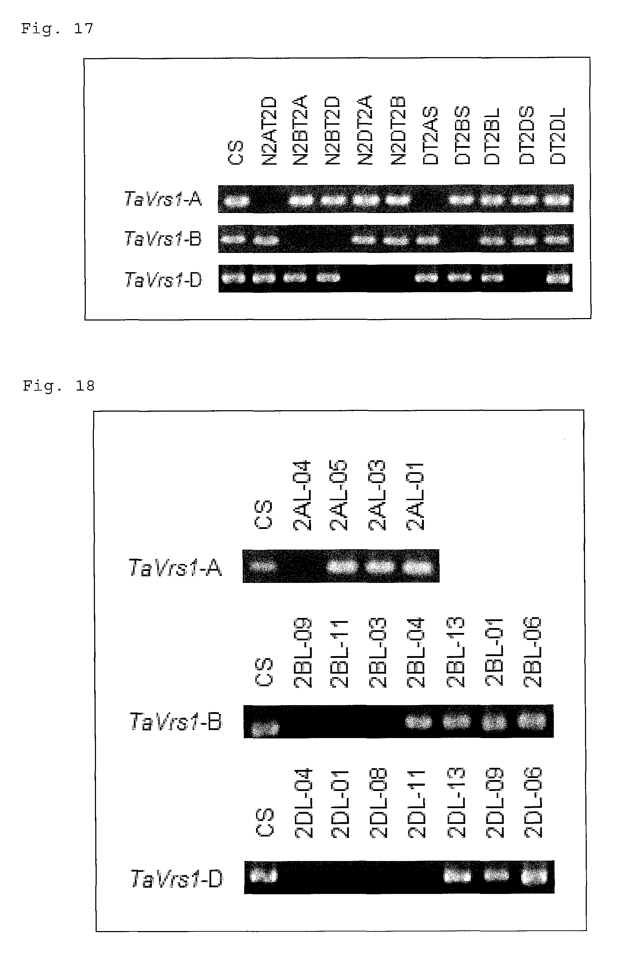

[Fig. 17] Fig. 17 is a photograph illustrating the PCR analysis result for determining the positions of the wheat Vrs1 genes (TaVrs1-A gene, TaVrs1-B gene, and TaVrs1-D gene) on the wheat chromosomes. In the figure, "CS" illustrates the analysis result of Chinese Spring, "N T" illustrates the results of the analysis on the nulli-tetrasomic line of CS having homoeologous group-2 chromosomes, and "DT" illustrates the results of the analysis on the ditelosomic line of CS having homoeologous group-2 chromosomes. "2A", "2B", and "2D" indicate the type of homoeologous group-2 chromosome of each line. "S" or "L" indicates a line having a "short arm" or a "long arm" in each line, respectively.

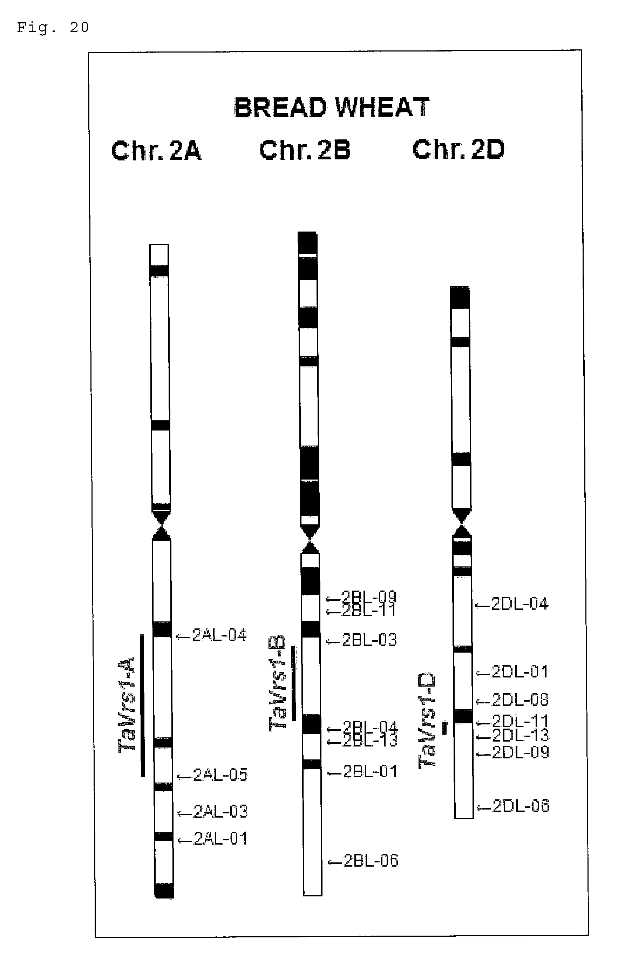

[Fig. 18] Fig. 18 is a photograph illustrating the PCR analysis result for determining the position of the wheat Vrs1 genes (TaVrs1-A gene, TaVrs1-B gene, and TaVrs1-D gene) on the wheat chromosomes. In the figure, "CS" illustrates the analysis result of Chinese Spring, "all except for CS" illustrate the results of the analysis on the deletion lines of CS having homoeologous group-2 chromosomes. "2AL-04" and the like represent the chromosome number (the position on each homoeologous group-2 chromosome) and mean that a part of the chromosome is deleted from this position in each line. The positions on the homoeologous group-2 chromosomes, as indicated by the corresponding chromosome numbers , are described in Fig. 20.

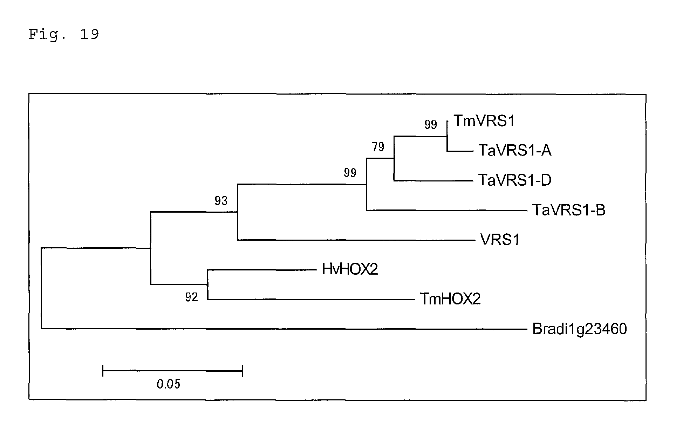

[Fig. 19] Fig. 19 is a genealogical tree illustrating the results of the analysis on the amino acid sequences expected from the barley Vrs1 gene and the wheat Vrs1 genes, by the neighbor-joining method. In the figure, VRS1 and HvHOX2 are genes derived from barley; TmVRS1, TaVRS1-A, TaVRS1-B, TaVRS1-D, and TmHOX2 are genes derived from wheat; and Bradilg23460 is a gene used as an outgroup in the neighbor-joining method. In this neighbor-joining method, 1,000 replicates are produced and the obtained local bootstrap values are given near the branching points.
[Fig. 20] Fig. 20 is a schematic diagram illustrating the positions of the wheat Vrs1 genes (TaVrs1-A gene, TaVrs1-B gene, and TaVrs1-D gene) on the wheat chromosomes.

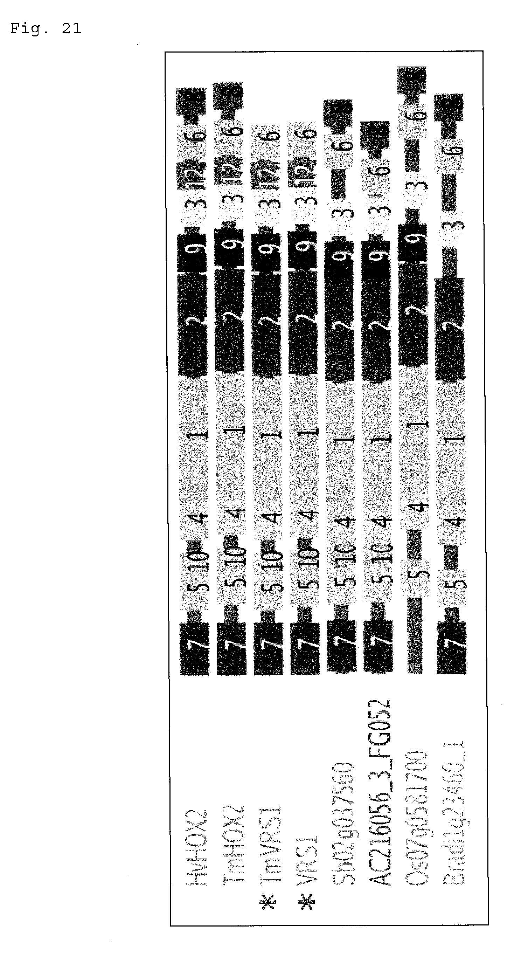

[Fig. 21] Fig. 21 is a schematic diagram illustrating the results of analysis and comparison of the motifs in the wheat VRS1 protein (TmVRS1 protein) and the HOX2 protein (TmHOX2 protein), barley VRS1 protein (VRS1 protein), and the HOX2 protein (HvHOX2 protein), and the like, with the comparative genome data base (SALAD: Surveyed conserved motif ALignment diagram and the Associating Dendrogram).

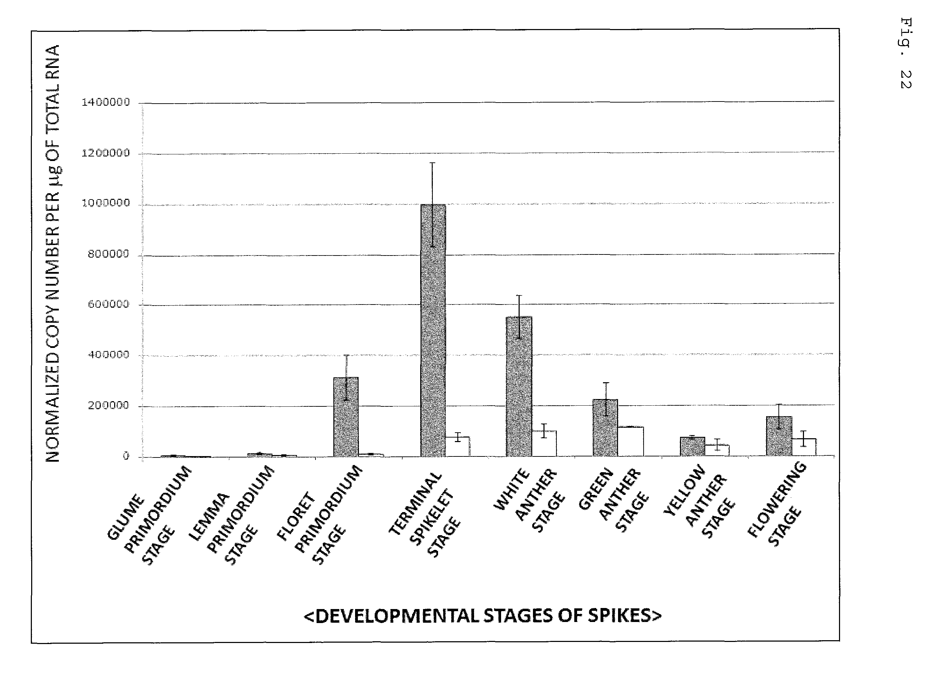

[Fig. 22] Fig. 22 is a graph illustrating the results of quantitative RT-PCR analysis on TmVrs1 mRNA and TmHox2 mRNA at each developmental stage of wheat spikes. In the figure, the gray bars indicate the amount (mean ± standard error) of TmVrs1 mRNA, and the white bars indicate the amount (mean ± standard error) of TmHox2 mRNA. At the terminal spikelet stage and the white anther stage, a significant difference (P value: 1%) was observed between TmVrs1 mRNA and TmHox2 mRNA. At the floret primordium stage, a significant difference (P value: 5%) was also observed between TmVrs1 mRNA and TmHox2 mRNA, but no significant difference was observed at other developmental stages.

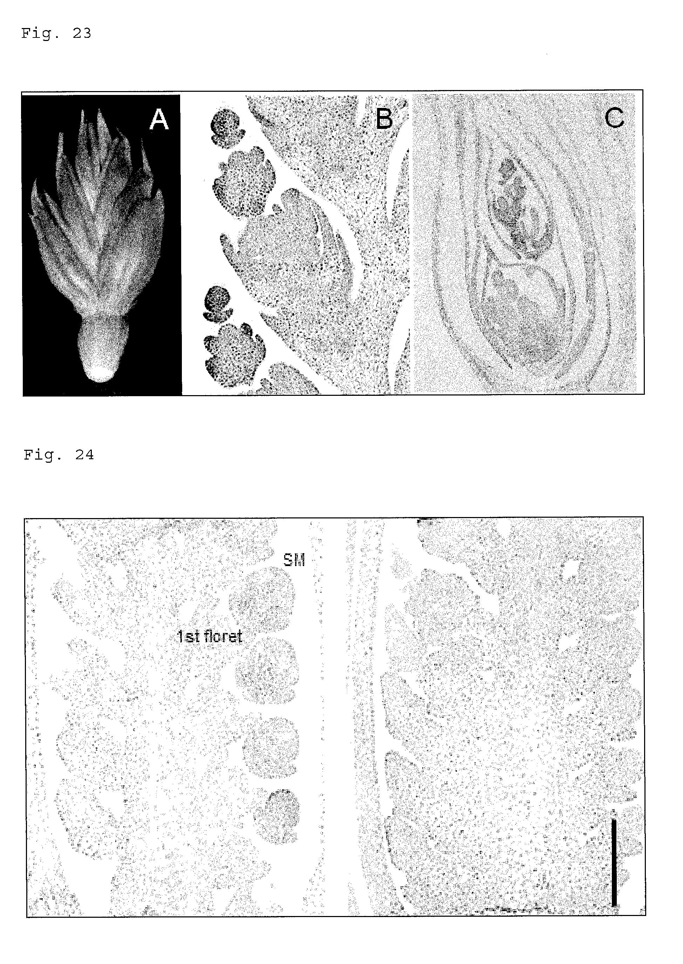

[Fig. 23] Fig. 23 is photographs illustrating the results of the RNA in situ hybridization analysis on the localization of TmVrs1 mRNA in a wheat spikelet (B and C in the figure). A illustrates the appearance of the wheat spikelet.
[Fig. 24] Fig. 24 is microscope photographs illustrating the results of the RNA in situ hybridization analysis on the localization of TmVrs1 mRNA at the floret primordium stage. In the figure, the left side illustrates the observation result after the hybridization with an antisense probe, whereas the right side illustrates the observation result after the hybridization with a sense probe. "1st floret" indicates a first floret, and "SM" indicates a spikelet meristem. The scale bar indicates 200 µm.

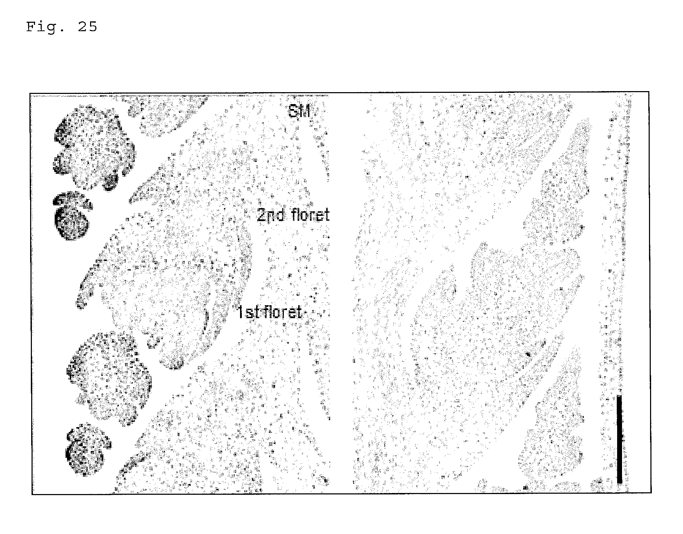

[Fig. 25] Fig. 25 is microscope photographs illustrating the results of the RNA in situ hybridization analysis on the localization of TmVrs1 mRNA at the terminal spikelet stage. In the figure, the left side illustrates the observation result after the hybridization with an antisense probe, whereas the right side illustrates the observation result after the hybridization with a sense probe. "1st floret" indicates a first floret, "2st floret" indicates a second floret, and "SM" indicates a spikelet meristem. The scale bar indicates 200 µm.

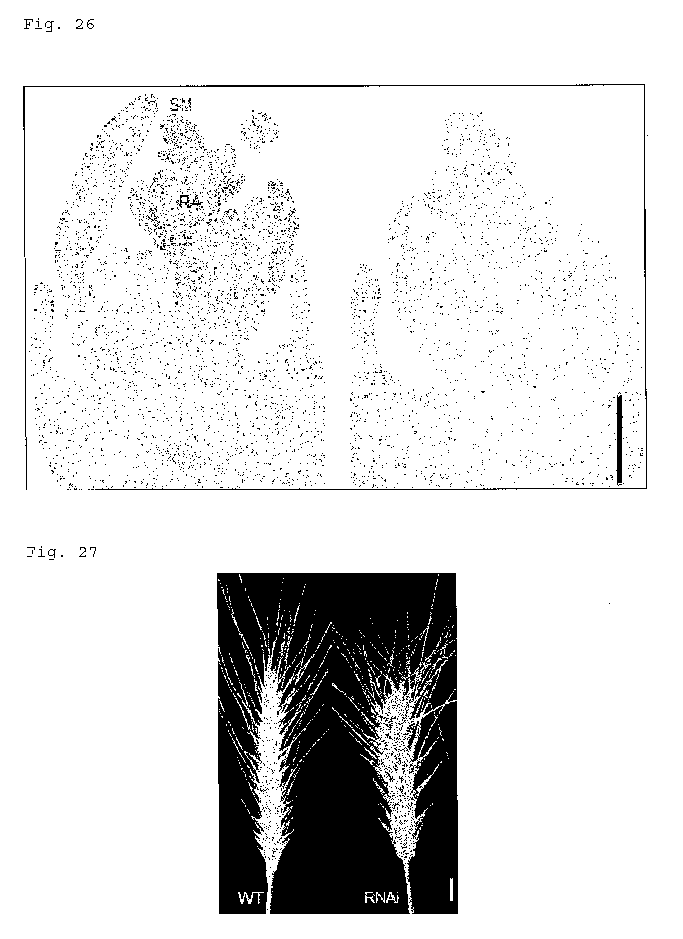

[Fig. 26] Fig. 26 is microscope photographs illustrating the results of the RNA in situ hybridization analysis on the localization of TmVrs1 mRNA at the white anther stage. In the figure, the left side illustrates the observation result after the hybridization with an antisense probe, whereas the right side illustrates the observation result after the hybridization with a sense probe. "RA" indicates a rachilla, and "SM" indicates a spikelet meristem. The scale bar indicates 200 µm.
[Fig. 27] Fig. 27 is a photograph illustrating the result of comparison of the spike shape between a wild-type wheat (WT) and a T0 TaVrs1-A RNAi transgenic wheat (RNAi). The scale bar indicates 1 cm.

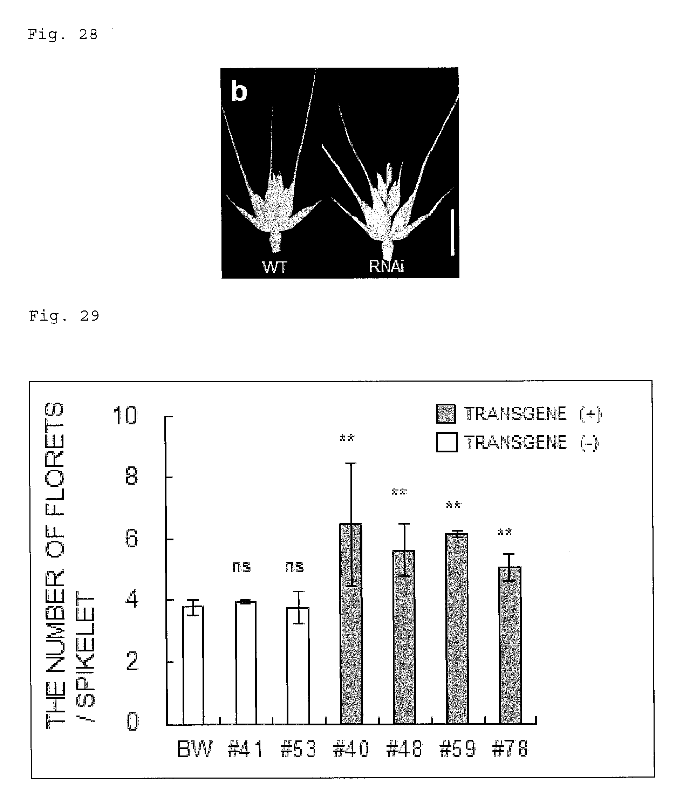

[Fig. 28] Fig. 28 is a photograph illustrating the result of comparison of the spikelet shape between a wild-type wheat (WT) and a T0 TaVrs1-A RNAi transgenic wheat (RNAi) . The scale bar indicates 1 cm.
[Fig. 29] Fig. 29 is a graph illustrating the number of florets per spikelet in wild-type wheats (transgene (-)) and T0 TaVrs1-ARNAi transgenic wheats (transgene (+)). In the figure, the values obtained by analyzing three spikes for each wheat are expressed as the mean ± standard deviation. The statistical comparison is performed by Student test. Two asterisks (**) mean P < 0.01, and "ns" means no significant difference.

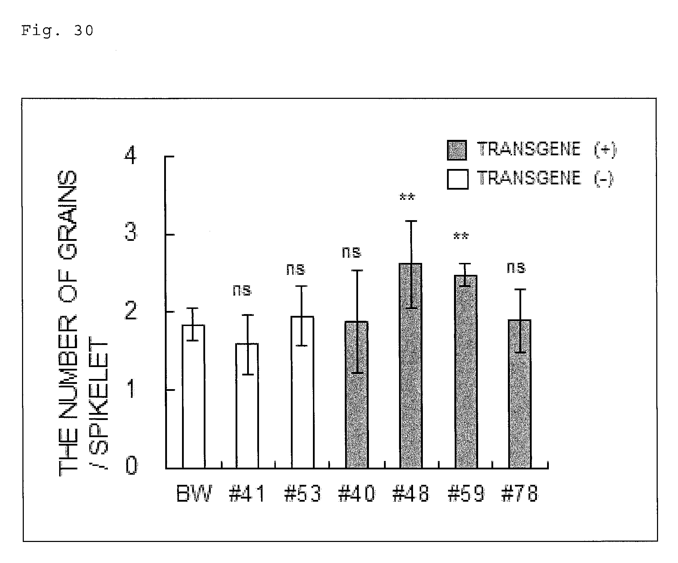

[Fig. 30] Fig. 30 is a graph illustrating the number of grains per spikelet in wild-type wheats (transgene (-)) and T0 TaVrs1-ARNAi transgenic wheats (transgene (+)). In the figure, the values obtained by analyzing three spikes for each wheat are expressed as the mean ± standard deviation. The statistical comparison is performed by Student test. Two asterisks (**) mean P < 0.01, and "ns" means no significant difference.

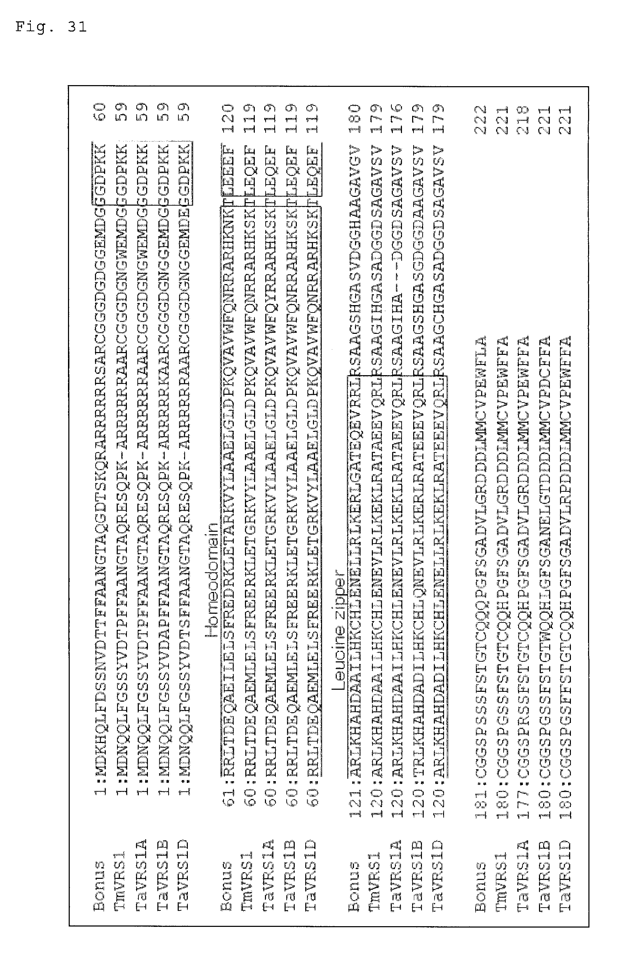

[Fig. 31] Fig. 31 is a figure illustrating the alignments of the amino acid sequences of the barley VRS1 and the wheat VRS1 (TmVRS1, TaVRS1-A, TaVRS1-B, and TaVRS1-D). In the figure, "Bonus" indicates the amino acid sequence of the barley VRS1, "TmVRS1" the amino acid sequence of TmVRS1, "TaVRS1A" the amino acid sequence of TaVRS1-A, "TaVRS1B" the amino acid sequence of TaVRS1-B, and "TaVRS1D" the amino acid sequence of TaVRS1-D, respectively. The numbers in the right and left of the amino acid sequence indicates the residue numbers of the amino acid residues at the ends. In the figure, "Homeodomain" and "Leucine zipper" indicate the regions of the HD domain motif and the LZ domain motif in the protein.

### [Description of Embodiments]

As described below, it was found that, in floret primordia of barley, the barley Vrs1 gene and protein were highly expressed in the pistil of floret organs in lateral spikelets that are expected to degenerate and become sterile in two-rowed barley, revealing that the VRS1 protein controls development of the pistil of florets in lateral spikelets to suppress the fertility of the florets. It was further found that the introduction of a DNA encoding siRNA complementary to the transcript of the Vrs1 gene into two-row barley successfully restored the fertility of lateral spikelets and thus can increase the number of grains per spikelet (per plant).

In wheat with no lateral spikelets, on the other hand, the presence of the wheat gene homologous to the barley Vrs1 gene was unknown (Sakuma, S, et al. , Plant Cell Physiol, 2011, volume 52, 738-749 pages). The present inventors, however, isolated homologous genes (Vrs1 genes derived from wheat (TmVrs1 gene, TaVrs1-A gene, TaVrs1-B gene, and TaVrs1-D gene)) to the barley Vrs1 gene for the first time, and found that the endogenous wheat Vrs1 genes are specifically expressed in upper florets which are expected to usually degenerate and become sterile in spikelets. Therefore, the suppression of the function of the endogenous wheat Vrs1 genes can cause grain bearing in upper florets whose development is originally expected be suppressed. The above results lead to completion of the present invention including a method for increasing the number of wheat grains by artificially suppressing the function of the endogenous wheat Vrs1 genes. It was further shown that the introduction of a DNA encoding a siRNA complementary to the transcript of the wheat Vrs1 gene into wheat increased the number of florets and the number of grains per wheat spikelet.

### <Wheat with Increased Number of Grains, and Method for Producing Wheat>

Therefore, the present invention provides a wheat with an increased number of grains as compared with a control wheat, wherein the function of the endogenous wheat Vrs1 gene is artificially suppressed; and a method for producing the wheat with an increased number of grains, wherein the method comprises a step of artificially suppressing the function of the endogenous Vrs1 gene in wheat.

In the present invention, "wheat" means plants belonging to Triticum. The "number of grains" means not only the number of seeds (so-called "grains", or "wheat grains") but also the number of fertile florets. The "control wheat" means wheat in which the function of the endogenous wheat Vrs1 gene is not artificially suppressed (for example, wild-type wheat). In the present invention, the "with an increased number of grains as compared with a control wheat" means that the number of grains per spikelet is larger than that of a control wheat.

The "wheat Vrs1 gene" typically means a DNA encoding the protein with the amino acid sequence described in SEQ ID NO: 3 (for example, the DNA with the nucleotide sequence described in SEQ ID NO: 1, this DNA being also referred to as the "TmVrs1 gene") in diploid wheat (Triticum monococcum etc.). In hexaploid wheat (Triticum aestivum(Bread wheat) etc.), the "wheat Vrs1 gene" typically means a DNA encoding the protein with the amino acid sequence described in SEQ ID NO: 58 (for example, the DNA with the nucleotide sequence described in SEQ ID NO: 56, this DNA being also referred to as the "TaVrs1-A gene" ), a DNA encoding the protein with the amino acid sequence described in SEQ ID NO: 61 (for example, the DNA with the nucleotide sequence described in SEQ ID NO: 59, this DNA being also referred to as the "TaVrs1-B gene"), and a DNA encoding the protein with the amino acid sequence described in SEQ ID NO: 64 (for example, the DNA with the nucleotide sequence described in SEQ ID NO: 62, this DNA being also referred to as the "TaVrs1-D gene"). Two or more of these genes may be collectively referred to as the "wheat Vrs1 gene."

The "wheat VRS1" or "wheat VRS1 protein" is a protein encoded by any one of the above wheat Vrs1 genes. The amino acid sequence of the protein is typically an amino acid sequence described in SEQ ID NO: 3, 58, 61, or 64. Two or more of these proteins may be collectively referred to as the "wheat VRS1" or "wheat VRS1 protein." The proteins encoded by the TmVrs1 gene, the TaVrs1-A gene, the TaVrs1-B gene, and the TaVrs1-D gene may be referred to as "TmVRS1" , "TaVRS1-A", "TaVRS1-B", and "TaVRS1-D", respectively.

The DNA encoding the protein with the amino acid sequence described in SEQ ID NO: 3 is isolated from experimental line KT-3-5 of Triticum monococcum and sequenced for the first time; and the DNA encoding the protein with the amino acid sequence described in SEQ ID NO: 58, the DNA encoding the protein with the amino acid sequence described in SEQ ID NO: 61, and the DNA encoding the protein with the amino acid sequence described in SEQ ID NO: 64 are isolated from Triticum aestivum (variety name : Chinese Spring) and sequenced for the first time in the present invention.

Since nucleotide sequences can mutate in nature, the wheat Vrs1 genes in the present invention not only include the DNA encoding the protein with the amino acid sequence described in SEQ ID NO: 3, the DNA encoding the protein with the amino acid sequence described in SEQ ID NO: 58, the DNA encoding the protein with the amino acid sequence described in SEQ ID NO: 61, and the DNA encoding the protein with the amino acid sequence described in SEQ ID NO: 64, but also include allele mutants encoding wheat VRS1 proteins having similar activity to these proteins. The homology of the DNA encoding the protein with the amino acid sequence described in SEQ ID NO: 3 and the like to the allele mutants thereof is usually 95% or more at the amino acid sequence level, preferably 97% or more, more preferably 99% or more from the viewpoint of keeping the function as a transcription factor.

The sequence homology can be determined using programs, such as BLASTP (amino acid level), BLASTN (nucleotide level), and BLASTX. The programs are based on BLAST, an algorithm by Karlin and Altschul (Proc. Natl. Acad. Sci. USA, 87: 2264-2268, 1990, Proc. Natl. Acad. Sci. USA, 90: 5873-5877, 1993). When the amino acid sequence is analyzed by BLASTP and the like, the parameters are set to, for example, score = 50, and wordlength = 3. When the amino acid sequence is analyzed using a Gapped BLAST program, the analysis can be performed as described in Altschul et al. (Nucleic Acids Res. 25: 3389-3402, 1997). When BLAST and the Gapped BLAST program are used, the default parameters in each program are used. Specific techniques of these analysis methods are known.

The "endogenous wheat Vrs1 gene" means the wheat Vrs1 gene which is already present in the genome of wheat cells.

The "artificial suppression of the function of the endogenous wheat Vrs1 gene" in the present invention includes both complete suppression (inhibition) and partial suppression of the function. It also includes artificial suppression of the expression of the endogenous Vrs1 gene, as well as artificial suppression of the activity of the protein encoded by the endogenous Vrs1 gene. Such activity includes the activity to suppress the transcriptional activation potential of the downstream gene responsible for development of upper florets by the HOX2 protein (see Examples below for the relation between the VRS1 protein and the HOX2 protein).

One aspect of the method for artificially suppressing the function of the endogenous Vrs1 gene in the wheat of the present invention includes a method in which a DNA encoding a dsRNA (double-stranded RNA) complementary to the transcript of the endogenous wheat Vrs1 gene (in the present invention, this method is called a "double-stranded RNA method" for short). The introduction of the dsRNA having the identical or similar sequence to the target gene sequence into cells can cause the phenomenon called RNAi (RNA interference), in which the expression of both the introduced foreign gene and the target endogenous gene is suppressed. When the dsRNA having about 40 to several hundreds of base pairs is introduced into cells, the RNase III-like nuclease having a helicase domain, called Dicer, cleaves the dsRNA into about 21 to 23 base pairs from the 3' end in the presence of ATP to produce siRNAs (short interference RNAs). To this siRNA, a specific protein is bound to form a nuclease complex (RISC: RNA-induced silencing complex). This complex recognizes and binds to the same sequence as the siRNA, and cleaves the transcript (mRNA) of the target gene at the center of the siRNA by means of the RNase III-like enzyme activity. Separately from this pathway, another pathway may be also considered, in which the antisense strand of the siRNA binds to the mRNA and acts as a primer for RNA-dependent RNA polymerase (RsRP) to synthesize a dsRNA, and this dsRNA becomes the substrate of Dicer again to produce a new siRNA and thus to amplify its action.

The DNA encoding the dsRNA of the present invention includes the target gene, i.e., the antisense DNA encoding the antisense RNA to any region of the transcripts (mRNAs) of the wheat Vrs1 genes, and the sense DNA encoding the sense RNA to any region of the mRNAs. The antisense DNA and the sense DNA can express the corresponding antisense RNA and sense RNA. These antisense RNA and sense RNA can produce dsRNA.

As the configuration where the dsRNA expression system of the present invention is carried by a vector or the like, the antisense RNA and sense RNA may be expressed from the same vector, or the antisense RNA and sense RNA may be expressed from different vectors. As the configuration where the antisense RNA and the sense RNA are expressed from the same vector, for example, an antisense RNA expression cassette and a sense RNA expression cassette are each constructed, in which promoters that may drive the expression of a short RNA, such as the pol III system, are ligated upstream of the antisense DNA and the sense DNA, respectively and these cassettes are then inserted into a vector in the same direction or in opposite directions.

An expression system can also be constructed in which the antisense DNA and the sense DNA are arranged in opposite directions on different strands so that these DNAs face each other. This construct includes one double-stranded DNA (siRNA-encoding DNA) in which an antisense RNA-encoding strand and a sense RNA-encoding strand are paired, and promoters are oppositely oriented on both sides of the double-stranded DNA to express the antisense RNA and the sense RNA from the respective strands. In this case, to avoid addition of unnecessary sequences downstream of the sense RNA and the antisense RNA, terminators are preferably placed at the 3' ends of the strands (antisense RNA-encoding strand and sense RNA-encoding strand), respectively. As this terminator, a sequence of four or more continuous A (adenine) nucleotides can be used. In this palindromic expression system, the types of the two promoters are preferably different.

As the configuration where the antisense RNA and sense RNA are expressed from different vectors, for example, an antisense RNA expression cassette and a sense RNA expression cassette are each constructed, in which promoters are ligated upstream of the antisense DNA and the sense DNA, respectively and these cassettes are then carried by different vectors. In these different vectors, the types of the promoters may be the same or different.

The dsRNA used in the present invention may be a siRNA. The "siRNA" means a double-stranded RNA with a short chain in the range without showing toxicity in cells.

The chain length of the dsRNA is not particularly limited as long as the dsRNA can suppress the expression of the target gene and also shows no toxicity. The chain length of the dsRNA is, for example, 15 to 49 base pairs, preferably 15 to 35 base pairs, more preferably 21 to 30 base pairs. The chain length is still more preferably 21 to 23 base pairs.

As the DNA encoding the dsRNA of the present invention, a construct that produces a double-stranded RNA having a hairpin structure (self-complementary 'hairpin' RNA (hpRNA)) upon insertion of an appropriate sequence (preferably an intron sequence) between the inverted repeats of the target sequence (Smith, N. A. et al., Nature, 407: 319, 2000; Wesley, S. V. et al., Plant J., 27: 581, 2001; Piccin, A. et al. , Nucleic Acids Res. , 29: E55, 2001) can be also used.

The DNA encoding the dsRNA of the present invention need not be completely identical to the nucleotide sequence the target gene, but has a sequence identity of at least 70% or more, preferably 80% or more, more preferably 90% or more (for example, 95%, 96%, 97%, 98%, 99% or more) to the nucleotide sequence of the full length or partial length (usually the region including 100 nucleotides or more, preferably the region including 200 nucleotides or more, more preferably the region including 300 nucleotides or more) of the target gene. The sequence identity can be determined by the above technique (BLAST program).

The double-stranded RNA portion, in which RNAs in the ds RNA are paired, is not limited to those in which RNAs are completely paired, but may include unpaired parts due to mismatch (where the corresponding nucleotide is not complementary), bulge (one of the strands lacks the corresponding nucleotide), or the like. In the present invention, the double-stranded RNA region in which the RNAs in the dsRNA are paired may include both bulge and mismatch.

As another aspect of the method for artificially suppressing the function of the Vrs1 gene in the wheat of the present invention, a method in which a DNA (antisense DNA) encoding the antisense RNA complementally to the transcript of the wheat Vrs1 gene is used (this method is called an "antisense method" for short in the present invention). The actions of the antisense DNA to suppress the expression of the target gene include inhibition of transcription initiation by triple strand formation; transcriptional repression by hybridization with the site where a local open loop structure is locally formed by an RNA polymerase ; transcription inhibition by hybridization with the RNA being synthesized; splicing repression by hybridization at the junction between an intron and an exon; splicing repression by hybridization with the spliceosome formation site; repression of mRNA translocation from the nucleus to the cytoplasm by hybridization with mRNA; splicing repression by hybridization with the capping site or the poly A addition site; repression of translation initiation by hybridization with the translation initiation factor binding site; translation repression by hybridization with the ribosome binding site near the start codon; and inhibition of peptide chain extension by hybridization with the translated region or the polysome binding site on mRNA; and the repression of the gene expression by hybridization with the interaction site between nucleic acids and proteins. These actions suppress the expression of the target gene by inhibiting the process of transcription, splicing, or translation (Hirashima and Inoue, "New Lectures on Biochemical Experiments 2 , Nucleic Acids IV, Replication and Expression of Genes," edited by the Japanese Biochemical Society, Tokyo Kagaku Dozin, pp. 319-347, 1993). The antisense DNA used in the present invention may suppress the expression of the target gene by any one of the above actions. In one aspect, an antisense sequence is designed to be complementary to the untranslated region near the 5' end of the mRNA of the target gene in order to effectively inhibit the gene translation. In addition, sequences complementary to the coding region or the untranslated region at the 3' side can be also used. Thus, the antisense DNAs used in the present invention include DNA including antisense sequences to the sequences not only of the translated regions but also of the untranslated regions of the gene. The antisense DNA to be used is ligated downstream of an appropriate promoter and is preferably ligated to a sequence including the transcription termination signal on the 3' side.

The antisense DNA can be prepared by the phosphorothioate method (Stein, Nucleic Acids Res., 16: 3209-3221, 1988) and the like based on the sequence information on the wheat Vrs1 gene (For example, the DNA with the nucleotide sequence described in SEQ ID NO: 1, the DNA with the nucleotide sequence described in SEQ ID NO: 2, the DNA with the nucleotide sequence described in SEQ ID NO: 56, the DNAwith the nucleotide sequence described in SEQ ID NO: 57, the DNA with the nucleotide sequence described in SEQ ID NO: 59, the DNA with the nucleotide sequence described in SEQ ID NO: 60, the DNA with the nucleotide sequence described in SEQ ID NO: 62, and the DNA with the nucleotide sequence described in SEQ ID NO: 63). The prepared DNA can be introduced into plants by the known methods described below. The sequence of the antisense DNA is preferably a sequence complementary to the transcript of the wheat Vrs1 gene, but the sequence need not be completely complementary to the transcript of the wheat Vrs1 gene as long as the antisense DNA can effectively inhibit the gene expression. The transcribed RNA is preferably 90% or more (for example, 95%, 96%, 97%, 98%, 99% or more) complementary to the transcript of the target gene. In order to effectively inhibit the expression of the target gene, the antisense DNA has a length of at least 15 nucleotides or more, preferably 100 nucleotides or more, more preferably 500 nucleotides or more. Usually, the length of the antisense DNA to be used is shorter than 5 kbs, preferably shorter than 2.5 kbs.

As another aspect of the method for artificially suppressing the function of the Vrs1 gene in the wheat of the present invention, a method in which a DNA encoding a RNA having a ribozyme activity to specifically cleave the transcript of the wheat Vrs1 gene may be mentioned (this method is called a "ribozyme method" for short in the present invention). Ribozymes include group I intron ribozymes, ribozymes having a size of 400 nucleotides or more, such as M1RNA included in RNase P, and ribozymes having an active domain of about 40 nucleotides, called hammer head type and hairpin type (Makoto Koizumi and Eiko Otsuka, Proteins, Nucleic acids, and Enzymes, 35: 2191, 1990).

For example, the self-cleavage domain of the hammerhead type ribozyme cleaves the 3' side of the C15 of G13U14C15, but base pair formation of the U14 with the A at position 9 is considered important for the activity. It has been also shown that, even when the nucleotide at position 15 is A or U instead of C, the cleavage to occur (Koizumi et.al., FEBS Lett. 228: 225, 1988). If the substrate binding site of the ribozyme is designed to be complementary to the RNA sequence near the target site, a restriction enzyme-like RNA cleaving ribozyme can be created which recognizes the sequence of UC, UU, or UA in the target RNA (Koizumi et.al. , FEBS Lett. , 239: 285, 1988, Makoto Koizumi and Eiko Otsuka, Proteins, Nucleic Acids, Enzymes, 35: 2191, 1990, Koizumi et. al. , Nucleic Acids. Res., 17: 7059, 1989).

The hairpin type ribozyme is also useful for the purpose of the present invention. The hairpin type ribozyme can be found, for example, in the minus strand of the satellite RNA of tobacco ringspot virus (Buzayan, Nature, 323: 349, 1986). It has been shown that this ribozyme can be designed to cause target-specific cleavage of RNA (Kikuchiand Sasaki, Nucleic Acids Res., 19: 6751, 1992, Hiroshi Kikuchi, Chemistry and Biology, 30: 112, 1992). The ribozyme designed to cleave the target is linked to a promoter such as the cauliflower mosaic virus 35S promoter and to a transcription termination sequence so that the ribozyme can be transcribed in wheat cells. The tandem arrangement of these structural units can cleave multiple sites in the target gene and also can increase the effect (Yuyama et al. , Biochem. Biophys. Res. Commun. 186: 1271, 1992). Specific cleavage of the target transcript of the target wheat Vrs1 gene using this ribozyme can suppress the expression of the DNA.

Although the double-stranded RNA method, the antisense method, and the ribozyme method according to the present invention are described above, the method for artificially suppressing the function of the Vrs1 gene in the wheat of the present invention is not limited to the methods in which the transcript of the above Vrs1 gene is the target, and the like. As another aspect of the method for artificially suppressing the function of the Vrs1 gene in the wheat of the present invention, a method in which mutation is introduced to the coding regions, the non-coding regions, the transcriptional control regions (promoter regions), or the like of the above gene may be mentioned.

The mutation to be introduced to the endogenous wheat Vrs1 gene in the present invention is not particularly limited as long as the mutation suppresses the function of the gene. For example, null mutations, such as nonsense mutations, frameshift mutations, insertion mutations, or splice site mutations are preferred. The number of mutations to be introduced to any one of the endogenous wheat Vrs1 genes is also not particularly limited as long as the mutation suppresses the function of the gene. The number of mutations may be one or more (for example, 2, 3 or less, 5 or less, 10 or less).

The activity of the wheat VRS1 protein encoded by the wheat Vrs1 gene can be artificially suppressed by introducing the mutation into the endogenous wheat Vrs1 gene region on the wheat genome, preferably the region encoding the wheat VRS1, with the guidance of the finding about the mutation in the Vrs1 gene in many six-rowed barley lines that have the Vrs1 gene encoding the barley VRS1 whose activity is suppressed.

As described below in Example 4 and the like, the activity of the barley VRS1 is to competitively inhibit the transcriptional activation of the downstream gene by the HOX2 protein which is a transcription factor essential to the development of floret primordia. As a result of the competitive inhibition by this VRS1, the floret primordia of the lateral spikelet where VRS1 is expressed cannot be differentiated into fertile florets.

There have been reported many examples of the mutation in the endogenous Vrs1 gene in many barley lines in which the activity of the barley VRS1 is suppressed to became six-rowed (NPLs 1 and 3).

As disclosed in NPL 1, the analysis on the mutation in the endogenous barley Vrs1 gene which is found in many six-rowed barley lines has revealed not only an example in which the base substitution in the region encoding the VRS1 protein suppressed the activity of VRS1 protein, but also an example in which the intron sequence near the region encoding the VRS1 protein suppressed the function of the VRS1 protein. As disclosed in NPL 3, lines having the mutation in the endogenous Vrs1 gene were selected using the TILLING method from the barley group in which the mutation was induced using ethyl methanesulfonate. As a result, two lines showing six-row (8408-1 and 11910-1) were obtained; the base substitution that changed the amino acid at position 95 of the VRS1 protein from leucine to glutamine was found in the Vrs1 gene of the 8408-1 line; and the base substitution that inhibited normal splicing of the Vrs1 gene transcript was found in the 11910-1 line.

In the present invention, the wheat Vrs1 gene was found to have similar biological functions to the barley Vrs1. The amino acid sequences of the wheat VRS1 proteins (TmVRS1, TaVRS1-A, TaVRS1-B, and TaVRS1-D) have a high homology of about 86% to the barley VRS1 protein based on the entire protein (see Fig. 31). In particular, the HD domain motif and the LZ motif, which are considered essential to the function of the VRS1 proteins, are conserved in the wheat VRS1 protein as well, and thus the barley VRS1 and the respective wheat VRSls (TmVRS1, TaVRS1-A, TaVRS1-B, and TaVRS1-D) have a high amino acid sequence homology of about 93% in the HD motif.

Therefore, the findings about the mutation in the barley Vrs1 gene in six-rowed barley can be guidance for producing wheats having suppressed function of the endogenous wheat Vrs1 gene by introducing the mutation into the wheat Vrs1 gene.

Reported in NPL 1 are many examples in which the mutation in the endogenous barley Vrs1 gene in six-rowed barley lines resulted in single amino acid residue substitution in the HD domain motif of VRS1 encoded by the gene, or resulted in nonsense mutation in the genomic nucleotide sequence encoding the HD domain motif. Disclosed in NPL 3 is a six-rowed barley having VRS1 in which leucine at position 95, which is an amino acid residue in the HD domain motif, was substituted with glutamine. These findings indicate that an unchanged or substantially unchanged amino acid sequence in the HD domain motif is essential for the function of the barley VRS1.

It can be supposed with high probability that an unchanged or substantially unchanged amino acid sequence in the HD domain motif is also essential for the activity of the wheat VRS1, based on a high homology in the amino acid sequence between the barley VRS1 and the wheat VRS1, and the similarity in the biological role of these proteins.

Therefore, to obtain the endogenous wheat Vrs1 gene with the function being suppressed, the mutation is preferably introduced so as to change or delete the entire or partial amino acid sequence in the HD domain motif of the VRS1 protein encoded by the gene. Specifically, the mutation is introduced to cause the amino acid substitution in the amino acid sequence in the HD domain motif of the wheat VRS1 (amino acids at positions 54 to 113 in the amino acid sequence described in SEQ ID NO: 3 for TmVRS1; amino acids at positions 54 to 113 in the amino acid sequence described in SEQ ID NO: 58 for TaVRS1-A; amino acids at positions 54 to 113 in the amino acid sequence described in SEQ ID NO: 61 for TaVRS1-B; and amino acids at positions 54 to 113 in the amino acid sequence described in SEQ ID NO: 64 for TaVRS1-D). Disclosed in NPL 3 are, in addition to the six-rowed barley line (8408-1 line) that has the Vrs1 gene having the base substitution which changes the amino acid at position 95 of the VRS1 protein from leucine to glutamine, a six-rowed barley line that has the Vrs1 gene having the base substitution which changes the amino acid residue at position 75 of the VRS1 protein from phenylalanine to isoleucine, a six-rowed barley line that has the Vrs1 gene having the base substitution which changes the amino acid residue at position 89 of the VRS1 protein from leucine to glutamine, a six-rowed barley line that has the Vrs1 gene having the base substitution which changes the amino acid residue at position 90 of the VRS1 protein from alanine to valine, a six-rowed barley line that has the Vrs1 gene having the base substitution which changes the amino acid residue at position 93 of the VRS1 protein from leucine to histidine, a six-rowed barley line that has the Vrs1 gene having the base substitution which changes the amino acid residue at position 103 of the VRS1 protein from tryptophan to arginine, and a six-rowed barley line that has the Vrs1 gene having the base substitution which changes the amino acid residue at position 107 of the VRS1 protein from arginine to leucine. These examples indicate that the activity of the barley VRS1 is suppressed by the amino acid substitutions found in these examples. All of the amino acid residues targeted for the amino acid substitution in these six-rowed barley lines are conserved in the amino acid sequence of the wheat VRS1. Therefore, to obtain the endogenous wheat Vrs1 gene with the function being suppressed, the base substitution is more preferably introduced in the endogenous wheat Vrs1 gene so that the substitutions of the amino acid residues at the positions corresponding to the above amino acid residues targeted for the amino acid substitutions in the six-rowed barley lines occur in the amino acid sequence of the wheat VRS1. Of six-rowed barley lines, however, some lines have been also found to have VRS1 in which the amino acid substitutions were introduced into other parts than the HD domain motif of the barley VRS1 (NPL 1). Accordingly, a wheat having a suppressed function of the endogenous wheat Vrs1 can be produced by modifying the endogenous wheat Vrs1 gene to encode VRS1 having the substitution of the amino acid residue in other parts than the HD domain motif of the wheat VRS1.

A nonsense mutation or frameshift mutation is more preferably introduced into the nucleotide sequences encoding the amino acid sequence in the HD domain motif of the wheat VRS1 and the amino acid sequence at the amino terminus side from this amino acid sequence in the endogenous wheat Vrs1 gene. This is because the entire or partial HD domain motif is deleted from the wheat VRS1 encoded by the gene. In the endogenous wheat Vrs1 gene, the region encoding the protein starts within the first exon and ends within the third exon. When the first intron sequence or the second intron sequence is not normally spliced from the transcript of the gene, the VRS1 protein having the activity is not produced. Therefore, in order to obtain the endogenous Vrs1 gene with the function being suppressed, the mutation is more preferably introduced into the donor site sequence (GA) or acceptor sequence (AG) in these intron sequences. Furthermore, it is known to those skilled in the art that there are essential sequences in the intron to cause normal splicing, in addition to the donor site sequence and the acceptor site sequence, and thus the mutation is also more preferably introduced so as to change the essential sequences to cause the above normal splicing in the above first and second introns. The introduction of these mutations can produce a null mutant of the wheat Vrs1 gene.

It is known that, when plants are irradiated with fast neutrons, gamma rays, or the like, the deletion of about the length including the entire endogenous wheat Vrs1 gene or longer occurs. To obtain the wheat in which the function of the endogenous wheat Vrs1 gene is suppressed or lost, the wheat deficient in the entire or most of the endogenous wheat Vrs1 gene is more preferably produced or selected. The introduction of these mutations can produce a null mutant of the wheat Vrs1 gene.

The introduction of the mutation to the endogenous wheat Vrs1 gene can be carried out by a method using a chemical mutagen, a physical mutation introduction method, a method for introducing a transposon or the like into genomic DNA, or a method using a DNA double strand cleaving enzyme such as zinc finger nuclease and TALEN, but the method is not limited to these.

Examples of the method using a chemical mutagen include a method in which seeds or the like are treated with a chemical mutagen (see Zwar and Chandler, Planta, 1995, volume 197, 39-48 pages, etc.). The chemical mutagen are not particularly limited, but examples thereof include ethyl methanesulfonate (EMS), N-ethyl-N-nitrosourea (ENU), N-methyl-N-nitrosourea (NNU), sodium azide, sodium hydrogensulfite, hydroxylamine, N-methyl-N'-nitro-N-nitroguanidine (MNNG), N-methyl-N'-nitrosoguanidine (NTG), O-methylhydroxylamine, nitrous acid, formic acid, and nucleotide analogs.

Examples of the physical mutation introduction method include fast neutron irradiation, gamma ray irradiation, heavy ion beam (HIB) irradiation, and ultraviolet irradiation (see Hayashi et al., Cyclotrons and Their Applications, 2007, 18th international conference, 237-239 pages, and Kazama et al., Plant Biotechnology, 2008, volume 25, 113-117 pages).

Examples of the method for introducing a transposon or the like into genomic DNA include methods in which a transposon such as TOS17, T-DNA, or the like is inserted in wheat genomic DNA (see Kumar et al. , Trends Plant Sci., 2001, volume 6, No. 3, 127-134 pages, and Tamara et al., Trends in Plant Science, 1999, volume 4, No. 3, 90-96 pages) .

The method using a DNA double strand cleaving enzyme refers to a method using a DNA double strand cleaving enzyme that stimulates the endogenous repair mechanism, and examples of the enzyme include endonuclease, zinc finger nuclease (ZFN), TALEN (registered trademark, transcriptional activator-like effector nuclease), transposase, and site specific recombinase. For the zinc finger nuclease technique, see Le Provost et al. , Trends in Biotechnology, 2009, volume 28, No. 3, 134-141 pages, Durai et al., Nucleic Acids Research, 2005, volume 33 , No. 18, 5978-5990 pages, and Liu et al. , Biotechnology and Bioengineering, 2010, volume 106, 97-105 pages. For the techniques about TALEN, see Miller et al., Nature Biotechnology, 2011, volume 29, No. 2, 143-148 pages, U.S. Pat. No. 8440431, U.S. Pat. No. 8440432, and U.S. Pat. No. 8450471.

In particular, it is known to those skilled in the art that, when the mutation is introduced into wheat genomic DNA using ZFN or TALEN, the position on the genomic nucleotide sequence at which DNA double strand cleaving enzymes for the wheat genomic DNA cause the double strand cleavage of genomic DNA can be appropriately selected. After the double strand cleavage of the genomic DNA by these DNA double strand cleaving enzymes, the recombination of the genomic DNA occurs. At that time, the base substitution or the deletion or insertion of the base with a length of one to several tens of bases also occur with constant probability at the position where the cleavage occurs, as also known to those skilled in the art.

Therefore, for example, by introducing into wheat cells the DNA double strand cleaving enzyme designed to cause the DNA double strand cleavage in the genomic nucleotide sequence encoding the amino acid sequence in the HD domainmotif of the wheat VRS1 described above, wheats having the wheat Vrs1 gene encoding a wheat VRS1 mutant with the substitution of the amino acid sequence in the HD domain can be produced. In addition, by introducing into wheat cells the DNA double strand cleaving enzyme designed to cause the DNA double strand cleavage in the genomic nucleotide sequence encoding the amino acid sequence in the HD domain motif of the wheat VRS1 and the amino acid sequence at the amino terminus side from that amino acid sequence, wheat cells having the Vrs1 gene with a nonsense mutation or frameshift mutation in that region can be produced. Furthermore, the regeneration of these wheat cells can produce wheats in which the function of the Vrs1 gene is suppressed. The mutated Vrs1 gene thus produced is a null mutant. In the wheat to which the mutation is introduced by the above methods, the introduction of the mutation into the endogenous wheat Vrs1 gene can be confirmed by known methods. Such known methods include Southern blot method, Northern blot method, the PCR method, the DNA sequence method, and the microarray analysis method. Such methods can determine whether the mutation is introduced into the Vrs1 gene by comparing the length or sequence of the gene before and after the mutation introduction. If the use of Northern blot method, the RT-PCR method, the Western blot method, the ELISA method, the microarray analysis method, or the like revealed the decreased expression of the transcript or translation product of the Vrs1 gene in the wheat in which the mutation is introduced into the transcriptional control region or the like, this wheat can also be confirmed to be the wheat in which the mutation is introduced into the Vrs1 gene. Other methods for confirming the introduction of the mutation into the Vrs1 gene include TILLING (Targeting Induced Local Lesions IN Genomes) (see Slade et al., Transgenic Res. , 2005, volume 14, 109-115 pages, and Comai et al., Plant J., 2004, volume 37, 778-786 pages).

In particular, when a non-selective mutation is introduced into the wheat genome using the chemical mutagen, fast neutron, or the like as described above, the individual having the mutation in the endogenous wheat Vrs1 gene DNA can be selected by the above TILLING or the like after the amplification of the endogenous wheat Vrs1 gene DNA or a part of the DNA by PCR. In this case, the wheat having a null mutant of the Vrs1 gene can be also obtained. Furthermore, wheats obtained by these methods are also included in the wheats in which the function of the endogenous wheat Vrs1 gene is suppressed.

The wheat to which the mutation is introduced by the above method is crossed with a wild-type wheat to undergo backcrossing, whereby the mutation introduced into other genes than the Vrs1 gene can be also removed.

In the present invention, artificial suppression of the function of the endogenous wheat Vrs1 gene canbe carried out on various wheat plants, wheat seeds, or wheat cells according to the above methods or the like. The wheat cells include cultured cells derived from wheat as well as cells in wheat plants. The wheat cells further include various forms of plant cells derived from wheat, for example, suspension cultured cells, protoplasts, leaf slices, calluses, immature embryos, and pollens.

The regeneration of plants from the wheat cells in which the function of the endogenous wheat Vrs1 gene is artificially suppressed by the above methods or the like can produce wheats with an increased number of grains. Examples of the regeneration method of such plants include the method described in "Taiichi Ogawa, Journal of Pesticide Science in Japan, 2010, volume 35, No. 2, 160-164 pages."

The wheats in which the function of the endogenous wheat Vrs1 gene is suppressed by introducing the mutation into any one of the endogenous wheat Vrs1 genes may be a heterozygote of the Vrs1 gene to which the mutation is introduced. In this case, for example, F1 plants obtained by crossing the heterozygote wheat and a control wheat are crossed together to produce F2 plants, whereby a homozygote having the Vrs1 gene to which the mutation is introduced can be selected from the F2 plants. In order to keep more stably the effect of suppressing the function of the Vrs1 gene with the mutated endogenous wheat Vrs1 gene, the wheat is preferably a homozygote having the Vrs1 gene to which the mutation is introduced. In this case, "the wheat which is a homozygote having the Vrs1 gene to which the mutation was introduced" includes not only wheats having two Vrs1 alleles that have the same mutation as each other, but also wheats having a first Vrs1 allele that has a first mutation and encodes the VRS1 protein with the function being suppressed and having a second Vrs1 allele that has a second mutation and encodes the VRS1 protein with the function being suppressed.

Hexaploid wheat can produce wheats having the mutation in the TaVrs1-A gene to suppress the function of the Vrs1 gene, wheats having the mutation in the TaVrs1-B gene to suppress the function of the Vrs1 gene, or wheats having the mutation in the TaVrs1-B gene to suppress the function of the Vrs1 gene, or wheats having the mutation in two or more of the TaVrs1-A gene, the TaVrs1-B gene, and the TaVrs1-D gene to suppress the function of the Vrs1 gene. In order to keep more stably the effect of suppressing the function of the Vrs1 gene with the mutation in the endogenous wheat Vrs1 gene, wheats having the mutation in two genes of the above genes are more preferably produced. Also, wheats having the mutation in all of the above genes are still more preferably produced.

In the present invention, the DNA encoding a double-stranded RNA, the DNA encoding an antisense RNA, the DNA encoding a RNA having a ribozyme activity, the DNA encoding transposons, the DNA encoding a DNA double strand cleaving enzyme, or the like, as described above, may be introduced into wheat cells in the form of being inserted in vectors. Vectors into which the above DNAs for artificially suppressing the function of the Vrs1 gene is to be inserted are not particularly limited as long as they can express the inserted gene in wheat cells, but the vectors may contain a promoter for constitutive or inductive expression of the above DNAs. Examples of the promoter for constitutive expression include the 35S promoter of cauliflower mosaic virus, the rice actin promoter, and the maize ubiquitin promoter. Examples of the promoter for inductive expression include promoters which are known to drive expression in response to external factors such as infection with or invasion of filamentous fungi, bacteria, and viruses, low temperature, high temperature, dryness, ultraviolet irradiation, and spreading of particular compounds. Examples of such promoters include the promoter of the rice chitinase gene and the promoter of the tobacco PR protein gene, which are expressed by infection with or invasion of filamentous fungi, bacteria, viruses; the promoter of the rice lip19 gene, which is induced by low temperature; the promoters of the rice hsp80 gene and the hsp72 gene, which are induced by high temperature; the promoter of the Arabidopsis thaliana rab16 gene, which is induced by dryness; the promoter of the parsley chalcone synthase gene, which is induced by ultraviolet irradiation; and the promoter of the maize alcohol dehydrogenase gene, which is induced under anaerobic conditions. In addition, the promoter of the rice chitinase gene and the promoter of the tobacco PR protein gene are also induced by particular compounds such as salicylic acid, and the rab16 is also induced by spreading of plant hormone abscisic acid. As promoters to drive the expression of the DNA encoding a short RNA such as siRNA as the DNA according to the present invention, the pol III promoters are preferably used.

As the method for introducing the above DNA or the vector and the like having the inserted DNA into wheat cells, for example, various methods known to those skilled in the art can be used, such as the particle bombardment method, the Agrobacterium-mediated method (Agrobacterium method), the polyethylene glycol method, and electroporation.

When the wheat plant having artificially suppressed function of the Vrs1 gene is once obtained by the above methods or the like, progenies can be obtained from the plant by sexual reproduction or asexual reproduction. In addition, propagation materials (for example, seeds, scions, stubble s, calluses, protoplasts, etc.) can be obtained from the above plant, or their progenies or clones, whereby the above plant can be mass-produced based on the propagation materials. Therefore, the present invention includes the progenies and clones of the wheat with an increased number of grains, as well as the propagation materials thereof. The present invention further includes processed products made from the seeds of the wheat. Such processed products include flour, starch, bread, Japanese noodle udon, and spaghetti, which are made from the seeds of the wheat of the present invention.

### <Agent for Increasing the Number of Wheat Grains>

As described above, at least one DNA selected from the group consisting of (a) to (c) below or a vector having the inserted DNA is preferably used to increase the number of wheat grains:
(a) a DNA encoding a double-stranded RNA complementary to the transcript of the wheat Vrs1 gene;
(b) a DNA encoding an antisense RNA complementary to the transcript of the wheat Vrs1 gene; and
(c) a DNA encoding a RNA having a ribozyme activity to specifically cleave the transcript of the wheat Vrs1 gene.

Therefore, the present invention provides as one aspect an agent for increasing the number of wheat grains, wherein the agent comprises as an active ingredient at least one DNA selected from the group consisting of (a) to (c) above or a vector having the inserted DNA.

The agent of the present invention may be at least one DNA itself selected from the group consisting of (a) to (c) above or a vector itself having the inserted DNA, which may be mixed with other components. Other components are not particularly limited, and examples thereof include sterilized water, physiological saline, vegetable oils, surfactants, lipids, solubilizing agents, buffers, preservatives, gold particles, polysaccharides, polyamines, and salts. When the transformed cells of the present invention are prepared by the above Agrobacterium-mediated method, the agent may contain Agrobacterium having the introduced DNA.

The preferred embodiments of the present invention are described above, but more preferred specific embodiments will be described below regarding "a method for producing a wheat with an increased number of grains, wherein the method comprises the steps of introducing into wheat cells the vector having the inserted DNA encoding the double-stranded RNA complementary to the transcript of the wheat Vrs1 gene to obtain transformed cells, and regenerating a plant from the transformed cells."

First, the construction of "the vector having the inserted DNA encoding the double-stranded RNA complementary to the transcript of the wheat Vrs1 gene" will be described.

In the construction of the vector according to the present invention, first, a part of the DNA of the wheat Vrs1 gene is preferably cloned into a cloning vector (for example, pENTR/D-TOPO vector (produced by Invitrogen)) according to the attached instruction of the vector. A part of the wheat Vrs1 gene to be cloned into the vector, i.e., the target sequence of double-stranded RNA, is preferably a DNA with the nucleotide sequence of 323 bp mainly including 3'-UTR of the third exon of the TaVrs1-A gene (the DNA with the nucleotide sequence at positions 998 to 1320 described in SEQ ID NO: 56) (see Fig. 1) when the wheat targeted for the increase of the number of grains is a hexaploid wheat (for example, Bobwhite), from the viewpoint of enabling significantly specific suppression of the expression of the wheat Vrs1 gene. The DNA with the above sequence can be amplified by PCR using primer sets with the nucleotide sequences described in SEQ ID NOS : 54 and 55 (see Table 12) and subjected to the cloning. When the nucleotide sequence at positions 998 to 1320 described in SEQ ID NO : 56 is the target sequence of the double-stranded RNA, it can suppress not only the expression of the TaVrs1-A gene but also the expression of the TaVrs1-B gene and the TaVrs1-D gene.

Next, the DNA encoding the double-stranded RNA complementary to a part of the DNA of the wheat Vrs1 gene is preferably cut out form this cloning vector and inserted to an RNAi vector. When, for example, a pANDA-β vector (see Fig. 2) is used as the RNAi vector here, the DNA can be easily transferred to the RNAi vector from the cloning vector through the LR reaction of the Gateway system.

Next, a method for introducing into wheat cells the vector according to the present invention which is constructed above will be described. The wheat cultivar to which the vector according to the present invention is to be introduced is not particularly limited, but Bobwhite may be mentioned from the viewpoint of high plant redifferentiation ability and high transformation efficiency. The wheat cells to which the vector according to the present invention is to be introduced is not particularly limited, but immature embryos (ones obtained by taking out the embryo part from immature seeds collected 14 to 15 days after flowering and removing the growing point (longer side: 1 to 2 mm)) are preferably used. The growing point is preferably removed because the cells in the growing point are relatively resistant to the agent and thus unsuitable to the selection of the transformed cells.

The above known techniques may be mentioned as the method for introducing the vector according to the present invention, but the method using particle bombardment is preferred from the viewpoint of relatively stable, easy production of transformants because the transformation of wheat is difficult. As the parameters of the particle bombardment, the conditions are described in Fig. 3 when using, for example, Biolistic (registered trademark) PDS-1000 / He particle delivery system (produced by Bio-rad).

A selection marker gene and a selection agent which are used to obtain the transformed cells having the introduced vector according to the present invention are not particularly limited, but a bar gene is preferred as the selection marker gene, and phosphinothricin or bialaphos is preferred as the selection agent. The combination of the HPT gene and Hygromycin B, and the combination of the NPTII gene and Geneticin (G418) can also be used in the method of the present invention.

The conditions for introducing the vector according to the present invention into wheat cells are not particularly limited, but the immature embryos of wheat as described above is desirably placed on a MSE3M medium with the scutellum facing upward when the vector according to the present invention is introduced to the immature embryos with the particle bombardment or the like (see Table 1 for the composition of the MSE3M medium).

The conditions for regenerating the plant from the transformed cells are not particularly limited, but the transformed cells are preferably cultured on a MSE3 medium from the next day to 14 days after the introduction of the DNA according to the present invention with the particle bombardment or the like; cultured on a MSR_P5 medium (selective medium) from 15 days; and more preferably cultured on a MS9_P5 medium (medium for rooting) after 70 days (see Tables 2, 3, and 4 for the compositions of the MSE3 medium, the MSR_P5 medium, and the MS9_P5 medium, respectively). Next, by transplanting the plant into the soil about 100 days after the day of the introduction of the DNA according to the present invention, the plant comes into flower 2 to 3 months after the transplantation, and further seeds can be collected 2 to 3 months after the flowering.

This method suppresses the expression of the wheat Vrs1 gene in wheat. This causes the fertility of florets which are supposed to degenerate in the wild type and thus increases the number of grains per spike.

**[Table 1]**

| Composition in 1 L of MSE3M Medium | |
|---|---|
| 5mg/ml Bialaphos | 1 ml |
| NH₄NO₃ | 1650mg |
| KNO₃ | 1900mg |
| 1M CaCl₂ | 3ml |
| 1M MgSO₄ | 1.5ml |
| 1M KH₂PO₄ | 1.25ml |
| MS-4 | 1 ml |
| KI × 1000 | 1ml |
| 10mM Fe-EDTA | 10ml |
| myo-inositol | 100mg |
| MS-5 | 1 ml |
| Asparagine | 150mg |
| Maltose | 150g |
| 0.1mg/ml 2.4-D | 25ml |
| pH | 5.8 |
| Agar | 8g |

**[Table 2]**

| Composition in 1 L of MSE3 Medium | |
|---|---|
| NH₄NO₃ | 1650mg |
| KNO₃ | 1900mg |
| 1M CaCl₂ | 3ml |
| 1M MgSO₄ | 1.5ml |
| 1M KH₂PO₄ | 1.25ml |
| MS-4 | 1 ml |
| KI × 1000 | 1 ml |
| 10mM Fe-EDTA | 10ml |
| myo-inositol | 10mg |
| MS-5 | 1 ml |
| Asparagine | 150mg |
| Sucrose | 30g |
| 0.1mg/ml 2.4-D | 25ml |
| pH | 5.8 |
| Agar | 8g |

**[Table 3]**

| Composition in 1 L of MSR_P5 Medium | |
|---|---|
| NH₄NO₃ | 1650mg |
| KNO₃ | 1900mg |
| 1M CaCl₂ | 3ml |
| 1M MgSO₄ | 1.5ml |
| 1M KH₂PO₄ | 1.25ml |
| MS-4 | 1 ml |
| KI × 1000 | 1 ml |
| 10mM Fe-EDTA | 10ml |
| Thiamine chloride | 40mg |
| Sucrose | 20g |
| 0.1mg/ml IAA | 5ml |
| 0.1 mg/ml BA | 10ml |
| pH | 5.8 |
| Agar | 8g |
| 5 mg/ml PPT after autoclaving | 1 ml |

**[Table 4]**

| Composition in 1 L of MS9_P5 Medium | |
|---|---|
| NH₄NO₃ | 825mg |
| KNO₃ | 950mg |
| 1M CaCl₂ | 1.5ml |
| 1M MgSO₄ | 0.75ml |
| 1M KH₂PO₄ | 0.625ml |
| MS-4 | 0.5ml |
| KI × 1000 | 0.5ml |
| 10mM Fe-EDTA | 5ml |
| myo-inositol | 50mg |
| MS-5 | 0.5ml |
| Asparagine | 75mg |
| Sucrose | 30g |
| pH | 5.8 |
| Agar | 8g |
| 5 mg/ml PPT after autoclaving | 1 ml |

The proportions of "KI × 1000", "0.1mg/mlBA", "MS-4", and "MS-5", which are added to the MSE3M medium, the MSE3 medium, the MSR_P5 medium, and the MS9_P5 medium, are described in Tables 5, 6, 7, and 8, respectively.

**[Table 5]**

| KI×1000 (100ml) | |
|---|---|
| KI | 83mg |

**[Table 6]**

| 0.1 mg/ml BA (100ml) | |
|---|---|
| BA | 10mg |
| 6N HCl | 2ml |

**[Table 7]**

| MS-4 (100ml) | | |
|---|---|---|
| H₃BO₃ | 620mg | (100*µ*M) |
| MnSO₄ 4H₂0 | 2230mg | (100*µ*M) |
| ZnSO₄ 7H₂0 | 860mg | (30*µ*M) |
| Na₂MoO₄ 2H₂O | 25mg | (1 *µ* M) |
| CuSO₄ 5H₂O | 2.5mg | (0.1 *µ* M) |
| CoCl₂ 6H₂0 | 2.5mg | (0.1 *µ* M) |

**[Table 8]**

| MS-5 (100ml) | | |
|---|---|---|
| nicotinic acid | 50mg | (4 *µ* M) |
| pyridoxic acid | 50mg | (2.5 *µ* M) |
| thiamine chloride | 10mg | (0.3 *µ* M) |
| glycine | 200mg | (27 *µ* M) |

### [Examples]

Although the present invention will be described below in more detail based on Examples, the present invention is not limited to the following Examples. Examples regarding barley were carried with the materials and method described below.

### <Barley>

For barley cultivars, Golden Promise (GP, JP 15923), Hanna (JP 15594)), Bonus (JP 15929), Haruna Nijo (HN, JP 67556), and Kanto Nakate Gold (KNG, JP 15436) were used as typical examples of the two-rowed barley variety. Morex (JP 46314), Azumamugi (AZ, JP 17209), and Hayakiso-2 (HK2, JP 18099) were used as typical examples of the six-rowed barley variety. The above varieties were obtained from the National Institute of Agrobiological Sciences (NIAS) Genebank (Japan).

### <RNA In Situ Hybridization Analysis>

A Vrs1 gene fragment including a part of 3'-UTR (300 bp), and a full-length cDNA of the HvHox2 gene (1072 bp) were amplified using primers specific to the sequences of the respective genes and using as a template a cDNA isolated from barley immature spikes. The primers used are described in Table 9.

**[Table 9]**

| **Experiment** | **Target gene** | **Primer** | | **5'-Sequence-3'** | **SEQ ID NO:** | **Vector** |
|---|---|---|---|---|---|---|
| Cloning | ***HvActin*** | Forward: | DN182500F276 | ATGTGGATATCAGGAAGGA | 7 | pBluescript II KS (+) |
| | | Reverse: | DN182500R533 | TCGCAACTTAGAAGCACTTCCG | 8 | |
| | ***Vrs1*** | Forward: | M111L15F84380 | CATACTTAACGCACGCCTAGAGATC | 9 | pCR4-TOPO |
| | | Reverse: | M111L15R85696 | TCACGATCCCTTCTTTCCCTC | 10 | |
| | ***HvHox2*** | Forward: | HvHox2 genome 1 U24 | GACGCACTCCCCTCCTTCAACTAG | 11 | pBluescript II KS (+) |
| | | Reverse: | HvHox2 genome 1260L24 | AACACAACACATCGTCTCTGAACT | 12 | |
| | | | | | | |
| qRT-PCR | ***HvActin*** | Forward: | DN182500F425 | AAGTACAGTGTCTGGATTGGAGGG | 13 | |
| | | Reverse: | DN182500R533 | TCGCAACTTAGAAGCACTTCCG | 8 | |
| | ***Vrs1*** | Forward: | Vrs1 genome 1651 U20 | CCGAGATAGCTGCTGCCGCC | 15 | |
| | | Reverse: | Vrs1 genome 1752L20 | TGCATCGCGGGCAATGGAGA | 16 | |
| | ***HvHox2*** | Forward: | HvHox2 genome 1040U24 | GCGTGGTCGAGTGGTTTAGCCTGT | 17 | |
| | | Reverse: | HvHox2 genome 1155L22 | CGCCTACAGATCATCCCAGCTT | 18 | |
| | | | | | | |
| in situ | ***Vrs1*** | Forward: | Vrs1 genome 1552U25 | GGTTTTTAGCATGAATTAGAGTTTA | 19 | pBluescript II KS (+) |
| hybridization | | Reverse: | Vrs1 genome 1827L25 | TATACAGGCTAAAAACCAAAGATTA | 20 | |
| | ***HvHox2*** | Forward: | HvHox2 genome 1 U21 | GACGCACTCCCCTCCTTCAAC | 21 | pBluescript II KS (+) |
| | | Reverse: | HvHox2 genome 1293L21 | GCTGAAGTAAAACATATACTA | 22 | |
| | | | | | | |
| Genotyping | Transgene | Forward: | GH-HYG-F1 | GATCGGACGATTGCGTCGCA | 23 | |
| | | Reverse: | GH-HYG-R2 | TATCGGCACTTTGCATCGGC | 24 | |
| | | Forward: | pAct3 PCR | GATCTTTCTTTCTTCTTTTTGTGGGTAGAATTTGAATCCC | 25 | |
| | | Reverse: | TnosRv2 PCR | CGGGACTCTAATCATAAAAACCCATCTCATAAATAACGTC | 26 | |
| | | Forward: | pUbi3 PCR | AGCTATATGTGGAIIIIIIIAGCCCTGCCTTCATACGCTA | 27 | |
| | | Reverse: | TnosRv2 PCR | CGGGACTCTAATCATAAAAACCCATCTCATAAATAACGTC | 25 | |

The obtained PCR product was cloned by inserting the PCR product into a pBluescript II KS (+) vector (produced by Stratagene). In two clones different in insertion direction, the Vrs1 gene was cleaved with Not1, and the HvHox2 gene was cleaved with EcoRI. These were then used as templates to produce an antisense probe and a sense probe with a T3 RNA polymerase or T7 RNA polymerase. RNA in situ hybridization was carried out as described in NPL 1.

### <Immunohistochemical Staining>

Anti-barley VRS1 antibodies were prepared by immunizing rabbits with a synthetic peptide (CXVPEWFLA, SEQ ID NO: 27). C (cysteine residue) is the amino acid which is introduced for the disulfide bond with a vehicle or the like. X represents aminohexanoic acid and "VPEWFLA" is derived from the amino acids at positions 216 to 222 of the VRS1 protein.

The immunohistochemical staining of immature spikes at the awn primordium stage was carried out as described in Yamaji, N. and Ma, J. F., Plant Physiol, 2007, volume 143, 1306-1313 pages.

### <RNA Extraction and Quantitative Real Time PCR>

As described in Kirby, E. J. M., Appleyard, M., "Cereal development guide", 1981, Kenilworth: Cereal Unit, the development process of immature spikes was observed under a stereoscopic microscope to classify the immature spikes, and about ten immature spikes were collected for each developmental stage. Total RNA was then extracted from 50 mg wet weight of the sample at each developmental stage using TRIzol (produced by Invitrogen). Independent RNA extraction was carried out 3 times for each sample (biological replicates).

The RNA was quantified using NanoDrop (produced by Thermo Fisher Scientific). In order to avoid DNA contamination, the RNA was treated with RNase-free DNase (produced by TaKaRa) according to the description of the manufacturer's instruction. Next, first-strand cDNA was synthesized using SuperScript III (produced by Invitrogen), and a cRNA prepared from 25 ng of the RNA was used as a template. The transcription level of each gene was determined by the quantitative real-time PCR (qRT-PCR) method using the StepOne Real-Time PCR system (produced by Applied-Biosystems) and the THUNDERBIRD SYBR qPCR Mix kit (produced by Toyobo Co., Ltd.) according to the description of the respective manufacturer's instructions. The primers used in the qRT-PCR are described in Table 9. Amplicons were fractionated by electrophoresis using agarose gel and visualized by ethidium bromide staining. The qRT-PCR analysis was carried out at least 3 times for each sample.

Each gene fragment was cloned by inserting the gene fragment into pCR4-TOPO (produced by Invitrogen) or pBluescript II KS (+) (produced by Stratagene). The plasmid DNA to which each gene fragment was inserted was used to make a calibration curve required for determining of the absolute quantity.

### <Transformation of Barley>

To prepare an RNAi construct, a barley Vrs1 gene fragment (628 bp) was amplified using a specific primer (the primers used are described in Table 9). The obtained PCR products in the forward and reverse directions were inserted into vector pIPKb008 and vector pIPKb009 and cloned. In vector pIPKb008 and vector pIPKb009, the expression of the transgene can be regulated under the rice actin promoter and the CaMV35S promoter, respectively (see Himmelbach, A., et al., Plant Physiol, 2007, volume 145, 1192-1200 pages).

The RNAi construct was introduced into barley immature embryos (Golden Promise cv.) by the Agrobacterium method using Agrobacterium tumefaciens strain AGL-1 as described inHensel, G. et al., J Plant Physiol, 2008, volume 165, 71-82 pages.

### (Example 1)

### <Analysis on Expression of Barley Vrs1 Gene and HvHox2 Gene by RNA In Situ Hybridization>

The expression of the Vrs1 gene in immature spikes at various developmental stages of two-rowed barley cv. (see Bonus, Kirby, E. J. M. and Appleyard, M., "Cereal development guide", 1981, Kenilworth: Cereal Unit) was detected by RNA in situ hybridization. The obtained results are shown in Fig. 4.

The probe used in this RNA in situ hybridization is a barley Vrs1-specific probe (about 300 bp of 3'UTR in the Vrs1 gene, see Fig. 5). The sequence of the probe is not present in the HvHox2 gene or the HvHox3 gene which is a gene homologous to the Vrs1 gene (see Sakuma, S. et al., Funct Integr Genomics, 2010, volume 10, 123-133 pages). The HvHox2 gene is a gene with the highest homology to the Vrs1 gene, and the HvHox3 gene is a gene with the second highest homology to the Vrs1 after the HvHox2 gene (Matsumoto, T, et al., Plant Physiol, 2011, volume 156, 20-28 pages). Therefore, the RNA in situ signal from this probe only indicates the expression of the Vrs1 mRNA. When the sense probe was actually used in two-row spikes, no signal was detected (see G of Fig. 4). A barley Vrs1 gene null mutant, hex-v.3, which has six-row spikes was used as a negative control and as a result, no signal was detected when this mutant was hybridized with the barley Vrs1-specific probe (see H in Fig. 4). This confirms that the Vrs1 probe can be used without the cross-hybridization with the sequence of HvHox2 mRNA or other homologous genes in the RNA in situ hybridization.

Barley spikes mature through the developmental stages of triple-mound stage, glume primordium stage, lemma primordium stage, stamen primordium stage, awn primordium stage, white anther stage, green anther stage, yellow anther stage, and flag-leaf stage.

As apparent from the results shown in Fig. 4, Barley Vrs1 mRNA was detected in the meristems of lateral spikelets of immature spikes at the stage of the differentiation of the spikelet primordium into three different spikelet meristems (triple-mound stage and glume primordium stage) (spike length: 1 to 1.5 mm, seeAandBofFig. 4). Therefore, as already disclosed by the present inventors (as described in NPL 1), it was confirmed that the barley Vrs1 gene was specifically expressed in the meristems of lateral spikelets.

Next, the expression of the Vrs1 gene was detected by RNA in situ hybridization at the white anther stage (spike length: 10 to 15 mm) at which floral organs were completely differentiated. The obtained results are shown in D and F of Fig. 4.

As apparent from the results shown in D and F of Fig. 4, at the white anther stage, the expression of the barley Vrs1 gene is localized in the lemma and the palea and most highly localized particularly in the pistil. The signal of RNA in situ hybridization was at about background level in the anther tissue. Furthermore, no expression of the Vrs 1 gene was detected in the glume. In addition, the expression of the Vrs1 gene as strong as that in the pistil was also detected in the rachilla having completely suppressed, undevelopedfloretsinbarley. Therefore, the barley Vrs1 gene was found to be specifically expressed in floral organs.

However, no expression of the Vrs1 gene was detected in the meristems of central spikelets at the triple-mound stage and glume primordium stage. In addition, no expression of the barley Vrs1 gene was detected in any tissue of central spikelets at the white anther stage.

Next, RNA in situ hybridization using a HvHox2 gene-specific probe was carried out. The obtained results are shown in Fig. 6.

Since the hex-v.3 mutant is deficient in all the DNA sequence of the barley Vrs1 gene, the homologous sequence of the Vrs1 gene is not detected in RNA in situ hybridization with the HvHox2 probe (see NPL 1). In consideration of this point, the immature spikes of this mutant were used to examine the expression of the HvHox2 by RNA in situ hybridization. The expression level of the HvHox2 gene is relatively low, and thus the expression of the HvHox2 gene cannot be easily detected by RNA in situ hybridization. Accordingly, in order to enhance the signal of the HvHox2 gene, a probe including the HvHox2 cDNA full length was used to perform RNA in situ hybridization (see Fig. 5).

As shown from the results in Fig. 6, the signal in the vascular tissue of the pedicel (j oint between the rachis and the spikelet) on two lateral spikelets was detected by RNA in situ hybridization using the HvHox2 specific probe (see B, D, and F of Fig. 6). The signal was localized in provascular cells at the white anther stage (see E and G of Fig. 6).

On the other hand, the signal of the HvHox2 expression was detected at the white anther stage, but no HvHox2 expression was detected at the triple-mound stage. No signal of HvHox2 was detected in other parts of central spikelets or immature spikes, either. The sense probe was not hybridized in any structure (see C of Fig. 6).

### (Example 2)

### <Analysis on Expression of Barley Vrs1 Protein in Barley by Immunostaining>

In order to examine the tissue-specific localization of the barley VRS1 protein, the immunostaining using an anti-VRS1 antibody was carried out. As a result, the signal of the barley VRS1 protein was detected in the lateral spikelets of the two-row spike (Bonus) at the awn primordium stage (see A and B of Fig. 7). No signal was detected in the central spikelet. These data correspond with the localization of the barley Vrs1 mRNA in the lateral spikelets as described above, indicating that the Vrs1 mRNA is translated into the VRS1 protein in the two-row spike.

PredictProtein database (http://www.predictprotein.org/) analysis predicts that the VRS1 protein and the HvHOX2 protein have nuclear localization signals of "RRRRRRSAR" (SEQ ID NO: 28) and "RPRARRRRRRAAR" (SEQ ID NO:29), respectively, which leads to the assumption that the targets of these two proteins are present in the nucleus. In order to confirm the localization of VRS1 in the nucleus, the coimmunostaining with DAPI, a dye for labeling DNA, was then carried out. Barley VRS1 was localized in nucleus (see C of Fig. 7).

In the negative control, however, only the autofluorescence signal was detected in the vascular tissue of the rachis (see D of Fig. 7). In the hex-v.3 mutant deficient in the Vrs1 gene, only strong autofluorescence was detected in the vascular bundles of the rachis and the spikelet (see E and F of Fig. 7). In the central spikelets and lateral spikelets of hex-v.3 as well as the nuclei thereof, however, the barley VRS1 protein was not able to be detected by an anti-barley VRS1 antibody (see G of Fig. 7). Since the strong signal as detected in the rachis in G of Fig. 7 was also observed in the negative control without using the anti-barley VRS1 antibody, the strong signal is autofluorescence.

### (Example 3)

### <Analysis 1 of Expression of Barley Vrs1 Gene and HvHox2 Gene by qRT-PCR>

In order to analyze the expression level of the barley Vrs1 gene and the HvHox2 gene in barley tissues, immature spikes were isolated from a two-row cultivar (Golden Promise (GP)) at the green anther stage. Awn tissues, stem tissues, and leaf tissues were also isolated from this variety at the flag-leaf stage. The quantitative reverse transcription PCR (qRT-PCR) analysis was then carried out. The obtained results are shown in Fig. 8.

In the qRT-PCR analysis, the PCR primers specific to 3'UTR of the respective genes were designed in order to compare the gene expression level at different developmental stages. It has been confirmed that no homology is observed between these primers and the HvHox3 gene. Furthermore, in the qRT-PCR analysis, the RNA expression level in each sample was normalized on the basis of the expression of the HvActin gene which is constitutively expressed during the developmental stages of spikes.

The barley Vrs1 gene was expressed only in the lateral spikelets as apparent from the results shown in Fig. 8. This results correspond with the tissue-specific expression pattern of the barley Vrs1 detected by RNA in situ hybridization (see Fig. 4). In addition, the expression of the HvHox2 gene was observed in the central spikelets,lateralspikelets,awntissues,andleaftissues, but the transcription level in the lateral spikelets was found to be highest. The transcription levels of the barley Vrs1 and HvHox2 were 4.9 × 10⁴ copies and 3.4 × 10⁴ copies per µg of total RNA, respectively, and they were similar in the lateral spikelets at the green anther stage.

### <Analysis 2 of Expression of Barley Vrs1 Gene and HvHox2 Gene by qRT-PCR>

Next, the absolute quantitative reverse transcription PCR (absolute qRT-PCR) was carried out on two two-row spike cultivars and two six-row spike cultivars in order to compare the expression level of the barley Vrs1 gene with the expression level of the HvHox2 gene though various developmental stages of the spike. The obtained results are shown in Figs. 9 to 12.

As apparent from the results shown in Fig. 9, in Bonus, a two-row spike cultivar having the Vrs1.b3 allele, the transcription level of the Vrs1 gene was significantly higher than the transcription level of the HvHox2 gene (P < 0.05) at the lemma primordium stage (P = 0.0104), the stamen primordium stage (P = 0.0040), the awn primordium stage (P = 0.0096), and the white anther stage (P= 0.0006) .

Specifically, the expression of the barley Vrs1 gene at the lemma primordium stage and the stamen primordium stage (mRNA copy number per µ of total RNA: 5.1 × 10⁵ at the lemma primordium stage, 5.8 × 10⁵ at the stamen primordium stage) was 20 times or more of that of the HvHox2 gene (2.4 × 10⁴ at the lemma primordium stage, 2.7 × 10⁴ at the stamen primordium stage).

The expression of Vrs1 (7. 5 × 10⁵ at the awn primordium stage, 5.8 × 10⁵ at the white anther stage) was three times or more of that of the HvHox2 gene at the awn primordium stage and the white anther stage (2.0 × 10⁵ at the awn primordium stage, 2.3 × 10⁵ at the white anther stage).

Furthermore, no significant difference (P > 0.05) in transcription level was observed at the triple-mound stage and the glume primordium stage, but the expression of the barley Vrs1 gene was higher than that of the HvHox2 gene. However, the expressions of these genes are at similar levels at the green anther stage and the yellow anther stage.

Like in Bonus, the expression patterns of the barley Vrs1 gene and the HvHox2 gene are also similar in Kanto Nakate Gold (KNG), a two-row spike cultivar possessing the Vrs1.b3 allele, and the expression of the barley Vrs1 mRNA was highest from the awn primordium stage to the white anther stage while the differentiation to the pistil started (see Fig. 10). On the one side, the expression of the HvHox2 mRNA was highest from the white anther stage to the yellow anther stage.

In Azumamugi (AZ), a six-row cultivar, the transcription level of the barley Vrs1 gene was higher than that of the HvHox2 gene. AZ possesses the vrs1.a1 allele encoding a truncated VRS1 protein (see NPL 1) (see Fig. 11).

The expression of the barley Vrs1 gene in Hayakiso 2 (HK2), another six-rowed cultivar, was one-fifth of that in Bonus and one-third of that in AZ (see Fig. 12).

HK2 possesses the vrs1. c allele, which does not alter the deduced amino acid sequence encoded by the barley Vrs1 gene (see Saisho, D. et al., Breed Sci, 2009, volume 59, 621-628 pages). Therefore, the expression control region of the Vrs1 gene in the vrs1.c allele may be mutated.

The above results suggested that the decreased barley Vrs1 expression found in HK2 (the expression level at the awn primordium stage was up to 1.5 × 10⁵ copies per µg) failed to sufficiently suppress the fertility of lateral spikelets, and thus produced fully fertile lateral spikelets.

In addition, no significant difference in the expression level of the HvHox2 gene was observed between AZ and HK2. Therefore, these data show that the development of six-row spikes in HK2 results from the decreased transcription level of the Vrs1 gene in this cultivar.

### (Example 4)

### <RNA Interference against Barley Vrs1 Gene>

Golden Promise, a two-row cultivar, was transformed with a hairpin RNA interference (RNAi) construct specific to the barley Vrs1 gene. A construct under the control of the rice Actin1 promoter was used to produce 50 independent T0 plants. As a result, it was observed that three T0 plants (BG87/1E18, BG87/1E35, and BG87/2E4) bore fertile lateral spikelets that yielded grains. In addition, a construct under the control of the CaMV 35S promoter was used to produce 47 independent T0 plants. As a result, no T0 transgenic plant bearing fertile lateral spikelets was observed.

Next, seven lines of T1 were produced from a T0 plant in which the expression of shRNA was under the control of the rice Actin1 promoter and the CaMV 35S promoter. Then, co-segregation analysis on development of lateral spikelets and transgenes was carried out. The obtained results are shown in Figs. 10 and 13.

**[Table 10]**

| Plant ID | Generation | Transgene | Number of plants | Number of spikes | Number of lateral spikelets | Length (mm) | | | | Fertility (%) | |
|---|---|---|---|---|---|---|---|---|---|---|---|
| | | | | | | Spike | Lateral spikelet (LS) | Lemma in LS | Awn in LS | Main spikelet | Lateral spikelet |
| Golden Promise | WT | - | 3 | 9 | 27 | 59.0±0.8 | 9.2±0.2 | 9.2±0.2 | 0 | 79.6±12.4 | 0 |
| Non-transgenic line | T1 | - | 3 | 9 | 27 | 61.6±2.1 | 8.1±0.1 | 8.1±0.1 | 0 | 75.6±11.3 | 0 |
| BG78/1E01 | T1 | + | 6 | 18 | 54 | 50.0±2.4 | 10.1±1.0 | 8.6±0.2 | 1.5±1.0 | 69.9±3.3 | 0 |
| BG78/1E02 | T1 | + | 6 | 18 | 54 | 57.9±3.6 | 17.2±1.1 | 9.1±0.1 | 8.1±1.1 | 68.6±9.9 | 0 |
| BG78/2E02 | T1 | + | 6 | 18 | 54 | 61.9±3.7 | 12.2±0.7 | 9.2±0.1 | 3.0±0.8 | 60.5±3.7 | 0 |
| BG78/3E02 | T1 | + | 6 | 18 | 54 | 59.1±5.0 | 11.8±1.8 | 8.7±0.4 | 3.1±1.6 | 75.9±7.7 | 0 |
| Non-transgenic line | T1 | - | 3 | 9 | 27 | 60.6±2.1 | 8.4±0.1 | 8.4±0.1 | 0 | 73.6±14.3 | 0 |
| BG87/1E18 | T1 | + | 7 | 21 | 63 | 67.6±4.5 | 15.6±1.8 | 8.8±0.3 | 6.8±1.6 | 66.0±7.7 | 2.0±1.8 (0-38*) |
| BG87/1E35 | T1 | + | 7 | 21 | 63 | 66.7±2.0 | 27.1±2.6 | 9.9±0.2 | 17.2±2.5 | 76.6±6.8 | 1.8±0.7 (0-14*) |
| BG87/2E04 | T1 | + | 7 | 21 | 63 | 73.0±3.5 | 25.1±1.8 | 9.8±0.1 | 15.3±1.9 | 78.3±6.7 | 0.3±0.3 (0-5*) |

| | | | | | | | | | | | |
|---|---|---|---|---|---|---|---|---|---|---|---|
| The data are expressed as the "mean ± standard error." * indicates "the minimum value to the maximum value." | | | | | | | | | | | |

As shown in the results in Table 10, the grain formation was observed only in the following three lines. The fertility rate is expressed in % as the ratio of flowers bearing seeds as a numerator to total flowers as a denominator.
BG87/1E18 (the range of the fertility rate in lateral spikelets: from 0% to 38%, large individual differences)
BG87/1E35 (the range of the fertility rate in lateral spikelets: from 0% to 14%)
BG87/2E04 (the range of the fertility rate in lateral spikelets: from 0% to 5%).

Therefore, the grain formation ability in lateral spikelets was found to descend to T1 plants (see Fig. 13). As shown in Table 10, the Vrs1 gene was expressed only in lateral spikelets, and no change in shape was observed in other organs than the lateral spikelets.

Next, qRT-PCR was carried out using a RNA isolated from immature spikes at the white anther stage to evaluate the correlation between the expression level of Vrs1 mRNA and the development of lateral spikelets. The obtained results are shown in Fig. 14.

As apparent from the results shown in Fig. 14, the expression levels of the barley Vrs1 gene in T1 plants derived from BG78/3E02, BG87/1E18, BG87/1E35, and BG87/2E04 lines (2.0 × 10⁵, 1.6 × 10⁵, 2.1 × 10⁵, and 2.1 × 10⁵ copies / 1 µ RNA, respectively) were about half of that of wild-type Golden Promise (3.5 × 10⁵) or those of non-transgenic plants separated from T1 family (4.0 × 10⁵ and 3.9 × 10⁵).

The expression levels of the barley Vrs1 gene in wild-type plants and T1 plants derived from BG78/1E01, BG78/1E02, and BG78/2 E02 lines were very similar. Moreover, no significant difference in expression level of the HvHox2 gene was observed between the wild-type plants and the transgenic plants (see Fig. 15).

These results revealed that the introduction of the DNA encoding the antisense RNA complementary to the transcript of the barley Vrs gene to suppress the expression of the barley Vrs1 gene gives the fertility to florets in lateral spikelets and accordingly can increase the number of grains per spikelet.

The VRS1 protein and its homologous protein, the HvHOX2 protein, are assumed to function as transcription factors because they both have the homeodomain (HD) motif and the leucine zipper as shown in Fig. 5. In fact, as also shown in Example 2, the barley VRS1 protein is localized in the nucleus, strongly suggesting that the barley VRS1 protein function as a transcription factor.

In view of the function of the rice OsHOX14 protein or the Arabidopsis thaliana HD-Zip I protein, which is an ortholog of the HvHOX2 protein, the HvHOX2 protein is assumed to function as a transcriptional activator responsible for the growth of lateral spikelets.

As described in NPL 1, however, 18 independent six-row spike mutants have single amino acid substitutions in the VRS1 protein. These mutants indicate that the HD of the wild-type VRS1 protein in combination with a cis-element functions as a negative regulator for the development of the flower organs in lateral spikelets.

The barley VRS1 protein is deficient in the C-terminal motif of an 8-amino acid length which is highly conserved in the protein orthologous to HvHOX2. This proposes that the transcriptional activation potential on downstream genes , which is observed in the protein orthologous to HvHOX2, is lost in the barley VRS1 protein (see Sakuma, S. et al., Funct Integr Genomics, 2010, volume 10, 123-133 pages).

Therefore, the results described in these findings and Examples above strongly suggests that the HD motifs of the barley VRS1 protein and the HvHOX2 protein are highly conserved and thus these proteins compete with each other in binding to the cis-element of the downstream gene responsible for the development of lateral spikelets.

In fact, as found in Example 3, in fertile central spikelets, no barley Vrs1 gene is expressed and the HvHox2 gene is weakly expressed at a similar level to that in leaves and stems (see Fig. 8). Therefore, it is strongly indicated that, in central spikelets, even such relatively low expression of the HvHox2 gene does not cause the expression of the barley VRS1 protein, a competitor, and thus can sufficiently enhance the development of central spikelets, thereby enabling grain bearing.

Although the HvHox2 gene and the barley Vrs1 gene are both expressed in lateral spikelets, the expression of the barley Vrs1 gene was 10 times higher than that of the HvHox2 gene at the early stage of spike development (see Figs. 9 to 12). Therefore, it is strongly suggested that such high expression of the barley VRS1 protein prevents the binding of the HvHOX2 protein to its binding site (cis-element etc.) to terminate the expression of the development gene involving in the enhancement of lateral spikelet formation, thereby inhibiting grain bearing in lateral spikelets.

The HD-Zip protein, a protein orthologous to HvHOX2, is expected to form a dimer through the leucine zipper before binding to a specific DNA sequence (see Sessa, G. et al., EMBO J, 1993, volume 12, 3507-3517 pages). The HD-Zip I and II proteins have also found to form a homodimer or heterodimer with proteins of the same class (see Meijer, A.H. et al., Mol Gen Genet, 2000, volume 263, 12-21 pages).

Therefore, the expressions of the HvHox2 gene and the barley Vrs1 gene at very similar levels cause the barley VRS1 protein and the HvHOX2 protein to form a heterodimer through the leucine zipper. It is then strongly indicated that the heterodimer formation occupies the cis-element to reduce the amount of a functional HvHOX2 protein homodimer in the nuclei of lateral spikelets, thereby inhibiting grain bearing in the lateral spikelets.

However, described above is the function of the Vrs1 and HvHox 2 genes found in barley. Unlike plants belonging to Triticum, such as wheat, barley has the unique characteristic of bearing only one floret per spikelet. On the other hand, wheat and the like bear a plurality of florets per spikelet.

Accordingly, barley and wheat are plants of the same Poaceae family, but their spikelet shapes are very different. Of course, wheat has no six-rowed or two-rowed spikelets, and has no lateral spikelets either, and thus the relation between the sterility of florets and the wheat gene homologous to the barley Vrs1 gene was not considered at all in wheat (Sakuma, S., et al., Plant Cell Physiol, 2011, volume 52, 738-749 pages).

Then, in wheat, the genes homologous to the barley Vrs1 gene and the HvHox2 gene are each isolated by the methods described in Examples 5 to 9 below, and analysis was made on the positions of these genes on the chromosomes , the motifs of the proteins encoded by these genes, and the expression patterns of these genes. In addition, whether the number of grains is increased by suppressing the function of the wheat Vrs1 gene by RNAi was examined. The wheats used in Examples 5 to 9 below are as follows.

### <Wheat>

Einkorn wheats, KT1-1 (Triticum monococcum L. ssp. boeoticum) and KT3-5 (Triticum monococcum L. ssp. monococcum, diploid wheat), were used in gene cloning, quantitative RT-PCR, and situ hybridization which are described below. A group of 115 F10 recombination inbred lines (RILs) derived from a cross of KT1-1 and KT3-5 was used for gene mapping described below. Chinese spring (CS, hexaploid wheat), a variety of Triticum aestivum, was used in gene cloning and qRT-PCR which are described below. The nulli-tetrasomic lines, ditelosomic lines, and deletion lines of CS having a homoeologous group-2 chromosome were used to determine the position of the Vrs1 gene on the wheat chromosomes.

These wheats were all provided by National Bioresource Project (NBRP)-wheat. Bobwhite, a Triticum aestivum, was provided by CIMMYT (Accession number: SH 98 26, see Pellegrineschi A. et al., Genome, 2002, volume 45, 421-430 pages) and used as the target for the introduction of the RNAi transgene against the wheat Vrs1 gene described below.

### (Example 5)

### <Isolation and Mapping of Vrs1 Gene Derived from Wheat or the Like>

To isolate the wheat Vrs1 gene, a genomic DNA was extracted from a diploid wheat (experimental line KT-3-5) according to the method described in Komatsuda et al. , Genome, 1998, volume 41, 680-685 pages. The obtained genomic DNA was used as a template to perform PCR. A PCR primer was designed with Oligo 5 software (W. Rychlick, National Bioscience, Plymouth, MN, USA) and synthesized by outsourcing to BEX Co., Ltd. The synthesized primer sequences are described in Table 11.

**[Table 11]**

| Experiment | Target gene | Primer | 5'-Sequence3' | SEQ ID NO: | Vector |
|---|---|---|---|---|---|
| PCR | *TmVrs1* | T.aestivum_contig_3876bp 2225U21 | CAAAGAATCCAGAGTCTCGAA | 30 | |
| | | RILWA1-117_Hox1 1579L21 | CGTGTCAATTTCAAATCACCC | 31 | |
| | *TmHox2* | M664D5_rev 97901L16 | CAGAGGGCCGATCTTT | 39 | |
| | | RILWA1-117_Hox2 1900L21 | TGCAAGCTTTGCCATTAATCA | 40 | |
| | *TaVrs1* | RILWA1-117_Hox1 1223U21 | TGTGCGTCCCCGAGTGGTTTT | 65 | |
| | (for BAC screening) | RILWA1-117_Hox1 1393L21 | CTTTGGAGGGATTTGTGGAGG | 37 | |
| | | | | | |
| Cloning | *TmVrs1* | RILWA1-117_Hox1 216U21 | CGCACTTTCCCCTCCTTCAAC | 32 | pBluescript II KS(+) |
| | | RILWA1-117_Hox1 1588L21 | TGTCCAGCTCGTGTCAATTTC | 49 | |
| | *TmHox2* | RILWA1-117_Hox2 678U21 | CACTCCCCTTCCTTACTTAAC | 50 | pBluescript II KS(+) |
| | | RILWA1-117_Hox2 1900L21 | TGCAAGCTTTGCCATTAATCA | 40 | |
| | | | | | |
| qRT-PCR | *TmVrs1* | RILWA7-117_Hox1 1335U21 | ATAGGCGCTATAGCTGCTCGG | 51 | |
| | | RILWA1-117_Hox1 1393L21 | CTTTGGAGGGATTTGTGGAGG | 37 | |
| | *TmHox2* | TmHox2 genome 873U24 | GCGTGGTCGAGTGGTTTAGCCTGT | 52 | |
| | | TmHox2 genome 977L24 | GCCCTAGAGATCACCCCAGCTTAA | 53 | |
| | *TaVrs1-A* | TaVrs1_A_7574U24 | ACAAAATAGGCGCTATAGCTGCTC | 66 | |
| | | TaVrs1_A_1676L24 | CGGGACAGATGATTTCTAGAGGTT | 67 | |
| | *TaVrs1-B* | TaVrs1_B_1131U21 | ACAAAATAGGTGCGTTAATTG | 68 | |
| | | TaVrs1_B_1227L21 | GGTATTTCCTGATTCTGCAGC | 69 | |
| | *TaVrs1-D* | TaVrs1_D_1358U20 | GCTATGCTATGGCTGCATGC | 70 | |
| | | TaVrs1_D_1438L20 | GATTTAGCGGCGGCCTTTTC | 71 | |
| | *Actin* | DN182500F425 | AAGTACAGTGTCTGGATTGGAGGG | 13 | |
| | | DN182500R533 | TCGCAACTTAGAAGCACTTCCG | 8 | |
| | GUS linker | GUS linker_225F21 | CGACTGGGCAGATGAACATGG | 72 | |
| | | GUS linker_341R21 | TTTCCCCGTTGACTGCCTCTT | 73 | |

The amplification reaction was performed in 50 µl of the reaction system containing 1.25 U ExTaq polymerase (produced by TaKaRa), 1 × Ex Taq polymerase buffer, 0. 3 µM of each primer, 200 µM of dNTP, 2 mM of MgCl₂, 2.5% (v/v) dimethyl sulfoxide (DMSO), and 100 ng of genomic DNA. In addition, each amplification reaction was performed under the following conditions: a denaturation step at 94 °C for 5 minutes; then 30 cycles of 94 °C for 30 seconds, 55 °C for 30 seconds, and 72 °C for 60 seconds; and final incubation at 72 °C for 7 minutes.

Then, the obtained PCR product was purified by QIAquick PCR Purification Kit (produced by Qiagen) and subjected to cycle sequencing using BigDye Terminator Kit (produced by Applied Biosystems). Specifically, a sequencing reaction solution was purified by Agencourt CleanSEQ (produced by Beckman), and analyzed with ABI PRISM 3130 Genetic Analyzer (produced by Applied Biosystems). The sequences of the primers used for sequencing are described in Table 12.

**[Table 12]**

| Experiment | Target gene | Primer | 5'-Sequence-3' | SEQ ID NO: | Vector |
|---|---|---|---|---|---|
| Sequencing | *TmVrs1* | RILWA1-117_Hox1 216U21 | CGCACTTTCCCCTCCTTCAAC | 32 | |
| | | RILWA1-117_Hox1 399L21 | TCCACGTAGGAGGAACCAAAG | 33 | |
| | | RILWA1-117_Hox1 483U21 | ATGATGCAGCTGCAGTAGCTT | 34 | |
| | | RILWA1-117_Hox1 925L21 | AGTGTGTATGAGCAAGCTTAC | 35 | |
| | | RILWA1-117_Hox1 1237U21 | TGGTTTTTCGCATGAATTAGC | 36 | |
| | | RILWA1-117_Hox1 1393L21 | CTTTGGAGGGATTTGTGGAGG | 37 | |
| | | R1LWA1-117_Hox1 1539L21 | CCAAAGATTAAAATCGCCAGA | 38 | |
| | | RILWA1-117_Hox1 1579L21 | CGTGTCAATTTCAAATCACCC | 31 | |
| | *TmHox2* | RILWA1-117_Hox2 529U21 | ACTTGGCTGCACCATACGTTT | 41 | |
| | | RILWA1-117_Hox2 529L21 | AAACGTATGGTGCAGCCAAGT | 42 | |
| | | RILWA1-117_Hox2 699U21 | ATCAAATAGTGCCTTGCGGTG | 43 | |
| | | RILWA1-117_Hox2 852U21 | CTTTGGTTCCTCCTACGTGGA | 44 | |
| | | RILWA1-117_Hox2 1302U22 | AGAGCAAGCTTATGGAGGAGGA | 45 | |
| | | RILWA1-117_Hox2 1466L22 | TGGCCATATGTGCGTCAGTCAG | 46 | |
| | | RILWA1-117_Hox2 1745U24 | GCGTGGTCGAGTGGTTTAGCCTGT | 47 | |
| | | RILWA1-117_Hox2 1842L21 | TCACCCCAGCTTAATTAACGC | 48 | |
| | | RILWA1-117_Hox2 1900L21 | TGCAAGCTTTGCCATTAATCA | 40 | |
| | | | | | |
| In situ hybridization | *TmVrs1* | RILWA1-117_Hox1 1237U21 | TGGTTTTTCGCATGAATTAGC | 36 | pBluescript II KS(+) |
| | | RILWA1-117_Hox1 1539L21 | CCAAAGATTAAAATCGCCAGA | 38 | |
| | | | | | |
| RNAi | *TaVrs1-A* | TaVrs1_RNAi_F25 | CACCTGGTTTTTCGCATGAATTAGC | 54 | pENTR/D-TOPO |
| | | TaVrs1_RNAi_R21 | CCAAAGATTAAAATCGCCGGA | 55 | pANDA-β |
| | Transgene | GUS linker_1R21 | GCCGACGCGAAGCGGGTAGAT | 74 | |
| | (for detection) | TaVrs1_RNAi_R21 | CCAAAGATTAAAATCGCCGGA | 55 | |
| | Transgene | GUS linker_891F21 | GCGCGTTGGCGGTAACAAGAA | 75 | |
| | (for detection) | TaVrs1_RNAi_R21 | CCAAAGATTAAAATCGCCGGA | 55 | |
| | Transgene | bar_FW2 | GTCTGCACCATCGTCAACC | 76 | |
| | (for detection) | bar_RV2 | GAAGTCCAGCTGCCAGAAAC | 77 | |

The sequence of the genomic DNA of diploid wheat Vrs1 gene (TmVrs1 gene), which is revealed by the above analysis for the first time, is described in SEQ ID NO: 1; the sequence of the cDNA encoded by the genomic DNA is described in SEQ ID NO: 2; and the amino acid sequence of the protein (TmVRS1 protein) encoded by the genomic DNA and the cDNA is described in SEQ ID NO: 3. Furthermore, the sequence of the genomic DNA of the Hox2 gene derived from wheat (TmHox2 gene) is described in SEQ ID NO: 4; the sequence of the cDNA encoded by the genomic DNA is described in SEQ ID NO: 5; and the amino acid sequence of the protein (TmHOX2 protein) encoded by the genomic DNA and the cDNA is described in SEQ ID NO: 6.

To identify the positions of the TmVrs1 gene and the TmHox2 gene on the wheat chromosomes, the genotype of the RILs of Einkorn wheat (cross of T. boeoticum and T. monococcum) was determined using polymorphic CAPS and dCAPS markers. Then, the data from the markers was combined to make a genetic map (linkage map) according to the method described in Hori et al. (Breeding Science, 2007, volume 57, 39-45 pages). The obtained results are shown in Fig. 16.

The genetic map shown in Fig. 16 indicated that the TmVrs1 gene is positioned in the long arm of chromosome 2Am, and the TmHox2 gene is positioned in the short arm of chromosome 2Am. Therefore, it was found that the order of the Vrs1 gene and the Hox2 gene was highly conserved between barley (chromosome 2H) and wheat (chromosome 2Am).

Vrs1 homologous genes (TaVrs1-A, TaVrs1-B, TaVrs1-D) were isolated from Triticum aestivum by a similar analysis to the above TmVrs1 gene analysis. Specifically, the BAC library of a hexaploid wheat (Chinese Spring) was screened by the PCR using a primer designed based on the TmVrs1 3'UTR sequence to identify 14 positive clones. Then, about 2 kb of the TaVrs 1 gene region in 14 clones was sequenced.

As a result, the phylogenetic analysis revealed for the first time that three haplotypes exist. The sequences of the genomic DNAs of these hexaploid wheat Vrs1 genes (TaVrs1-A gene, TaVrs1-B gene, and TaVrs1-D gene) are described in SEQ ID NOS: 56, 59, and 62, respectively. The sequences of the cDNAs encoded by the genomic DNAs are described in SEQ ID NO: 57, 60, and 63, respectively. The amino acid sequences of the proteins encoded by the genomic DNAs and the cDNAs are described in SEQ ID NO: 58, 61, and 64, respectively.

The heteroploid lines of the CS nulli-tetrasomic line, the CS ditelosomic line, and the CS deletion line were analyzed by the PCR using primers specific to the respective homologous genes to determine the positions of the three haplotypes (TaVrs1-A, TaVrs1-B, and TaVrs1-D genes) on the chromosomes. The obtained results are shown in Figs. 17 to 20. The sequences of the primers used in the analysis are described in Table 11.

As shown in Figs. 17 to 20, 11 clones (TaVrs1-A) of the 14 clones are positioned in the long arm of chromosome 2A, one clone (TaVrs1-B) is positioned in the long arm of chromosome 2B, and the rest of two clones (TaVrs1-D) are positioned in the long arm of chromosome 2D.

### (Example 6)

### <Comparison of Protein TmVRS1 (TmHOX1) Encoded by Tmvrs1 Gene with Homologous Proteins of Other Poaceae Plants>

The amino acid sequence (SEQ ID NO: 3) of the TmVRS1 protein and the amino acid sequence (SEQ ID NO: 6) of the TmHOX2 protein which were obtained in Example 5 were compared with those of homologous proteins of other Poaceae plants. Specifically, the peptide motifs were analyzed using the comparative genome database (SALAD: Surveyed conserved motif ALignment diagram and the Associating Dendrogram, http://salad.dna.affrc.go.jp/salad/en/). The obtained results are shown in Fig. 21.

Since the TmVRS1 protein has the homeodomain motif and leucine zipper motif described above, the TmVRS1 protein is assumed to be a transcription factor, similar to the VRS1 protein derived from barley (VRS1 protein). The TmVRS1 protein and the VRS1 protein had a very high homology of 86% at the amino acid sequence level.

As apparent from the results shown in Fig. 21, the TmVRS1 protein and the VRS1 protein had identical motifs. Specifically, it was found that, different from the HOX2 protein derived from barley (HvHOX2 protein) and the TmHOX2 protein, the TmVRS1 protein and the barley VRS1 protein had a premature stop codon and thus they were deficient in motif 8 which was well conserved in the HOX2 proteins.

### (Example 7)

### <Analysis on Expression Patterns of TmVrs1 Gene and TmHox2 Gene>

The expression level of the TmVrs1 gene and the TmHox2 gene at each developmental stage of immature spikes of wheat was analyzed. The immature spikes were observed under a stereoscopic microscope to classify them according to the developmental stages (Kirby, E. J. M. and Appleyard, M., 1981 "Cereal development guide", 1981, Kenilworth: Cereal Unit). About ten immature spikes at each developmental stage were collected to extract total RNA. Next, total RNA was extracted from a sample of 50 mg of the collected immature spikes at each developmental stage using TRIzol (produced by Invitrogen). Independent RNA extraction was carried out 3 times for each sample (biological replicates).

The extracted RNA was quantified using NanoDrop (produced by Thermo Fisher Scientific). In order to avoid DNA contamination, the RNA was treated with RNase-free DNase (produced by TaKaRa) according to the description of the manufacturer's instruction. Next, first-strand cDNA was synthesized using SuperScript III (produced by Invitrogen). Then, the transcription level of each gene was determined by the quantitative real-time PCR (qRT-PCR) methodusing the StepOne Real-Time PCR system (produced by Applied-Biosystems) and the THUNDERBIRD SYBR qPCR Mix kit (produced by Toyobo Co. , Ltd.) according to the description of the respective manufacturer's instructions, with the cDNA prepared from 25 ng of RNA as a template,. The obtained results are shown in Fig. 22.

The primers used in the qRT-PCR are described in Table 11. Amplicons were fractionated by electrophoresis using agarose gel and visualized by ethidium bromide staining. The qRT-PCR analysis was carried out at least 3 times for each sample. Each gene fragment was cloned by inserting each gene fragment into pCR4-TOPO (produced by Invitrogen) or pBluescript II KS (+) (produced by Stratagene). Then, in order to make a calibration curve required for quantification of the absolute quantity, a plasmid DNA to which each gene fragment was inserted was prepared.

As apparent from the results shown in Fig. 22, similar to the expression patterns (see Figs. 9 to 12) in barley as shown in Example 3, the transcription level of TmVrs1 in spikes during the developmental stages from the floret primordium stage to the white anther stage was relatively higher than that at other stages. In particular, the TmVrs1 gene showed 10 times or higher expression than the TmHox2 gene at the middle stages of young panicle development (from the terminal spikelet stage to the white anther stage) of wheat.

### (Example 8)

### <Analysis on Organ-Specific Expression of TmVrs1 Gene in Wheat Spikelets>

As mentioned above, unlike barley, wheat bears a plurality of florets in one spikelet (see A of Fig. 23). It is also known that upper florets are sterile in spikelets of wheat. Then, RNA in situ hybridization was carried out to determine the localization of the transcript (mRNA) of the TmVrs1 gene in spikelets.

Specifically, first, aDNAfragment (about 300 bp) specific to the TmVrs1 gene including a 3'-UTR part was amplified with a specific primer (see Table 12) using a cDNA isolated from immature spikes of wheat as a template. The obtained PCR product was cloned by inserting the PCR product into a pBluescript II KS (+) vector (produced by Stratagene). Next, two clones different in insertion direction were linearized by cleavage with NotI or EcoRI. These clones were then used as templates to produce an antisense probe and a sense probe with a T3 RNA polymerase or T7 RNA polymerase. RNA in situ hybridization was carried out according to the method described in NPL 1. The obtained results are shown in Figs. 23 to 26.

As apparent from the results shown in Fig. 23, the expression of the TmVrs1 gene was rarely observed in lower florets among florets in spikelets, and higher expression was observed in upper florets (see B and C of Fig. 23).

When described in more detail, the expression of the TmVrs1 gene was localized in the meristems of spikes which were able to be differentiated into the meristems of florets at the floret primordium stage (see Fig. 24). At the terminal spikelet stage, the expression of the TmVrs1 gene was detected in the meristems of spikelets and the second florets after the differentiation into the second and third floret primordiums (see Fig. 25). The expression of the TmVrs1 at the white anther stage continued to be localized in the meristems of spikelets. Furthermore, the TmVrs1 signal was detected clearly in rachillae having florets (see Fig. 26).

In Einkorn wheat, two florets usually develop and one of them becomes fertile. Therefore, the above results indicated that the function of the TmVrs1 is to inhibit development of upper florets (third, fourth, and fifth florets).

Based on the results described in Examples 6 to 8 above, the sequences and the motifs of the VRS1 protein and the HOX2 protein are highly conserved in barley and wheat, which strongly indicates that their functions are also conserved well. In fact, as described in Example 7, the TmVrs1 gene showed 10 times or higher expression than the TmHox2 gene at the middle stages of young panicle development in barley as well as in wheat. In particular, as described in Example 8, the TmVrs1 gene was found to be very highly expressed in sterile terminal spikelets (upper florets) of wheat, as well as in florets of lateral spikelets of barley.

Therefore, it was found that, in the upper florets of wheat, the expression of the TmVRS1 protein also competitively inhibited the development and grain-bearing of florets in charge of the TmHOX2 protein, and the suppressed function of the TmVrs1 gene can increase the number of wheat grains.

### (Example 9)

### <RNA Interference against Vrs1 Gene in Wheat >

In order to confirm that the TmVrs1 gene suppresses development of upper florets in wheat, and in order to confirm that the suppressed function of the TmVrs1 gene can increase the number of wheat grains, the transcript of the TmVrs1 gene (TaVrs1-A gene) was suppressed by RNAi.

Specifically, first, a TaVrs1-A gene fragment (323 bp) was amplified using a specific primer to prepare an RNAi construct (see Table 12). The region to be amplified by the above PCR using the primer set including the nucleotide sequences described in Table 12 (SEQIDNOS: 54and55) is the region with the nucleotide sequence at positions 998 to 1320 of the TaVrs1-A gene (SEQ ID NO: 56). When this region is the target sequence of the RNAi, it can suppress not only the expression of the TaVrs1-A gene but also the expression of the TaVrs1-B gene and the TaVrs1-D gene.

The amplified fragment thus obtained was cloned into a pENTR D-TOPO vector (produced by Invitrogen). Next, the cloned fragment was introduced by the LR recombination reaction into a pANDA-b vector in which the expression of the transgene was under the control of the maize ubiquitin promoter (see Miki D, etal., Plant Cell Physiol, 2004, volume45, No. 4, 490-495 pages).

Then, this vector was then introduced into wheat immature embryos by the particle bombardment method to produce a transgenic wheat plant (see Pellegrineschi A. et al., Genome, 2002, volume 45, 421-430 pages). The transgenic plant was grown in a greenhouse in which the temperature in the daytime was controlled at 25 °C and the temperature at night was controlled at 20 °C. The presence of the transgene (RNAi transgene) was confirmedbythe PCRamplificationusingavector-specificprimer (see Table 12).

Then, 22 independent T0 transgene plants having the RNAi transgene was identified by PCR. The expression of the transgene in flag-leaves was observed by qRT-PCR in 71 plants of 22 transgenic plants. In addition, the spikelets in the top part and the bottom part did not develop well in the RNAi transgenic plants, and thus the number of florets per spikelet, except for these terminal spikelets, was counted. As a result, the number of florets significantly increased as compared with wild-type plants (see Figs. 27 to 29). The number of grains per spikelet also significantly increased in some RNAi transgenic plants (see Fig. 30). The highest number of florets per spikelet was 12 and the highest number of grains per spikelet was 5 in T0 transgenic plants.

The above results indicated that the TmVrs1 gene suppressed the development of upper florets in wheat and controlled the number of florets and the number of grains per spikelet.

### [Industrial Applicability]

As described above, the present invention can give the fertility to sterile florets by suppressing the function of the wheat Vrs1 gene and thus can increase the number of wheat grains. Therefore, the method, the agent, and the like for increasing the number of wheat grains of the present invention are useful for satisfying the demand of wheat which is globally in short supply.

### [Sequence Listing Free Text]

SEQ ID NOS: 7 to 26, 30 to 55, and 65 to 77
<223> Artificially synthesized primer sequence
SEQ ID NO: 27
<223> Artificially synthesized polypeptide antigen
<223> X represents aminohexanoic acid

## Claims

1. A wheat with an increased number of grains as compared with a control wheat, wherein the function of an endogenous wheat Vrs1 gene is artificially suppressed.

2. The wheat according to claim 1, wherein the function of the endogenous wheat Vrs1 gene is artificially suppressed by a double-stranded RNA method, an antisense method, or a ribozyme method.

3. The wheat according to claim 1, wherein the function of the endogenous wheat Vrs1 gene is artificially suppressed by introducing one or more mutations into the gene.

4. A propagation material of the wheat according to any one of claims 1 to 3.

5. A processed product made from seeds of the wheat according to any one of claims 1 to 3.

6. A method for producing a wheat with an increased number of grains as compared with a control wheat, the method comprising a step of artificially suppressing the function of the endogenous wheat Vrs1 gene.

7. A progeny wheat or cloned wheat of the wheat produced by the method according to claim 6, wherein the function of the endogenous wheat Vrs1 gene is artificially suppressed to increase the number of grains as compared with a control wheat.

8. An agent for increasing the number of wheat grains, the agent comprising as an active ingredient at least one DNA selected from the group consisting of (a) to (c) below or a vector having the inserted DNA:
(a) a DNA encoding a double-stranded RNA complementary to the transcript of the wheat Vrs1 gene;
(b) a DNA encoding an antisense RNA complementary to the transcript of the wheat Vrs1 gene; and
(c) a DNA encoding a RNA having a ribozyme activity to specifically cleave the transcript of the wheat Vrs1 gene.
